# EUROPEAN PATENT SPECIFICATION

(11) **EP 2 603 518 B1**
(45) Date of publication and mention of the grant of the patent: **17.06.2015**
(21) Application number: 11743526.3
(22) Date of filing: 10.08.2011
(51) Int. Cl.: C07K 14/33

(54) **SELECTIVE MANUFACTURE OF RECOMBINANT NEUROTOXIN POLYPEPTIDES**
SELEKTIVE HERSTELLUNG VON REKOMBINANTEN NEUROTOXIN-POLYPEPTIDEN
FABRICATION SÉLECTIVE DE POLYPEPTIDES DE NEUROTOXINE RECOMBINANTS

(30) Priority: 11.08.2010 US 401334 P; 11.08.2010 EP 10172526
(43) Date of publication of application: 19.06.2013
(73) Proprietor: Merz Pharma GmbH & Co. KGaA, 60318 Frankfurt am Main (DE)
(72) Inventor: GREIN, Swen, 61118 Bad Vilbel (DE); HÖLSCHER, Kerstin, 61440 Oberursel (DE); STÖVEKEN, Tim, 61184 Karben (DE); EISELE, Karl-Heinz, 60385 Frankfurt am Main (DE)
(74) Representative: Herzog, Fiesser & Partner Patentanwälte PartG mbB
(86) International application number: PCT/EP2011/063774
(87) International publication number: WO 2012/020057

(56) References cited:
- WO-A1-2010/124998
- WO-A2-2004/024909
- WO-A2-2006/076902

### SEQUENCE LISTING

<110> Merz Pharma GmbH & Co.KGaA
<120> Selective manufacture of recombinant neurotoxin polypeptides
<130> MP66826PC
<160> 16
<170> PatentIn version 3.5
<210> 1
   <211> 3891
   <212> DNA
   <213> Clostridium botulinum
<400> 1
   atgccatttg ttaataaaca atttaattat aaagatcctg taaatggtgt tgatattgct      60
   tatataaaaa ttccaaatgc aggacaaatg caaccagtaa aagcttttaa aattcataat     120
   aaaatatggg ttattccaga aagagataca tttacaaatc ctgaagaagg agatttaaat     180
   ccaccaccag aagcaaaaca agttccagtt tcatattatg attcaacata tttaagtaca     240
   gataatgaaa aagataatta tttaaaggga gttacaaaat tatttgagag aatttattca     300
   actgatcttg gaagaatgtt gttaacatca atagtaaggg gaataccatt ttggggtgga     360
   agtacaatag atacagaatt aaaagttatt gatactaatt gtattaatgt gatacaacca     420
   gatggtagtt atagatcaga agaacttaat ctagtaataa taggaccctc agctgatatt     480
   atacagtttg aatgtaaaag ctttggacat gaagttttga atcttacgcg aaatggttat     540
   ggctctactc aatacattag atttagccca gattttacat ttggttttga ggagtcactt     600
   gaagttgata caaatcctct tttaggtgca ggcaaatttg ctacagatcc agcagtaaca     660
   ttagcacatg aacttataca tgctggacat agattatatg gaatagcaat taatccaaat     720
   agggttttta aagtaaatac taatgcctat tatgaaatga gtgggttaga agtaagcttt     780
   gaggaactta gaacatttgg gggacatgat gcaaagttta tagatagttt acaggaaaac     840
   gaatttcgtc tatattatta taataagttt aaagatatag caagtacact taataaagct     900
   aaatcaatag taggtactac tgcttcatta cagtatatga aaaatgtttt taaagagaaa     960
   tatctcctat ctgaagatac atctggaaaa ttttcggtag ataaattaaa atttgataag    1020
   ttatacaaaa tgttaacaga gatttacaca gaggataatt ttgttaagtt ttttaaagta    1080
   cttaacagaa aaacatattt gaattttgat aaagccgtat ttaagataaa tatagtacct    1140
   aaggtaaatt acacaatata tgatggattt aatttaagaa atacaaattt agcagcaaac    1200
   tttaatggtc aaaatacaga aattaataat atgaatttta ctaaactaaa aaattttact    1260
   ggattgtttg aattttataa gttgctatgt gtaagaggga taataacttc taaaactaaa    1320
   tcattagata aaggatacaa taaggcatta aatgatttat gtatcaaagt taataattgg    1380
   gacttgtttt ttagtccttc agaagataat tttactaatg atctaaataa aggagaagaa    1440
   attacatctg atactaatat agaagcagca gaagaaaata ttagtttaga tttaatacaa    1500
   caatattatt taacctttaa ttttgataat gaacctgaaa atatttcaat agaaaatctt    1560
   tcaagtgaca ttataggcca attagaactt atgcctaata tagaaagatt tcctaatgga 1620
   aaaaagtatg agttagataa atatactatg ttccattatc ttcgtgctca agaatttgaa 1680
   catggtaaat ctaggattgc tttaacaaat tctgttaacg aagcattatt aaatcctagt 1740
   cgtgtttata catttttttc ttcagactat gtaaagaaag ttaataaagc tacggaggca 1800
   gctatgtttt taggctgggt agaacaatta gtatatgatt ttaccgatga aactagcgaa 1860
   gtaagtacta cggataaaat tgcggatata actataatta ttccatatat aggacctgct 1920
   ttaaatatag gtaatatgtt atataaagat gattttgtag gtgctttaat attttcagga 1980
   gctgttattc tgttagaatt tataccagag attgcaatac ctgtattagg tacttttgca 2040
   cttgtatcat atattgcgaa taaggttcta accgttcaaa caatagataa tgctttaagt 2100
   aaaagaaatg aaaaatggga tgaggtctat aaatatatag taacaaattg gttagcaaag 2160
   gttaatacac agattgatct aataagaaaa aaaatgaaag aagctttaga aaatcaagca 2220
   gaagcaacaa aggctataat aaactatcag tataatcaat atactgagga agagaaaaat 2280
   aatattaatt ttaatattga tgatttaagt tcgaaactta atgagtctat aaataaagct 2340
   atgattaata taaataaatt tttgaatcaa tgctctgttt catatttaat gaattctatg 2400
   atcccttatg gtgttaaacg gttagaagat tttgatgcta gtcttaaaga tgcattatta 2460
   aagtatatat atgataatag aggaacttta attggtcaag tagatagatt aaaagataaa 2520
   gttaataata cacttagtac agatatacct tttcagcttt ccaaatacgt agataatcaa 2580
   agattattat ctacatttac tgaatatatt aagaatatta ttaatacttc tatattgaat 2640
   ttaagatatg aaagtaatca tttaatagac ttatctaggt atgcatcaaa aataaatatt 2700
   ggtagtaaag taaattttga tccaatagat aaaaatcaaa ttcaattatt taatttagaa 2760
   agtagtaaaa ttgaggtaat tttaaaaaat gctattgtat ataatagtat gtatgaaaat 2820
   tttagtacta gcttttggat aagaattcct aagtatttta acagtataag tctaaataat 2880
   gaatatacaa taataaattg tatggaaaat aattcaggat ggaaagtatc acttaattat 2940
   ggtgaaataa tctggacttt acaggatact caggaaataa aacaaagagt agtttttaaa 3000
   tacagtcaaa tgattaatat atcagattat ataaacagat ggatttttgt aactatcact 3060
   aataatagat taaataactc taaaatttat ataaatggaa gattaataga tcaaaaacca 3120
   atttcaaatt taggtaatat tcatgctagt aataatataa tgtttaaatt agatggttgt 3180
   agagatacac atagatatat ttggataaaa tattttaatc tttttgataa ggaattaaat 3240
   gaaaaagaaa tcaaagattt atatgataat caatcaaatt caggtatttt aaaagacttt 3300
   tggggtgatt atttacaata tgataaacca tactatatgt taaatttata tgatccaaat 3360
   aaatatgtcg atgtaaataa tgtaggtatt agaggttata tgtatcttaa agggcctaga 3420
   ggtagcgtaa tgactacaaa catttattta aattcaagtt tgtatagggg gacaaaattt 3480
   attataaaaa aatatgcttc tggaaataaa gataatattg ttagaaataa tgatcgtgta 3540
   tatattaatg tagtagttaa aaataaagaa tataggttag ctactaatgc atcacaggca 3600
   ggcgtagaaa aaatactaag tgcattagaa atacctgatg taggaaatct aagtcaagta 3660
   gtagtaatga agtcaaaaaa tgatcaagga ataacaaata aatgcaaaat gaatttacaa 3720
   gataataatg ggaatgatat aggctttata ggatttcatc agtttaataa tatagctaaa 3780
   ctagtagcaa gtaattggta taatagacaa atagaaagat ctagtaggac tttgggttgc 3840
   tcatgggaat ttattcctgt agatgatgga tggggagaaa ggccactgta a 3891
<210> 2
   <211> 1296
   <212> PRT
   <213> Clostridium botulinum
<400> 2
   Met Pro Phe Val Asn Lys Gln Phe Asn Tyr Lys Asp Pro Val Asn Gly
   1               5                   10                  15
   Val Asp Ile Ala Tyr Ile Lys Ile Pro Asn Ala Gly Gln Met Gln Pro
               20                  25                  30
   Val Lys Ala Phe Lys Ile His Asn Lys Ile Trp Val Ile Pro Glu Arg
           35                  40                  45
   Asp Thr Phe Thr Asn Pro Glu Glu Gly Asp Leu Asn Pro Pro Pro Glu
       50                  55                  60
   Ala Lys Gln Val Pro Val Ser Tyr Tyr Asp Ser Thr Tyr Leu Ser Thr
   65                  70                  75                  80
   Asp Asn Glu Lys Asp Asn Tyr Leu Lys Gly Val Thr Lys Leu Phe Glu
                   85                  90                  95
   Arg Ile Tyr Ser Thr Asp Leu Gly Arg Met Leu Leu Thr Ser Ile Val
               100                 105                 110
   Arg Gly Ile Pro Phe Trp Gly Gly Ser Thr Ile Asp Thr Glu Leu Lys
           115                 120                 125
   Val Ile Asp Thr Asn Cys Ile Asn Val Ile Gln Pro Asp Gly Ser Tyr
       130                 135                 140
   Arg Ser Glu Glu Leu Asn Leu Val Ile Ile Gly Pro Ser Ala Asp Ile
   145                 150                 155                 160
   Ile Gln Phe Glu Cys Lys Ser Phe Gly His Glu Val Leu Asn Leu Thr
                   165                 170                 175
   Arg Asn Gly Tyr Gly Ser Thr Gln Tyr Ile Arg Phe Ser Pro Asp Phe
               180                 185                 190
   Thr Phe Gly Phe Glu Glu Ser Leu Glu Val Asp Thr Asn Pro Leu Leu
           195                 200                 205
   Gly Ala Gly Lys Phe Ala Thr Asp Pro Ala Val Thr Leu Ala His Glu
       210                 215                 220
   Leu Ile His Ala Gly His Arg Leu Tyr Gly Ile Ala Ile Asn Pro Asn
   225                 230                 235                 240
   Arg Val Phe Lys Val Asn Thr Asn Ala Tyr Tyr Glu Met Ser Gly Leu
                   245                 250                 255
   Glu Val Ser Phe Glu Glu Leu Arg Thr Phe Gly Gly His Asp Ala Lys
               260                 265                 270
   Phe Ile Asp Ser Leu Gln Glu Asn Glu Phe Arg Leu Tyr Tyr Tyr Asn
           275                 280                 285
   Lys Phe Lys Asp Ile Ala Ser Thr Leu Asn Lys Ala Lys Ser Ile Val
       290                 295                 300
   Gly Thr Thr Ala Ser Leu Gln Tyr Met Lys Asn Val Phe Lys Glu Lys
   305                 310                 315                 320
   Tyr Leu Leu Ser Glu Asp Thr Ser Gly Lys Phe Ser Val Asp Lys Leu
                   325                 330                 335
   Lys Phe Asp Lys Leu Tyr Lys Met Leu Thr Glu Ile Tyr Thr Glu Asp
               340                 345                 350
   Asn Phe Val Lys Phe Phe Lys Val Leu Asn Arg Lys Thr Tyr Leu Asn
           355                 360                 365
   Phe Asp Lys Ala Val Phe Lys Ile Asn Ile Val Pro Lys Val Asn Tyr
       370                 375                 380
   Thr Ile Tyr Asp Gly Phe Asn Leu Arg Asn Thr Asn Leu Ala Ala Asn
   385                 390                 395                 400
   Phe Asn Gly Gln Asn Thr Glu Ile Asn Asn Met Asn Phe Thr Lys Leu
                   405                 410                 415
   Lys Asn Phe Thr Gly Leu Phe Glu Phe Tyr Lys Leu Leu Cys Val Arg
               420                 425                 430
   Gly Ile Ile Thr Ser Lys Thr Lys Ser Leu Asp Lys Gly Tyr Asn Lys
           435                 440                 445
   Ala Leu Asn Asp Leu Cys Ile Lys Val Asn Asn Trp Asp Leu Phe Phe
       450                 455                 460
   Ser Pro Ser Glu Asp Asn Phe Thr Asn Asp Leu Asn Lys Gly Glu Glu
   465                 470                 475                 480
   Ile Thr Ser Asp Thr Asn Ile Glu Ala Ala Glu Glu Asn Ile Ser Leu
   485 490 495
   Asp Leu Ile Gln Gln Tyr Tyr Leu Thr Phe Asn Phe Asp Asn Glu Pro
   500 505 510
   Glu Asn Ile Ser Ile Glu Asn Leu Ser Ser Asp Ile Ile Gly Gln Leu
   515 520 525
   Glu Leu Met Pro Asn Ile Glu Arg Phe Pro Asn Gly Lys Lys Tyr Glu
   530 535 540
   Leu Asp Lys Tyr Thr Met Phe His Tyr Leu Arg Ala Gln Glu Phe Glu
   545 550 555 560
   His Gly Lys Ser Arg Ile Ala Leu Thr Asn Ser Val Asn Glu Ala Leu
   565 570 575
   Leu Asn Pro Ser Arg Val Tyr Thr Phe Phe Ser Ser Asp Tyr Val Lys
   580 585 590
   Lys Val Asn Lys Ala Thr Glu Ala Ala Met Phe Leu Gly Trp Val Glu
   595 600 605
   Gln Leu Val Tyr Asp Phe Thr Asp Glu Thr Ser Glu Val Ser Thr Thr
   610 615 620
   Asp Lys Ile Ala Asp Ile Thr Ile Ile Ile Pro Tyr Ile Gly Pro Ala
   625 630 635 640
   Leu Asn Ile Gly Asn Met Leu Tyr Lys Asp Asp Phe Val Gly Ala Leu
   645 650 655
   Ile Phe Ser Gly Ala Val Ile Leu Leu Glu Phe Ile Pro Glu Ile Ala
   660 665 670
   Ile Pro Val Leu Gly Thr Phe Ala Leu Val Ser Tyr Ile Ala Asn Lys
   675 680 685
   Val Leu Thr Val Gln Thr Ile Asp Asn Ala Leu Ser Lys Arg Asn Glu
   690 695 700
   Lys Trp Asp Glu Val Tyr Lys Tyr Ile Val Thr Asn Trp Leu Ala Lys
   705 710 715 720
   Val Asn Thr Gln Ile Asp Leu Ile Arg Lys Lys Met Lys Glu Ala Leu
   725 730 735
   Glu Asn Gln Ala Glu Ala Thr Lys Ala Ile Ile Asn Tyr Gln Tyr Asn
   740 745 750
   Gln Tyr Thr Glu Glu Glu Lys Asn Asn Ile Asn Phe Asn Ile Asp Asp
   755 760 765
   Leu Ser Ser Lys Leu Asn Glu Ser Ile Asn Lys Ala Met Ile Asn Ile
   770 775 780
   Asn Lys Phe Leu Asn Gln Cys Ser Val Ser Tyr Leu Met Asn Ser Met
   785 790 795 800
   Ile Pro Tyr Gly Val Lys Arg Leu Glu Asp Phe Asp Ala Ser Leu Lys
   805 810 815
   Asp Ala Leu Leu Lys Tyr Ile Tyr Asp Asn Arg Gly Thr Leu Ile Gly
   820 825 830
   Gln Val Asp Arg Leu Lys Asp Lys Val Asn Asn Thr Leu Ser Thr Asp
   835 840 845
   Ile Pro Phe Gln Leu Ser Lys Tyr Val Asp Asn Gln Arg Leu Leu Ser
   850 855 860
   Thr Phe Thr Glu Tyr Ile Lys Asn Ile Ile Asn Thr Ser Ile Leu Asn
   865 870 875 880
   Leu Arg Tyr Glu Ser Asn His Leu Ile Asp Leu Ser Arg Tyr Ala Ser
   885 890 895
   Lys Ile Asn Ile Gly Ser Lys Val Asn Phe Asp Pro Ile Asp Lys Asn
   900 905 910
   Gln Ile Gln Leu Phe Asn Leu Glu Ser Ser Lys Ile Glu Val Ile Leu
   915 920 925
   Lys Asn Ala Ile Val Tyr Asn Ser Met Tyr Glu Asn Phe Ser Thr Ser
   930 935 940
   Phe Trp Ile Arg Ile Pro Lys Tyr Phe Asn Ser Ile Ser Leu Asn Asn
   945 950 955 960
   Glu Tyr Thr Ile Ile Asn Cys Met Glu Asn Asn Ser Gly Trp Lys Val
   965 970 975
   Ser Leu Asn Tyr Gly Glu Ile Ile Trp Thr Leu Gln Asp Thr Gln Glu
   980 985 990
   Ile Lys Gln Arg Val Val Phe Lys Tyr Ser Gln Met Ile Asn Ile Ser
   995 1000 1005
   Asp Tyr Ile Asn Arg Trp Ile Phe Val Thr Ile Thr Asn Asn Arg
   1010 1015 1020
   Leu Asn Asn Ser Lys Ile Tyr Ile Asn Gly Arg Leu Ile Asp Gln
   1025 1030 1035
   Lys Pro Ile Ser Asn Leu Gly Asn Ile His Ala Ser Asn Asn Ile
   1040 1045 1050
   Met Phe Lys Leu Asp Gly Cys Arg Asp Thr His Arg Tyr Ile Trp
   1055 1060 1065
   Ile Lys Tyr Phe Asn Leu Phe Asp Lys Glu Leu Asn Glu Lys Glu
   1070 1075 1080
   Ile Lys Asp Leu Tyr Asp Asn Gln Ser Asn Ser Gly Ile Leu Lys
   1085 1090 1095
   Asp Phe Trp Gly Asp Tyr Leu Gln Tyr Asp Lys Pro Tyr Tyr Met
   1100 1105 1110
   Leu Asn Leu Tyr Asp Pro Asn Lys Tyr Val Asp Val Asn Asn Val
   1115 1120 1125
   Gly Ile Arg Gly Tyr Met Tyr Leu Lys Gly Pro Arg Gly Ser Val
   1130 1135 1140
   Met Thr Thr Asn Ile Tyr Leu Asn Ser Ser Leu Tyr Arg Gly Thr
   1145 1150 1155
   Lys Phe Ile Ile Lys Lys Tyr Ala Ser Gly Asn Lys Asp Asn Ile
   1160 1165 1170
   Val Arg Asn Asn Asp Arg Val Tyr Ile Asn Val Val Val Lys Asn
   1175 1180 1185
   Lys Glu Tyr Arg Leu Ala Thr Asn Ala Ser Gln Ala Gly Val Glu
   1190 1195 1200
   Lys Ile Leu Ser Ala Leu Glu Ile Pro Asp Val Gly Asn Leu Ser
   1205 1210 1215
   Gln Val Val Val Met Lys Ser Lys Asn Asp Gln Gly Ile Thr Asn
   1220 1225 1230
   Lys Cys Lys Met Asn Leu Gln Asp Asn Asn Gly Asn Asp Ile Gly
   1235 1240 1245
   Phe Ile Gly Phe His Gln Phe Asn Asn Ile Ala Lys Leu Val Ala
   1250 1255 1260
   Ser Asn Trp Tyr Asn Arg Gln Ile Glu Arg Ser Ser Arg Thr Leu
   1265 1270 1275
   Gly Cys Ser Trp Glu Phe Ile Pro Val Asp Asp Gly Trp Gly Glu
   1280 1285 1290
   Arg Pro Leu
   1295
<210> 3
   <211> 3876
   <212> DNA
   <213> Clostridium botulinum
<400> 3
   atgccagtta caataaataa ttttaattat aatgatccta ttgataataa taatattatt      60
   atgatggagc ctccatttgc gagaggtacg gggagatatt ataaagcttt taaaatcaca     120
   gatcgtattt ggataatacc ggaaagatat acttttggat ataaacctga ggattttaat     180
   aaaagttccg gtatttttaa tagagatgtt tgtgaatatt atgatccaga ttacttaaat     240
   actaatgata aaaagaatat atttttacaa acaatgatca agttatttaa tagaatcaaa     300
   tcaaaaccat tgggtgaaaa gttattagag atgattataa atggtatacc ttatcttgga     360
   gatagacgtg ttccactcga agagtttaac acaaacattg ctagtgtaac tgttaataaa     420
   ttaatcagta atccaggaga agtggagcga aaaaaaggta ttttcgcaaa tttaataata     480
   tttggacctg ggccagtttt aaatgaaaat gagactatag atataggtat acaaaatcat     540
   tttgcatcaa gggaaggctt cgggggtata atgcaaatga agttttgccc agaatatgta     600
   agcgtattta ataatgttca agaaaacaaa ggcgcaagta tatttaatag acgtggatat     660
   ttttcagatc cagccttgat attaatgcat gaacttatac atgttttaca tggattatat     720
   ggcattaaag tagatgattt accaattgta ccaaatgaaa aaaaattttt tatgcaatct     780
   acagatgcta tacaggcaga agaactatat acatttggag gacaagatcc cagcatcata     840
   actccttcta cggataaaag tatctatgat aaagttttgc aaaattttag agggatagtt     900
   gatagactta acaaggtttt agtttgcata tcagatccta acattaatat taatatatat     960
   aaaaataaat ttaaagataa atataaattc gttgaagatt ctgagggaaa atatagtata    1020
   gatgtagaaa gttttgataa attatataaa agcttaatgt ttggttttac agaaactaat    1080
   atagcagaaa attataaaat aaaaactaga gcttcttatt ttagtgattc cttaccacca    1140
   gtaaaaataa aaaatttatt agataatgaa atctatacta tagaggaagg gtttaatata    1200
   tctgataaag atatggaaaa agaatataga ggtcagaata aagctataaa taaacaagct    1260
   tatgaagaaa ttagcaagga gcatttggct gtatataaga tacaaatgtg taaaagtgtt    1320
   aaagctccag gaatatgtat tgatgttgat aatgaagatt tgttctttat agctgataaa    1380
   aatagttttt cagatgattt atctaaaaac gaaagaatag aatataatac acagagtaat    1440
   tatatagaaa atgacttccc tataaatgaa ttaattttag atactgattt aataagtaaa    1500
   atagaattac caagtgaaaa tacagaatca cttactgatt ttaatgtaga tgttccagta    1560
   tatgaaaaac aacccgctat aaaaaaaatt tttacagatg aaaataccat ctttcaatat    1620
   ttatactctc agacatttcc tctagatata agagatataa gtttaacatc ttcatttgat    1680
   gatgcattat tattttctaa caaagtttat tcattttttt ctatggatta tattaaaact    1740
   gctaataaag tggtagaagc aggattattt gcaggttggg tgaaacagat agtaaatgat 1800
   tttgtaatcg aagctaataa aagcaatact atggataaaa ttgcagatat atctctaatt 1860
   gttccttata taggattagc tttaaatgta ggaaatgaaa cagctaaagg aaattttgaa 1920
   aatgcttttg agattgcagg agccagtatt ctactagaat ttataccaga acttttaata 1980
   cctgtagttg gagccttttt attagaatca tatattgaca ataaaaataa aattattaaa 2040
   acaatagata atgctttaac taaaagaaat gaaaaatgga gtgatatgta cggattaata 2100
   gtagcgcaat ggctctcaac agttaatact caattttata caataaaaga gggaatgtat 2160
   aaggctttaa attatcaagc acaagcattg gaagaaataa taaaatacag atataatata 2220
   tattctgaaa aagaaaagtc aaatattaac atcgatttta atgatataaa ttctaaactt 2280
   aatgagggta ttaaccaagc tatagataat ataaataatt ttataaatgg atgttctgta 2340
   tcatatttaa tgaaaaaaat gattccatta gctgtagaaa aattactaga ctttgataat 2400
   actctcaaaa aaaatttgtt aaattatata gatgaaaata aattatattt gattggaagt 2460
   gcagaatatg aaaaatcaaa agtaaataaa tacttgaaaa ccattatgcc gtttgatctt 2520
   tcaatatata ccaatgatac aatactaata gaaatgttta ataaatataa tagcgaaatt 2580
   ttaaataata ttatcttaaa tttaagatat aaggataata atttaataga tttatcagga 2640
   tatggggcaa aggtagaggt atatgatgga gtcgagctta atgataaaaa tcaatttaaa 2700
   ttaactagtt cagcaaatag taagattaga gtgactcaaa atcagaatat catatttaat 2760
   agtgtgttcc ttgattttag cgttagcttt tggataagaa tacctaaata taagaatgat 2820
   ggtatacaaa attatattca taatgaatat acaataatta attgtatgaa aaataattcg 2880
   ggctggaaaa tatctattag ggtaataggg ataatatgga ctttaattga tataaatgga 2940
   aaaaccaaat cggtattttt tgaatataac ataagagaag atatatcaga gtatataaat 3000
   agatggtttt ttgtaactat tactaataat ttgaataacg ctaaaattta tattaatggt 3060
   aagctagaat caaatacaga tattaaagat ataagagaag ttattgctaa tggtgaaata 3120
   atatttaaat tagatggtga tatagataga acacaattta tttggatgaa atatttcagt 3180
   atttttaata cggaattaag tcaatcaaat attgaagaaa gatataaaat tcaatcatat 3240
   agcgaatatt taaaagattt ttggggaaat cctttaatgt acaataaaga atattatatg 3300
   tttaatgcgg ggaataaaaa ttcatatatt aaactaaaga aagattcacc tgtaggtgaa 3360
   attttaacac gtagcaaata taatcaaaat tctaaatata taaattatag agatttatat 3420
   attggagaaa aatttattat aagaagaaag tcaaattctc aatctataaa tgatgatata 3480
   gttagaaaag aagattatat atatctagat ttttttaatt taaatcaaga gtggagagta 3540
   tatacctata aatattttaa gaaagaggaa gaaaaattgt ttttagctcc tataagtgat 3600
   tctgatgagt tttacaatac tatacaaata aaagaatatg atgaacagcc aacatatagt 3660
   tgtcagttgc tttttaaaaa agatgaagaa agtactgatg agataggatt gattggtatt 3720
   catcgtttct acgaatctgg aattgtattt gaagagtata aagattattt ttgtataagt 3780
   aaatggtact taaaagaggt aaaaaggaaa ccatataatt taaaattggg atgtaattgg 3840
   cagtttattc ctaaagatga agggtggact gaataa 3876
<210> 4
   <211> 1291
   <212> PRT
   <213> Clostridium botulinum
<400> 4
   Met Pro Val Thr Ile Asn Asn Phe Asn Tyr Asn Asp Pro Ile Asp Asn
   1 5 10 15
   Asn Asn Ile Ile Met Met Glu Pro Pro Phe Ala Arg Gly Thr Gly Arg
   20 25 30
   Tyr Tyr Lys Ala Phe Lys Ile Thr Asp Arg Ile Trp Ile Ile Pro Glu
   35 40 45
   Arg Tyr Thr Phe Gly Tyr Lys Pro Glu Asp Phe Asn Lys Ser Ser Gly
   50 55 60
   Ile Phe Asn Arg Asp Val Cys Glu Tyr Tyr Asp Pro Asp Tyr Leu Asn
   65 70 75 80
   Thr Asn Asp Lys Lys Asn Ile Phe Leu Gln Thr Met Ile Lys Leu Phe
   85 90 95
   Asn Arg Ile Lys Ser Lys Pro Leu Gly Glu Lys Leu Leu Glu Met Ile
   100 105 110
   Ile Asn Gly Ile Pro Tyr Leu Gly Asp Arg Arg Val Pro Leu Glu Glu
   115 120 125
   Phe Asn Thr Asn Ile Ala Ser Val Thr Val Asn Lys Leu Ile Ser Asn
   130 135 140
   Pro Gly Glu Val Glu Arg Lys Lys Gly Ile Phe Ala Asn Leu Ile Ile
   145 150 155 160
   Phe Gly Pro Gly Pro Val Leu Asn Glu Asn Glu Thr Ile Asp Ile Gly
   165 170 175
   Ile Gln Asn His Phe Ala Ser Arg Glu Gly Phe Gly Gly Ile Met Gln
   180 185 190
   Met Lys Phe Cys Pro Glu Tyr Val Ser Val Phe Asn Asn Val Gln Glu
   195 200 205
   Asn Lys Gly Ala Ser Ile Phe Asn Arg Arg Gly Tyr Phe Ser Asp Pro
   210 215 220
   Ala Leu Ile Leu Met His Glu Leu Ile His Val Leu His Gly Leu Tyr
   225 230 235 240
   Gly Ile Lys Val Asp Asp Leu Pro Ile Val Pro Asn Glu Lys Lys Phe
   245 250 255
   Phe Met Gln Ser Thr Asp Ala Ile Gln Ala Glu Glu Leu Tyr Thr Phe
   260 265 270
   Gly Gly Gln Asp Pro Ser Ile Ile Thr Pro Ser Thr Asp Lys Ser Ile
   275 280 285
   Tyr Asp Lys Val Leu Gln Asn Phe Arg Gly Ile Val Asp Arg Leu Asn
   290 295 300
   Lys Val Leu Val Cys Ile Ser Asp Pro Asn Ile Asn Ile Asn Ile Tyr
   305 310 315 320
   Lys Asn Lys Phe Lys Asp Lys Tyr Lys Phe Val Glu Asp Ser Glu Gly
   325 330 335
   Lys Tyr Ser Ile Asp Val Glu Ser Phe Asp Lys Leu Tyr Lys Ser Leu
   340 345 350
   Met Phe Gly Phe Thr Glu Thr Asn Ile Ala Glu Asn Tyr Lys Ile Lys
   355 360 365
   Thr Arg Ala Ser Tyr Phe Ser Asp Ser Leu Pro Pro Val Lys Ile Lys
   370 375 380
   Asn Leu Leu Asp Asn Glu Ile Tyr Thr Ile Glu Glu Gly Phe Asn Ile
   385 390 395 400
   Ser Asp Lys Asp Met Glu Lys Glu Tyr Arg Gly Gln Asn Lys Ala Ile
   405 410 415
   Asn Lys Gln Ala Tyr Glu Glu Ile Ser Lys Glu His Leu Ala Val Tyr
   420 425 430
   Lys Ile Gln Met Cys Lys Ser Val Lys Ala Pro Gly Ile Cys Ile Asp
   435 440 445
   Val Asp Asn Glu Asp Leu Phe Phe Ile Ala Asp Lys Asn Ser Phe Ser
   450 455 460
   Asp Asp Leu Ser Lys Asn Glu Arg Ile Glu Tyr Asn Thr Gln Ser Asn
   465 470 475 480
   Tyr Ile Glu Asn Asp Phe Pro Ile Asn Glu Leu Ile Leu Asp Thr Asp
   485 490 495
   Leu Ile Ser Lys Ile Glu Leu Pro Ser Glu Asn Thr Glu Ser Leu Thr
   500 505 510
   Asp Phe Asn Val Asp Val Pro Val Tyr Glu Lys Gln Pro Ala Ile Lys
   515 520 525
   Lys Ile Phe Thr Asp Glu Asn Thr Ile Phe Gln Tyr Leu Tyr Ser Gln
   530 535 540
   Thr Phe Pro Leu Asp Ile Arg Asp Ile Ser Leu Thr Ser Ser Phe Asp
   545 550 555 560
   Asp Ala Leu Leu Phe Ser Asn Lys Val Tyr Ser Phe Phe Ser Met Asp
   565 570 575
   Tyr Ile Lys Thr Ala Asn Lys Val Val Glu Ala Gly Leu Phe Ala Gly
   580 585 590
   Trp Val Lys Gln Ile Val Asn Asp Phe Val Ile Glu Ala Asn Lys Ser
   595 600 605
   Asn Thr Met Asp Lys Ile Ala Asp Ile Ser Leu Ile Val Pro Tyr Ile
   610 615 620
   Gly Leu Ala Leu Asn Val Gly Asn Glu Thr Ala Lys Gly Asn Phe Glu
   625 630 635 640
   Asn Ala Phe Glu Ile Ala Gly Ala Ser Ile Leu Leu Glu Phe Ile Pro
   645 650 655
   Glu Leu Leu Ile Pro Val Val Gly Ala Phe Leu Leu Glu Ser Tyr Ile
   660 665 670
   Asp Asn Lys Asn Lys Ile Ile Lys Thr Ile Asp Asn Ala Leu Thr Lys
   675 680 685
   Arg Asn Glu Lys Trp Ser Asp Met Tyr Gly Leu Ile Val Ala Gln Trp
   690 695 700
   Leu Ser Thr Val Asn Thr Gln Phe Tyr Thr Ile Lys Glu Gly Met Tyr
   705 710 715 720
   Lys Ala Leu Asn Tyr Gln Ala Gln Ala Leu Glu Glu Ile Ile Lys Tyr
   725 730 735
   Arg Tyr Asn Ile Tyr Ser Glu Lys Glu Lys Ser Asn Ile Asn Ile Asp
   740 745 750
   Phe Asn Asp Ile Asn Ser Lys Leu Asn Glu Gly Ile Asn Gln Ala Ile
   755 760 765
   Asp Asn Ile Asn Asn Phe Ile Asn Gly Cys Ser Val Ser Tyr Leu Met
   770 775 780
   Lys Lys Met Ile Pro Leu Ala Val Glu Lys Leu Leu Asp Phe Asp Asn
   785 790 795 800
   Thr Leu Lys Lys Asn Leu Leu Asn Tyr Ile Asp Glu Asn Lys Leu Tyr
   805 810 815
   Leu Ile Gly Ser Ala Glu Tyr Glu Lys Ser Lys Val Asn Lys Tyr Leu
   820 825 830
   Lys Thr Ile Met Pro Phe Asp Leu Ser Ile Tyr Thr Asn Asp Thr Ile
   835 840 845
   Leu Ile Glu Met Phe Asn Lys Tyr Asn Ser Glu Ile Leu Asn Asn Ile
   850 855 860
   Ile Leu Asn Leu Arg Tyr Lys Asp Asn Asn Leu Ile Asp Leu Ser Gly
   865 870 875 880
   Tyr Gly Ala Lys Val Glu Val Tyr Asp Gly Val Glu Leu Asn Asp Lys
   885 890 895
   Asn Gln Phe Lys Leu Thr Ser Ser Ala Asn Ser Lys Ile Arg Val Thr
   900 905 910
   Gln Asn Gln Asn Ile Ile Phe Asn Ser Val Phe Leu Asp Phe Ser Val
   915 920 925
   Ser Phe Trp Ile Arg Ile Pro Lys Tyr Lys Asn Asp Gly Ile Gln Asn
   930 935 940
   Tyr Ile His Asn Glu Tyr Thr Ile Ile Asn Cys Met Lys Asn Asn Ser
   945 950 955 960
   Gly Trp Lys Ile Ser Ile Arg Gly Asn Arg Ile Ile Trp Thr Leu Ile
   965 970 975
   Asp Ile Asn Gly Lys Thr Lys Ser Val Phe Phe Glu Tyr Asn Ile Arg
   980 985 990
   Glu Asp Ile Ser Glu Tyr Ile Asn Arg Trp Phe Phe Val Thr Ile Thr
   995 1000 1005
   Asn Asn Leu Asn Asn Ala Lys Ile Tyr Ile Asn Gly Lys Leu Glu
   1010 1015 1020
   Ser Asn Thr Asp Ile Lys Asp Ile Arg Glu Val Ile Ala Asn Gly
   1025 1030 1035
   Glu Ile Ile Phe Lys Leu Asp Gly Asp Ile Asp Arg Thr Gln Phe
   1040 1045 1050
   Ile Trp Met Lys Tyr Phe Ser Ile Phe Asn Thr Glu Leu Ser Gln
   1055 1060 1065
   Ser Asn Ile Glu Glu Arg Tyr Lys Ile Gln Ser Tyr Ser Glu Tyr
   1070 1075 1080
   Leu Lys Asp Phe Trp Gly Asn Pro Leu Met Tyr Asn Lys Glu Tyr
   1085 1090 1095
   Tyr Met Phe Asn Ala Gly Asn Lys Asn Ser Tyr Ile Lys Leu Lys
   1100 1105 1110
   Lys Asp Ser Pro Val Gly Glu Ile Leu Thr Arg Ser Lys Tyr Asn
   1115 1120 1125
   Gln Asn Ser Lys Tyr Ile Asn Tyr Arg Asp Leu Tyr Ile Gly Glu
   1130 1135 1140
   Lys Phe Ile Ile Arg Arg Lys Ser Asn Ser Gln Ser Ile Asn Asp
   1145 1150 1155
   Asp Ile Val Arg Lys Glu Asp Tyr Ile Tyr Leu Asp Phe Phe Asn
   1160 1165 1170
   Leu Asn Gln Glu Trp Arg Val Tyr Thr Tyr Lys Tyr Phe Lys Lys
   1175 1180 1185
   Glu Glu Glu Lys Leu Phe Leu Ala Pro Ile Ser Asp Ser Asp Glu
   1190 1195 1200
   Phe Tyr Asn Thr Ile Gln Ile Lys Glu Tyr Asp Glu Gln Pro Thr
   1205 1210 1215
   Tyr Ser Cys Gln Leu Leu Phe Lys Lys Asp Glu Glu Ser Thr Asp
   1220 1225 1230
   Glu Ile Gly Leu Ile Gly Ile His Arg Phe Tyr Glu Ser Gly Ile
   1235 1240 1245
   Val Phe Glu Glu Tyr Lys Asp Tyr Phe Cys Ile Ser Lys Trp Tyr
   1250 1255 1260
   Leu Lys Glu Val Lys Arg Lys Pro Tyr Asn Leu Lys Leu Gly Cys
   1265 1270 1275
   Asn Trp Gln Phe Ile Pro Lys Asp Glu Gly Trp Thr Glu
   1280 1285 1290
<210> 5
   <211> 3843
   <212> DNA
   <213> Clostridium botulinum
<400> 5
   atgccaataa caattaacaa ctttaattat tcagatcctg ttgataataa aaatatttta 60
   tatttagata ctcatttaaa tacattagct aatgagcctg aaaaagcctt tcgcattata 120
   gggaatatat gggtaatacc cgatagattt tcaagagatt ctaatccaaa tttaaataaa 180
   cctcctcgag ttacaagccc taaaagtggt tattatgatc ctaattattt gagtactgat 240
   tctgaaaaag atacattttt aaaagaaatt ataaagttat ttaaaagaat taactctaga 300
   gaaataggag aagaattaat atatagactt gcaacagaca taccctttcc tgggaataac 360
   aatactccaa ttaatacttt tgattttgat gtagatttta acagtgttga tgttaaaact 420
   agacaaggta acaactgggt taaaactggt agtataaatc ctagtgttat aataactgga 480
   cctagagaaa acattataga cccagaaact tctacgttta aattaactaa caatactttt 540
   gcggcacaag aaggatttgg tgctttatca ataatttcaa tatcacctag atttatgcta 600
   acatatagta atgcaactaa taatgtagga gagggtagat tttctaagtc tgaattttgc 660
   atggatccaa tactaatttt aatgcatgaa cttaatcatg caatgcataa tttatatgga 720
   atagctatac caaatgatca aagaatttca tctgtaacta gtaatatttt ttattctcaa 780
   tataaggtga aattagagta tgcagaaata tatgcatttg gaggtccaac tatagacctt 840
   attcctaaaa gtgcaaggaa atattttgag gaaaaggcat tggattatta tagatccata 900
   gctaaaagac ttaatagtat aactactgca aatccttcaa gctttaataa atatatagga 960
   gaatataaac agaaacttat tagaaagtat agattcgtag tagaatcttc aggtgaagtt 1020
   gcagtagatc gtaataagtt tgctgagtta tataaagaac ttacacaaat atttacagaa 1080
   tttaactacg ctaaaatata taatgtacaa aataggaaaa tatatctttc aaatgtatat 1140
   actccggtta cggcaaatat attagacgat aatgtttatg atatacaaaa tggatttaac 1200
   atacctaaaa gtaatttaaa tgtactattt atgggtcaaa atttatctcg aaatccagca 1260
   ttaagaaaag tcaatcctga aaatatgctt tatttattta caaaattttg ccataaagca 1320
   atagatggta gatcattata taataaaaca ttagattgta gagagctttt agttaaaaat 1380
   actgacttac cctttatagg tgatattagt gatatcaaaa ctgatatatt tttaagcaaa 1440
   gatattaatg aagaaactga agttatagac tatccggaca atgtttcagt ggatcaagtt 1500
   attctcagta agaatacctc agaacatgga caactagatt tattataccc tattattgaa 1560
   ggtgagagtc aagtattacc gggagagaat caagtctttt atgataatag aactcaaaat 1620
   gttgattatt tgaattctta ttattaccta gaatctcaaa aactaagtga taatgttgaa 1680
   gattttactt ttacgacatc aattgaggaa gctttggata atagtggaaa agtatatact 1740
   tactttccta aactagctga taaagtaaat acgggtgttc aaggtggttt atttttaatg 1800
   tgggcaaatg atgtagttga agattttact acaaatattc taagaaaaga tacattagat 1860
   aaaatatcag atgtatcagc tattattccc tatataggac ctgcattaaa tataagtaat 1920
   tctgtaagaa ggggaaattt tactgaagca tttgcagtta ccggtgtaac tattttatta 1980
   gaagcgtttc aagaatttac aatacctgca cttggtgcat ttgtgattta tagtaaggtt 2040
   caagaaagaa acgagattat taaaactata gataattgtt tagaacaaag gattaaaaga 2100
   tggaaagatt catatgaatg gatgatagga acgtggttat ccaggattac tactcaattt 2160
   aataatataa gttatcaaat gtatgattct ttaaattatc aggcagatgc aatcaaagat 2220
   aaaatagatt tagaatataa aaaatactca ggaagtgata aagaaaatat aaaaagtcaa 2280
   gttgaaaatt taaaaaatag tttagatata aaaatctcgg aagcaatgaa taatataaat 2340
   aaatttatac gagaatgttc tgtaacatac ttatttaaaa atatgctccc taaagtaatt 2400
   gatgaattaa ataagtttga tttaaaaact aaaacagaat taattaatct tatagatagt 2460
   cataatatta ttctagttgg tgaagtagat agattaaaag caaaagtaaa tgagagtttt 2520
   gaaaatacaa taccctttaa tattttttca tatactaata attctttatt aaaagatata 2580
   attaatgaat atttcaatag tattaatgat tcaaaaattt tgagcttaca aaacaaaaaa 2640
   aatgctttag tggatacatc aggatataat gcagaagtga ggctagaagg tgatgttcaa 2700
   gttaatacga tatatacaaa tgattttaaa ttaagtagtt caggagataa aattatagta 2760
   aatttaaata ataatatttt atatagcgct atttatgaga actctagtgt tagtttttgg 2820
   attaagatat ctaaagattt aactaattct cataatgaat atacaataat taatagtata 2880
   aaacaaaatt ctgggtggaa attatgtatt aggaatggca atatagaatg gattttacaa 2940
   gatattaata gaaagtataa aagtttaatt tttgattata gtgaatcatt aagtcataca 3000
   ggatatacaa ataaatggtt ttttgttact ataactaata atataatggg gtatatgaaa 3060
   ctttatataa atggagaatt aaagcagagt gaaagaattg aagatttaaa tgaggttaag 3120
   ttagataaaa ccatagtatt tggaatagat gagaatatag atgagaatca gatgctttgg 3180
   attagagatt ttaatatttt ttctaaagaa ttaagcaatg aagatattaa tattgtatat 3240
   gagggacaaa tattaagaaa tgttattaaa gattattggg gaaatccttt gaagtttgat 3300
   acagaatatt atattattaa tgataattat atagataggt atatagcacc taaaagtaat 3360
   atacttgtac ttgttcagta tccagataga tctaaattat atactggaaa tcctattact 3420
   attaaatcag tatctgataa gaatccttat agtagaattt taaatggaga taatataatg 3480
   tttcatatgt tatataatag tgggaaatat atgataataa gagatactga tacaatatat 3540
   gcaatagaag gaagagagtg ttcaaaaaat tgtgtatatg cattaaaatt acagagtaat 3600
   ttaggtaatt atggtatagg tatatttagt ataaaaaata ttgtatctca aaataaatat 3660
   tgtagtcaaa ttttctctag ttttatgaaa aatacaatgc ttctagcaga tatatataaa 3720
   ccttggagat ttcttttgga aaatgcatac acgccagttg cagtaactaa ttatgagaca 3780
   aaactattat caacttcatc tttttggaaa tttatttcta gggatccagg atgggtagag 3840
   taa                                                                3843
<210> 6
   <211> 1280
   <212> PRT
   <213> Clostridium botulinum
<400> 6
   Met Pro Ile Thr Ile Asn Asn Phe Asn Tyr Ser Asp Pro Val Asp Asn
   1               5                   10                  15
   Lys Asn Ile Leu Tyr Leu Asp Thr His Leu Asn Thr Leu Ala Asn Glu
               20                  25                  30
   Pro Glu Lys Ala Phe Arg Ile Ile Gly Asn Ile Trp Val Ile Pro Asp
           35                  40                  45
   Arg Phe Ser Arg Asp Ser Asn Pro Asn Leu Asn Lys Pro Pro Arg Val
       50                  55                  60
   Thr Ser Pro Lys Ser Gly Tyr Tyr Asp Pro Asn Tyr Leu Ser Thr Asp
   65                  70                  75                  80
   Ser Glu Lys Asp Thr Phe Leu Lys Glu Ile Ile Lys Leu Phe Lys Arg
                   85                  90                  95
   Ile Asn Ser Arg Glu Ile Gly Glu Glu Leu Ile Tyr Arg Leu Ala Thr
               100                 105                 110
   Asp Ile Pro Phe Pro Gly Asn Asn Asn Thr Pro Ile Asn Thr Phe Asp
           115                 120                 125
   Phe Asp Val Asp Phe Asn Ser Val Asp Val Lys Thr Arg Gln Gly Asn
       130                 135                 140
   Asn Trp Val Lys Thr Gly Ser Ile Asn Pro Ser Val Ile Ile Thr Gly
   145                 150                 155                 160
   Pro Arg Glu Asn Ile Ile Asp Pro Glu Thr Ser Thr Phe Lys Leu Thr
                   165                 170                 175
   Asn Asn Thr Phe Ala Ala Gln Glu Gly Phe Gly Ala Leu Ser Ile Ile
               180                 185                 190
   Ser Ile Ser Pro Arg Phe Met Leu Thr Tyr Ser Asn Ala Thr Asn Asn
           195                 200                 205
   Val Gly Glu Gly Arg Phe Ser Lys Ser Glu Phe Cys Met Asp Pro Ile
       210                 215                 220
   Leu Ile Leu Met His Glu Leu Asn His Ala Met His Asn Leu Tyr Gly
   225                 230                 235                 240
   Ile Ala Ile Pro Asn Asp Gln Arg Ile Ser Ser Val Thr Ser Asn Ile
                   245                 250                 255
   Phe Tyr Ser Gln Tyr Lys Val Lys Leu Glu Tyr Ala Glu Ile Tyr Ala
               260                 265                 270
   Phe Gly Gly Pro Thr Ile Asp Leu Ile Pro Lys Ser Ala Arg Lys Tyr
   275 280 285
   Phe Glu Glu Lys Ala Leu Asp Tyr Tyr Arg Ser Ile Ala Lys Arg Leu
   290 295 300
   Asn Ser Ile Thr Thr Ala Asn Pro Ser Ser Phe Asn Lys Tyr Ile Gly
   305 310 315 320
   Glu Tyr Lys Gln Lys Leu Ile Arg Lys Tyr Arg Phe Val Val Glu Ser
   325 330 335
   Ser Gly Glu Val Ala Val Asp Arg Asn Lys Phe Ala Glu Leu Tyr Lys
   340 345 350
   Glu Leu Thr Gln Ile Phe Thr Glu Phe Asn Tyr Ala Lys Ile Tyr Asn
   355 360 365
   Val Gln Asn Arg Lys Ile Tyr Leu Ser Asn Val Tyr Thr Pro Val Thr
   370 375 380
   Ala Asn Ile Leu Asp Asp Asn Val Tyr Asp Ile Gln Asn Gly Phe Asn
   385 390 395 400
   Ile Pro Lys Ser Asn Leu Asn Val Leu Phe Met Gly Gln Asn Leu Ser
   405 410 415
   Arg Asn Pro Ala Leu Arg Lys Val Asn Pro Glu Asn Met Leu Tyr Leu
   420 425 430
   Phe Thr Lys Phe Cys His Lys Ala Ile Asp Gly Arg Ser Leu Tyr Asn
   435 440 445
   Lys Thr Leu Asp Cys Arg Glu Leu Leu Val Lys Asn Thr Asp Leu Pro
   450 455 460
   Phe Ile Gly Asp Ile Ser Asp Ile Lys Thr Asp Ile Phe Leu Ser Lys
   465 470 475 480
   Asp Ile Asn Glu Glu Thr Glu Val Ile Asp Tyr Pro Asp Asn Val Ser
   485 490 495
   Val Asp Gln Val Ile Leu Ser Lys Asn Thr Ser Glu His Gly Gln Leu
   500 505 510
   Asp Leu Leu Tyr Pro Ile Ile Glu Gly Glu Ser Gln Val Leu Pro Gly
   515 520 525
   Glu Asn Gln Val Phe Tyr Asp Asn Arg Thr Gln Asn Val Asp Tyr Leu
   530 535 540
   Asn Ser Tyr Tyr Tyr Leu Glu Ser Gln Lys Leu Ser Asp Asn Val Glu
   545 550 555 560
   Asp Phe Thr Phe Thr Thr Ser Ile Glu Glu Ala Leu Asp Asn Ser Gly
   565 570 575
   Lys Val Tyr Thr Tyr Phe Pro Lys Leu Ala Asp Lys Val Asn Thr Gly
   580 585 590
   Val Gln Gly Gly Leu Phe Leu Met Trp Ala Asn Asp Val Val Glu Asp
   595 600 605
   Phe Thr Thr Asn Ile Leu Arg Lys Asp Thr Leu Asp Lys Ile Ser Asp
   610 615 620
   Val Ser Ala Ile Ile Pro Tyr Ile Gly Pro Ala Leu Asn Ile Ser Asn
   625 630 635 640
   Ser Val Arg Arg Gly Asn Phe Thr Glu Ala Phe Ala Val Thr Gly Val
   645 650 655
   Thr Ile Leu Leu Glu Ala Phe Gln Glu Phe Thr Ile Pro Ala Leu Gly
   660 665 670
   Ala Phe Val Ile Tyr Ser Lys Val Gln Glu Arg Asn Glu Ile Ile Lys
   675 680 685
   Thr Ile Asp Asn Cys Leu Glu Gln Arg Ile Lys Arg Trp Lys Asp Ser
   690 695 700
   Tyr Glu Trp Met Ile Gly Thr Trp Leu Ser Arg Ile Thr Thr Gln Phe
   705 710 715 720
   Asn Asn Ile Ser Tyr Gln Met Tyr Asp Ser Leu Asn Tyr Gln Ala Asp
   725 730 735
   Ala Ile Lys Asp Lys Ile Asp Leu Glu Tyr Lys Lys Tyr Ser Gly Ser
   740 745 750
   Asp Lys Glu Asn Ile Lys Ser Gln Val Glu Asn Leu Lys Asn Ser Leu
   755 760 765
   Asp Ile Lys Ile Ser Glu Ala Met Asn Asn Ile Asn Lys Phe Ile Arg
   770 775 780
   Glu Cys Ser Val Thr Tyr Leu Phe Lys Asn Met Leu Pro Lys Val Ile
   785 790 795 800
   Asp Glu Leu Asn Lys Phe Asp Leu Lys Thr Lys Thr Glu Leu Ile Asn
   805 810 815
   Leu Ile Asp Ser His Asn Ile Ile Leu Val Gly Glu Val Asp Arg Leu
   820 825 830
   Lys Ala Lys Val Asn Glu Ser Phe Glu Asn Thr Ile Pro Phe Asn Ile
   835 840 845
   Phe Ser Tyr Thr Asn Asn Ser Leu Leu Lys Asp Ile Ile Asn Glu Tyr
   850 855 860
   Phe Asn Ser Ile Asn Asp Ser Lys Ile Leu Ser Leu Gln Asn Lys Lys
   865 870 875 880
   Asn Ala Leu Val Asp Thr Ser Gly Tyr Asn Ala Glu Val Arg Leu Glu
   885 890 895
   Gly Asp Val Gln Val Asn Thr Ile Tyr Thr Asn Asp Phe Lys Leu Ser
   900 905 910
   Ser Ser Gly Asp Lys Ile Ile Val Asn Leu Asn Asn Asn Ile Leu Tyr
   915 920 925
   Ser Ala Ile Tyr Glu Asn Ser Ser Val Ser Phe Trp Ile Lys Ile Ser
   930 935 940
   Lys Asp Leu Thr Asn Ser His Asn Glu Tyr Thr Ile Ile Asn Ser Ile
   945 950 955 960
   Lys Gln Asn Ser Gly Trp Lys Leu Cys Ile Arg Asn Gly Asn Ile Glu
   965 970 975
   Trp Ile Leu Gln Asp Ile Asn Arg Lys Tyr Lys Ser Leu Ile Phe Asp
   980 985 990
   Tyr Ser Glu Ser Leu Ser His Thr Gly Tyr Thr Asn Lys Trp Phe Phe
   995 1000 1005
   Val Thr Ile Thr Asn Asn Ile Met Gly Tyr Met Lys Leu Tyr Ile
   1010 1015 1020
   Asn Gly Glu Leu Lys Gln Ser Glu Arg Ile Glu Asp Leu Asn Glu
   1025 1030 1035
   Val Lys Leu Asp Lys Thr Ile Val Phe Gly Ile Asp Glu Asn Ile
   1040 1045 1050
   Asp Glu Asn Gln Met Leu Trp Ile Arg Asp Phe Asn Ile Phe Ser
   1055 1060 1065
   Lys Glu Leu Ser Asn Glu Asp Ile Asn Ile Val Tyr Glu Gly Gln
   1070 1075 1080
   Ile Leu Arg Asn Val Ile Lys Asp Tyr Trp Gly Asn Pro Leu Lys
   1085 1090 1095
   Phe Asp Thr Glu Tyr Tyr Ile Ile Asn Asp Asn Tyr Ile Asp Arg
   1100 1105 1110
   Tyr Ile Ala Pro Lys Ser Asn Ile Leu Val Leu Val Gln Tyr Pro
   1115 1120 1125
   Asp Arg Ser Lys Leu Tyr Thr Gly Asn Pro Ile Thr Ile Lys Ser
   1130 1135 1140
   Val Ser Asp Lys Asn Pro Tyr Ser Arg Ile Leu Asn Gly Asp Asn
   1145 1150 1155
   Ile Met Phe His Met Leu Tyr Asn Ser Gly Lys Tyr Met Ile Ile
   1160 1165 1170
   Arg Asp Thr Asp Thr Ile Tyr Ala Ile Glu Gly Arg Glu Cys Ser
   1175 1180 1185
   Lys Asn Cys Val Tyr Ala Leu Lys Leu Gln Ser Asn Leu Gly Asn
   1190 1195 1200
   Tyr Gly Ile Gly Ile Phe Ser Ile Lys Asn Ile Val Ser Gln Asn
   1205 1210 1215
   Lys Tyr Cys Ser Gln Ile Phe Ser Ser Phe Met Lys Asn Thr Met
   1220 1225 1230
   Leu Leu Ala Asp Ile Tyr Lys Pro Trp Arg Phe Ser Phe Glu Asn
   1235 1240 1245
   Ala Tyr Thr Pro Val Ala Val Thr Asn Tyr Glu Thr Lys Leu Leu
   1250 1255 1260
   Ser Thr Ser Ser Phe Trp Lys Phe Ile Ser Arg Asp Pro Gly Trp
   1265 1270 1275
   Val Glu
   1280
<210> 7
   <211> 3858
   <212> DNA
   <213> Clostridium botulinum
<400> 7
   atgacatggc cagtaaaaga ttttaattat agtgatcctg ttaatgacaa tgatatatta 60
   tatttaagaa taccacaaaa taagttaatt actacacctg taaaagcttt tatgattact 120
   caaaatattt gggtaatacc agaaagattt tcatcagata ctaatccaag tttaagtaaa 180
   ccgcctagac ctacttcaaa gtatcaaagt tattatgatc ctagttattt atctactgat 240
   gagcaaaaag atacattttt aaaagggatt ataaaattat ttaaaagaat taatgaaaga 300
   gatataggaa aaaaattaat aaattattta gtagttggtt caccttttat gggagattca 360
   agtacgcctg aagatacatt tgattttaca cgtcatacta ctaatattgc agttgaaaag 420
   tttgaaaatg gtagttggaa agtaacaaat attataacac caagtgtatt gatatttgga 480
   ccacttccta atatattaga ctatacagca tcccttacat tgcaaggaca acaatcaaat 540
   ccatcatttg aagggtttgg aacattatct atactaaaag tagcacctga atttttgtta 600
   acatttagtg atgtaacatc taatcaaagt tcagctgtat taggcaaatc tatattttgt 660
   atggatccag taatagcttt aatgcatgag ttaacacatt ctttgcatca attgtatgga 720
   ataaatatac catctgataa aaggattcgt ccacaagtta gcgagggatt tttttctcaa 780
   gatggaccca acgtacaatt tgaggaatta tacacatttg gaggatcaga tgttgaaata 840
   atacctcaaa ttgaaagatt acaattaaga gaaaaagcat taggtcacta taaagatata 900
   gcgaaaagac ttaataatat taataaaact attccttcta gttggagtag taatatagat 960
   aaatataaaa aaatattttc tgaaaagtat aattttgata aagataatac aggaaatttt 1020
   gttgtaaata ttgataaatt caatagctta tattcagact tgactaatgt tatgtcagaa 1080
   gttgtttatt cttcgcaata taatgttaaa aacaggactc attatttttc aaagcattat 1140
   ctacctgtat ttgcaaatat attagatgat aatatttata ctataataaa cggttttaat 1200
   ttaacaacta aaggttttaa tatagaaaat tcgggtcaga atatagaaag gaatcctgca 1260
   ctacaaaaac ttagttcaga aagtgtagta gatttgttta caaaagtatg tttaagatta 1320
   acaagaaata gtagagatga ttcaacatgt attcaagtta aaaataatac attaccttat 1380
   gtagctgata aagatagcat ttcacaagaa atatttgaaa gtcaaattat tacagatgag 1440
   actaatgtag aaaattattc agataatttt tcattagatg aatctatttt agatgcaaaa 1500
   gtccctacta atcctgaagc agtagatcca ctgttaccca atgttaatat ggaaccttta 1560
   aatgttccag gtgaagaaga agtattttat gatgatatta ctaaagatgt tgattattta 1620
   aactcttatt attatttgga agcccaaaaa ttaagtaata atgttgaaaa tattactctt 1680
   acaacttcag ttgaagaagc attaggttat agcaataaga tatacacatt tttacctagc 1740
   ttagctgaaa aagtgaataa aggtgttcaa gcaggtttat tcttaaattg ggcgaatgaa 1800
   gtagttgagg attttactac aaatattatg aaaaaagata cattggataa aatatcagat 1860
   gtatcagcca taattccata tataggacct gccttaaata taggaaattc agcattaagg 1920
   ggaaacttta agcaagcatt tgcaacagct ggtgtagctt ttttgttaga aggatttcca 1980
   gagtttacaa tacctgcact cggtgtattt accttttata gttctattca agaaagagag 2040
   aaaattatta aaactataga aaattgttta gaacaaagag ttaagagatg gaaagattca 2100
   tatcaatgga tggtatcaaa ttggttgtca agaattacta ctcgatttaa tcatataagt 2160
   tatcaaatgt atgattcttt gagttatcag gcagatgcaa tcaaagctaa aatagattta 2220
   gaatataaaa aatactcagg aagtgataaa gaaaatataa aaagtcaagt tgaaaattta 2280
   aaaaatagtt tagatgtaaa aatctcggaa gcaatgaata atataaataa atttatacga 2340
   gaatgttctg taacatactt atttaaaaat atgctcccta aagtaattga tgaattaaat 2400
   aagtttgatt taaaaactaa aacagaatta attaatctta tagatagtca taatattatt 2460
   ctagttggtg aagtagatag attaaaagca aaagtaaatg agagttttga aaatacaata 2520
   ccctttaata ttttttcata tactaataat tctttattaa aagatatgat taatgaatat 2580
   ttcaatagta ttaatgattc aaaaattttg agcttacaaa ataaaaaaaa tactttgatg 2640
   gatacatcag gatataacgc agaagtgaga gtagaaggca atgttcagct taatccaata 2700
   tttccatttg actttaaatt aggtagttca ggggatgata gaggtaaagt tatagtaacc 2760
   cagaatgaaa atattgtata taatgctatg tatgaaagtt ttagtattag tttttggatt 2820
   aggataaata aatgggtaag taatttacct ggatatacta taattgatag tgttaaaaat 2880
   aactcaggtt ggagtatagg tattattagt aattttttag tgtttacttt aaaacaaaat 2940
   gaaaatagtg aacaagatat aaactttagt tatgatatat caaagaatgc tgcgggatat 3000
   aataaatggt tttttgtaac tattactacc aatatgatgg gaaatatgat gatttatata 3060
   aatggaaaat taatagatac tataaaagtt aaagagttaa ctggaattaa ttttagcaaa 3120
   actataacat ttcaaatgaa taaaattcca aatactggct taattacctc agattctgat 3180
   aacatcaata tgtggataag ggatttttat atctttgcta aagaattaga tgataaagat 3240
   attaatatat tatttaatag cttgcaatat actaatgttg taaaagatta ttggggaaat 3300
   gatttaagat atgataaaga atattacatg attaacgtaa attatatgaa tagatatatg 3360
   tctaaaaaag gcaatggaat tgtttttaat acacgtaaaa ataataatga cttcaatgaa 3420
   ggatataaaa ttataataaa aagaattaga ggaaatacaa atgatactag agtacgagga 3480
   gaaaatgtat tatattttaa tactacaatt gataacaaac aatatagttt aggtatgtat 3540
   aaaccttcta gaaatctagg gactgattta gttccactag gtgcattgga tcaaccaatg 3600
   gatgagatac gtaaatatgg ttcgtttata atacaaccat gcaatacttt tgattactat 3660
   gcatcacaat tatttttgtc aagtaatgca acaacaaata ggcttggaat actatcaatt 3720
   ggtagttata gtttcaaact tggagatgac tattggttta atcacgaata tttaattcct 3780
   gttataaaaa tagagcatta tgcttcatta ttagaatcaa catcaactca ttgggttttt 3840
   gtacctgcaa gtgaataa                                                3858
<210> 8
   <211> 1285
   <212> PRT
   <213> Clostridium botulinum
<400> 8
   Met Thr Trp Pro Val Lys Asp Phe Asn Tyr Ser Asp Pro Val Asn Asp
   1               5                   10                  15
   Asn Asp Ile Leu Tyr Leu Arg Ile Pro Gln Asn Lys Leu Ile Thr Thr
               20                  25                  30
   Pro Val Lys Ala Phe Met Ile Thr Gln Asn Ile Trp Val Ile Pro Glu
           35                  40                  45
   Arg Phe Ser Ser Asp Thr Asn Pro Ser Leu Ser Lys Pro Pro Arg Pro
       50                  55                  60
   Thr Ser Lys Tyr Gln Ser Tyr Tyr Asp Pro Ser Tyr Leu Ser Thr Asp
   65                  70                  75                  80
   Glu Gln Lys Asp Thr Phe Leu Lys Gly Ile Ile Lys Leu Phe Lys Arg
                   85                  90                  95
   Ile Asn Glu Arg Asp Ile Gly Lys Lys Leu Ile Asn Tyr Leu Val Val
               100                 105                 110
   Gly Ser Pro Phe Met Gly Asp Ser Ser Thr Pro Glu Asp Thr Phe Asp
           115                 120                 125
   Phe Thr Arg His Thr Thr Asn Ile Ala Val Glu Lys Phe Glu Asn Gly
       130                 135                 140
   Ser Trp Lys Val Thr Asn Ile Ile Thr Pro Ser Val Leu Ile Phe Gly
   145                 150                 155                 160
   Pro Leu Pro Asn Ile Leu Asp Tyr Thr Ala Ser Leu Thr Leu Gln Gly
                   165                 170                 175
   Gln Gln Ser Asn Pro Ser Phe Glu Gly Phe Gly Thr Leu Ser Ile Leu
               180                 185                 190
   Lys Val Ala Pro Glu Phe Leu Leu Thr Phe Ser Asp Val Thr Ser Asn
           195                 200                 205
   Gln Ser Ser Ala Val Leu Gly Lys Ser Ile Phe Cys Met Asp Pro Val
       210                 215                 220
   Ile Ala Leu Met His Glu Leu Thr His Ser Leu His Gln Leu Tyr Gly
   225                 230                 235                 240
   Ile Asn Ile Pro Ser Asp Lys Arg Ile Arg Pro Gln Val Ser Glu Gly
                   245                 250                 255
   Phe Phe Ser Gln Asp Gly Pro Asn Val Gln Phe Glu Glu Leu Tyr Thr
               260                 265                 270
   Phe Gly Gly Ser Asp Val Glu Ile Ile Pro Gln Ile Glu Arg Leu Gln
           275                 280                 285
   Leu Arg Glu Lys Ala Leu Gly His Tyr Lys Asp Ile Ala Lys Arg Leu
       290                 295                 300
   Asn Asn Ile Asn Lys Thr Ile Pro Ser Ser Trp Ser Ser Asn Ile Asp
   305                 310                 315                 320
   Lys Tyr Lys Lys Ile Phe Ser Glu Lys Tyr Asn Phe Asp Lys Asp Asn
   325 330 335
   Thr Gly Asn Phe Val Val Asn Ile Asp Lys Phe Asn Ser Leu Tyr Ser
   340 345 350
   Asp Leu Thr Asn Val Met Ser Glu Val Val Tyr Ser Ser Gln Tyr Asn
   355 360 365
   Val Lys Asn Arg Thr His Tyr Phe Ser Lys His Tyr Leu Pro Val Phe
   370 375 380
   Ala Asn Ile Leu Asp Asp Asn Ile Tyr Thr Ile Ile Asn Gly Phe Asn
   385 390 395 400
   Leu Thr Thr Lys Gly Phe Asn Ile Glu Asn Ser Gly Gln Asn Ile Glu
   405 410 415
   Arg Asn Pro Ala Leu Gln Lys Leu Ser Ser Glu Ser Val Val Asp Leu
   420 425 430
   Phe Thr Lys Val Cys Leu Arg Leu Thr Arg Asn Ser Arg Asp Asp Ser
   435 440 445
   Thr Cys Ile Gln Val Lys Asn Asn Thr Leu Pro Tyr Val Ala Asp Lys
   450 455 460
   Asp Ser Ile Ser Gln Glu Ile Phe Glu Ser Gln Ile Ile Thr Asp Glu
   465 470 475 480
   Thr Asn Val Glu Asn Tyr Ser Asp Asn Phe Ser Leu Asp Glu Ser Ile
   485 490 495
   Leu Asp Ala Lys Val Pro Thr Asn Pro Glu Ala Val Asp Pro Leu Leu
   500 505 510
   Pro Asn Val Asn Met Glu Pro Leu Asn Val Pro Gly Glu Glu Glu Val
   515 520 525
   Phe Tyr Asp Asp Ile Thr Lys Asp Val Asp Tyr Leu Asn Ser Tyr Tyr
   530 535 540
   Tyr Leu Glu Ala Gln Lys Leu Ser Asn Asn Val Glu Asn Ile Thr Leu
   545 550 555 560
   Thr Thr Ser Val Glu Glu Ala Leu Gly Tyr Ser Asn Lys Ile Tyr Thr
   565 570 575
   Phe Leu Pro Ser Leu Ala Glu Lys Val Asn Lys Gly Val Gln Ala Gly
   580 585 590
   Leu Phe Leu Asn Trp Ala Asn Glu Val Val Glu Asp Phe Thr Thr Asn
   595 600 605
   Ile Met Lys Lys Asp Thr Leu Asp Lys Ile Ser Asp Val Ser Ala Ile
   610 615 620
   Ile Pro Tyr Ile Gly Pro Ala Leu Asn Ile Gly Asn Ser Ala Leu Arg
   625 630 635 640
   Gly Asn Phe Lys Gln Ala Phe Ala Thr Ala Gly Val Ala Phe Leu Leu
   645 650 655
   Glu Gly Phe Pro Glu Phe Thr Ile Pro Ala Leu Gly Val Phe Thr Phe
   660 665 670
   Tyr Ser Ser Ile Gln Glu Arg Glu Lys Ile Ile Lys Thr Ile Glu Asn
   675 680 685
   Cys Leu Glu Gln Arg Val Lys Arg Trp Lys Asp Ser Tyr Gln Trp Met
   690 695 700
   Val Ser Asn Trp Leu Ser Arg Ile Thr Thr Arg Phe Asn His Ile Ser
   705 710 715 720
   Tyr Gln Met Tyr Asp Ser Leu Ser Tyr Gln Ala Asp Ala Ile Lys Ala
   725 730 735
   Lys Ile Asp Leu Glu Tyr Lys Lys Tyr Ser Gly Ser Asp Lys Glu Asn
   740 745 750
   Ile Lys Ser Gln Val Glu Asn Leu Lys Asn Ser Leu Asp Val Lys Ile
   755 760 765
   Ser Glu Ala Met Asn Asn Ile Asn Lys Phe Ile Arg Glu Cys Ser Val
   770 775 780
   Thr Tyr Leu Phe Lys Asn Met Leu Pro Lys Val Ile Asp Glu Leu Asn
   785 790 795 800
   Lys Phe Asp Leu Lys Thr Lys Thr Glu Leu Ile Asn Leu Ile Asp Ser
   805 810 815
   His Asn Ile Ile Leu Val Gly Glu Val Asp Arg Leu Lys Ala Lys Val
   820 825 830
   Asn Glu Ser Phe Glu Asn Thr Ile Pro Phe Asn Ile Phe Ser Tyr Thr
   835 840 845
   Asn Asn Ser Leu Leu Lys Asp Met Ile Asn Glu Tyr Phe Asn Ser Ile
   850 855 860
   Asn Asp Ser Lys Ile Leu Ser Leu Gln Asn Lys Lys Asn Thr Leu Met
   865 870 875 880
   Asp Thr Ser Gly Tyr Asn Ala Glu Val Arg Val Glu Gly Asn Val Gln
   885 890 895
   Leu Asn Pro Ile Phe Pro Phe Asp Phe Lys Leu Gly Ser Ser Gly Asp
   900 905 910
   Asp Arg Gly Lys Val Ile Val Thr Gln Asn Glu Asn Ile Val Tyr Asn
   915 920 925
   Ala Met Tyr Glu Ser Phe Ser Ile Ser Phe Trp Ile Arg Ile Asn Lys
   930 935 940
   Trp Val Ser Asn Leu Pro Gly Tyr Thr Ile Ile Asp Ser Val Lys Asn
   945 950 955 960
   Asn Ser Gly Trp Ser Ile Gly Ile Ile Ser Asn Phe Leu Val Phe Thr
   965 970 975
   Leu Lys Gln Asn Glu Asn Ser Glu Gln Asp Ile Asn Phe Ser Tyr Asp
   980 985 990
   Ile Ser Lys Asn Ala Ala Gly Tyr Asn Lys Trp Phe Phe Val Thr Ile
   995 1000 1005
   Thr Thr Asn Met Met Gly Asn Met Met Ile Tyr Ile Asn Gly Lys
   1010 1015 1020
   Leu Ile Asp Thr Ile Lys Val Lys Glu Leu Thr Gly Ile Asn Phe
   1025 1030 1035
   Ser Lys Thr Ile Thr Phe Gln Met Asn Lys Ile Pro Asn Thr Gly
   1040 1045 1050
   Leu Ile Thr Ser Asp Ser Asp Asn Ile Asn Met Trp Ile Arg Asp
   1055 1060 1065
   Phe Tyr Ile Phe Ala Lys Glu Leu Asp Asp Lys Asp Ile Asn Ile
   1070 1075 1080
   Leu Phe Asn Ser Leu Gln Tyr Thr Asn Val Val Lys Asp Tyr Trp
   1085 1090 1095
   Gly Asn Asp Leu Arg Tyr Asp Lys Glu Tyr Tyr Met Ile Asn Val
   1100 1105 1110
   Asn Tyr Met Asn Arg Tyr Met Ser Lys Lys Gly Asn Gly Ile Val
   1115 1120 1125
   Phe Asn Thr Arg Lys Asn Asn Asn Asp Phe Asn Glu Gly Tyr Lys
   1130 1135 1140
   Ile Ile Ile Lys Arg Ile Arg Gly Asn Thr Asn Asp Thr Arg Val
   1145 1150 1155
   Arg Gly Glu Asn Val Leu Tyr Phe Asn Thr Thr Ile Asp Asn Lys
   1160 1165 1170
   Gln Tyr Ser Leu Gly Met Tyr Lys Pro Ser Arg Asn Leu Gly Thr
   1175 1180 1185
   Asp Leu Val Pro Leu Gly Ala Leu Asp Gln Pro Met Asp Glu Ile
   1190 1195 1200
   Arg Lys Tyr Gly Ser Phe Ile Ile Gln Pro Cys Asn Thr Phe Asp
   1205 1210 1215
   Tyr Tyr Ala Ser Gln Leu Phe Leu Ser Ser Asn Ala Thr Thr Asn
   1220 1225 1230
   Arg Leu Gly Ile Leu Ser Ile Gly Ser Tyr Ser Phe Lys Leu Gly
   1235 1240 1245
   Asp Asp Tyr Trp Phe Asn His Glu Tyr Leu Ile Pro Val Ile Lys
   1250 1255 1260
   Ile Glu His Tyr Ala Ser Leu Leu Glu Ser Thr Ser Thr His Trp
   1265 1270 1275
   Val Phe Val Pro Ala Ser Glu
   1280 1285
<210> 9
   <211> 3756
   <212> DNA
   <213> Clostridium botulinum
<400> 9
   atgccaaaaa ttaatagttt taattataat gatcctgtta atgatagaac aattttatat     60
   attaaaccag gcggttgtca agaattttat aaatcattta atattatgaa aaatatttgg    120
   ataattccag agagaaatgt aattggtaca accccccaag attttcatcc gcctacttca    180
   ttaaaaaatg gagatagtag ttattatgac cctaattatt tacaaagtga tgaagaaaag    240
   gatagatttt taaaaatagt cacaaaaata tttaatagaa taaataataa tctttcagga    300
   gggattttat tagaagaact gtcaaaagct aatccatatt tagggaatga taatactcca    360
   gataatcaat tccatattgg tgatgcatca gcagttgaga ttaaattctc aaatggtagc    420
   caagacatac tattacctaa tgttattata atgggagcag agcctgattt atttgaaact    480
   aacagttcca atatttctct aagaaataat tatatgccaa gcaatcaccg ttttggatca    540
   atagctatag taacattctc acctgaatat tcttttagat ttaatgataa ttgtatgaat    600
   gaatttattc aagatcctgc tcttacatta atgcatgaat taatacattc attacatgga    660
   ctatatgggg ctaaagggat tactacaaag tatactataa cacaaaaaca aaatccccta    720
   ataacaaata taagaggtac aaatattgaa gaattcttaa cttttggagg tactgattta 780
   aacattatta ctagtgctca gtccaatgat atctatacta atcttctagc tgattataaa 840
   aaaatagcgt ctaaacttag caaagtacaa gtatctaatc cactacttaa tccttataaa 900
   gatgtttttg aagcaaagta tggattagat aaagatgcta gcggaattta ttcggtaaat 960
   ataaacaaat ttaatgatat ttttaaaaaa ttatacagct ttacggaatt tgatttacga 1020
   actaaatttc aagttaaatg taggcaaact tatattggac agtataaata cttcaaactt 1080
   tcaaacttgt taaatgattc tatttataat atatcagaag gctataatat aaataattta 1140
   aaggtaaatt ttagaggaca gaatgcaaat ttaaatccta gaattattac accaattaca 1200
   ggtagaggac tagtaaaaaa aatcattaga ttttgtaaaa atattgtttc tgtaaaaggc 1260
   ataaggaaat caatatgtat cgaaataaat aatggtgagt tattttttgt ggcttccgag 1320
   aatagttata atgatgataa tataaatact cctaaagaaa ttgacgatac agtaacttca 1380
   aataataatt atgaaaatga tttagatcag gttattttaa attttaatag tgaatcagca 1440
   cctggacttt cagatgaaaa attaaattta actatccaaa atgatgctta tataccaaaa 1500
   tatgattcta atggaacaag tgatatagaa caacatgatg ttaatgaact taatgtattt 1560
   ttctatttag atgcacagaa agtgcccgaa ggtgaaaata atgtcaatct cacctcttca 1620
   attgatacag cattattaga acaacctaaa atatatacat ttttttcatc agaatttatt 1680
   aataatgtca ataaacctgt gcaagcagca ttatttgtaa gctggataca acaagtgtta 1740
   gtagatttta ctactgaagc taaccaaaaa agtactgttg ataaaattgc agatatttct 1800
   atagttgttc catatatagg tcttgcttta aatataggaa atgaagcaca aaaaggaaat 1860
   tttaaagatg cacttgaatt attaggagca ggtattttat tagaatttga acccgagctt 1920
   ttaattccta caattttagt attcacgata aaatcttttt taggttcatc tgataataaa 1980
   aataaagtta ttaaagcaat aaataatgca ttgaaagaaa gagatgaaaa atggaaagaa 2040
   gtatatagtt ttatagtatc gaattggatg actaaaatta atacacaatt taataaaaga 2100
   aaagaacaaa tgtatcaagc tttacaaaat caagtaaatg caattaaaac aataatagaa 2160
   tctaagtata atagttatac tttagaggaa aaaaatgagc ttacaaataa atatgatatt 2220
   aagcaaatag aaaatgaact taatcaaaag gtttctatag caatgaataa tatagacagg 2280
   ttcttaactg aaagttctat atcctattta atgaaaataa taaatgaagt aaaaattaat 2340
   aaattaagag aatatgatga gaatgtcaaa acgtatttat tgaattatat tatacaacat 2400
   ggatcaatct tgggagagag tcagcaagaa ctaaattcta tggtaactga taccctaaat 2460
   aatagtattc cttttaagct ttcttcttat acagatgata aaattttaat ttcatatttt 2520
   aataaattct ttaagagaat taaaagtagt tcagttttaa atatgagata taaaaatgat 2580
   aaatacgtag atacttcagg atatgattca aatataaata ttaatggaga tgtatataaa 2640
   tatccaacta ataaaaatca atttggaata tataatgata aacttagtga agttaatata 2700
   tctcaaaatg attacattat atatgataat aaatataaaa attttagtat tagtttttgg 2760
   gtaagaattc ctaactatga taataagata gtaaatgtta ataatgaata cactataata 2820
   aattgtatga gagataataa ttcaggatgg aaagtatctc ttaatcataa tgaaataatt 2880
   tggacattcg aagataatcg aggaattaat caaaaattag catttaacta tggtaacgca 2940
   aatggtattt ctgattatat aaataagtgg atttttgtaa ctataactaa tgatagatta 3000
   ggagattcta aactttatat taatggaaat ttaatagatc aaaaatcaat tttaaattta 3060
   ggtaatattc atgttagtga caatatatta tttaaaatag ttaattgtag ttatacaaga 3120
   tatattggta ttagatattt taatattttt gataaagaat tagatgaaac agaaattcaa 3180
   actttatata gcaatgaacc taatacaaat attttgaagg atttttgggg aaattatttg 3240
   ctttatgaca aagaatacta tttattaaat gtgttaaaac caaataactt tattgatagg 3300
   agaaaagatt ctactttaag cattaataat ataagaagca ctattctttt agctaataga 3360
   ttatatagtg gaataaaagt taaaatacaa agagttaata atagtagtac taacgataat 3420
   cttgttagaa agaatgatca ggtatatatt aattttgtag ccagcaaaac tcacttattt 3480
   ccattatatg ctgatacagc taccacaaat aaagagaaaa caataaaaat atcatcatct 3540
   ggcaatagat ttaatcaagt agtagttatg aattcagtag gaaattgtac aatgaatttt 3600
   aaaaataata atggaaataa tattgggttg ttaggtttca aggcagatac tgtcgttgct 3660
   agtacttggt attatacaca tatgagagat catacaaaca gcaatggatg tttttggaac 3720
   tttatttctg aagaacatgg atggcaagaa aaataa 3756
<210> 10
   <211> 1251
   <212> PRT
   <213> Clostridium botulinum
<400> 10
   Met Pro Lys Ile Asn Ser Phe Asn Tyr Asn Asp Pro Val Asn Asp Arg
   1               5                   10                  15
   Thr Ile Leu Tyr Ile Lys Pro Gly Gly Cys Gln Glu Phe Tyr Lys Ser
               20                  25                  30
   Phe Asn Ile Met Lys Asn Ile Trp Ile Ile Pro Glu Arg Asn Val Ile
           35                  40                  45
   Gly Thr Thr Pro Gln Asp Phe His Pro Pro Thr Ser Leu Lys Asn Gly
       50                  55                  60
   Asp Ser Ser Tyr Tyr Asp Pro Asn Tyr Leu Gln Ser Asp Glu Glu Lys
   65                  70                  75                  80
   Asp Arg Phe Leu Lys Ile Val Thr Lys Ile Phe Asn Arg Ile Asn Asn
                   85                  90                  95
   Asn Leu Ser Gly Gly Ile Leu Leu Glu Glu Leu Ser Lys Ala Asn Pro
               100                 105                 110
   Tyr Leu Gly Asn Asp Asn Thr Pro Asp Asn Gln Phe His Ile Gly Asp
   115 120 125
   Ala Ser Ala Val Glu Ile Lys Phe Ser Asn Gly Ser Gln Asp Ile Leu
   130 135 140
   Leu Pro Asn Val Ile Ile Met Gly Ala Glu Pro Asp Leu Phe Glu Thr
   145 150 155 160
   Asn Ser Ser Asn Ile Ser Leu Arg Asn Asn Tyr Met Pro Ser Asn His
   165 170 175
   Arg Phe Gly Ser Ile Ala Ile Val Thr Phe Ser Pro Glu Tyr Ser Phe
   180 185 190
   Arg Phe Asn Asp Asn Cys Met Asn Glu Phe Ile Gln Asp Pro Ala Leu
   195 200 205
   Thr Leu Met His Glu Leu Ile His Ser Leu His Gly Leu Tyr Gly Ala
   210 215 220
   Lys Gly Ile Thr Thr Lys Tyr Thr Ile Thr Gln Lys Gln Asn Pro Leu
   225 230 235 240
   Ile Thr Asn Ile Arg Gly Thr Asn Ile Glu Glu Phe Leu Thr Phe Gly
   245 250 255
   Gly Thr Asp Leu Asn Ile Ile Thr Ser Ala Gln Ser Asn Asp Ile Tyr
   260 265 270
   Thr Asn Leu Leu Ala Asp Tyr Lys Lys Ile Ala Ser Lys Leu Ser Lys
   275 280 285
   Val Gln Val Ser Asn Pro Leu Leu Asn Pro Tyr Lys Asp Val Phe Glu
   290 295 300
   Ala Lys Tyr Gly Leu Asp Lys Asp Ala Ser Gly Ile Tyr Ser Val Asn
   305 310 315 320
   Ile Asn Lys Phe Asn Asp Ile Phe Lys Lys Leu Tyr Ser Phe Thr Glu
   325 330 335
   Phe Asp Leu Arg Thr Lys Phe Gln Val Lys Cys Arg Gln Thr Tyr Ile
   340 345 350
   Gly Gln Tyr Lys Tyr Phe Lys Leu Ser Asn Leu Leu Asn Asp Ser Ile
   355 360 365
   Tyr Asn Ile Ser Glu Gly Tyr Asn Ile Asn Asn Leu Lys Val Asn Phe
   370 375 380
   Arg Gly Gln Asn Ala Asn Leu Asn Pro Arg Ile Ile Thr Pro Ile Thr
   385 390 395 400
   Gly Arg Gly Leu Val Lys Lys Ile Ile Arg Phe Cys Lys Asn Ile Val
   405 410 415
   Ser Val Lys Gly Ile Arg Lys Ser Ile Cys Ile Glu Ile Asn Asn Gly
   420 425 430
   Glu Leu Phe Phe Val Ala Ser Glu Asn Ser Tyr Asn Asp Asp Asn Ile
   435 440 445
   Asn Thr Pro Lys Glu Ile Asp Asp Thr Val Thr Ser Asn Asn Asn Tyr
   450 455 460
   Glu Asn Asp Leu Asp Gln Val Ile Leu Asn Phe Asn Ser Glu Ser Ala
   465 470 475 480
   Pro Gly Leu Ser Asp Glu Lys Leu Asn Leu Thr Ile Gln Asn Asp Ala
   485 490 495
   Tyr Ile Pro Lys Tyr Asp Ser Asn Gly Thr Ser Asp Ile Glu Gln His
   500 505 510
   Asp Val Asn Glu Leu Asn Val Phe Phe Tyr Leu Asp Ala Gln Lys Val
   515 520 525
   Pro Glu Gly Glu Asn Asn Val Asn Leu Thr Ser Ser Ile Asp Thr Ala
   530 535 540
   Leu Leu Glu Gln Pro Lys Ile Tyr Thr Phe Phe Ser Ser Glu Phe Ile
   545 550 555 560
   Asn Asn Val Asn Lys Pro Val Gln Ala Ala Leu Phe Val Ser Trp Ile
   565 570 575
   Gln Gln Val Leu Val Asp Phe Thr Thr Glu Ala Asn Gln Lys Ser Thr
   580 585 590
   Val Asp Lys Ile Ala Asp Ile Ser Ile Val Val Pro Tyr Ile Gly Leu
   595 600 605
   Ala Leu Asn Ile Gly Asn Glu Ala Gln Lys Gly Asn Phe Lys Asp Ala
   610 615 620
   Leu Glu Leu Leu Gly Ala Gly Ile Leu Leu Glu Phe Glu Pro Glu Leu
   625 630 635 640
   Leu Ile Pro Thr Ile Leu Val Phe Thr Ile Lys Ser Phe Leu Gly Ser
   645 650 655
   Ser Asp Asn Lys Asn Lys Val Ile Lys Ala Ile Asn Asn Ala Leu Lys
   660 665 670
   Glu Arg Asp Glu Lys Trp Lys Glu Val Tyr Ser Phe Ile Val Ser Asn
   675 680 685
   Trp Met Thr Lys Ile Asn Thr Gln Phe Asn Lys Arg Lys Glu Gln Met
   690 695 700
   Tyr Gln Ala Leu Gln Asn Gln Val Asn Ala Ile Lys Thr Ile Ile Glu
   705 710 715 720
   Ser Lys Tyr Asn Ser Tyr Thr Leu Glu Glu Lys Asn Glu Leu Thr Asn
   725 730 735
   Lys Tyr Asp Ile Lys Gln Ile Glu Asn Glu Leu Asn Gln Lys Val Ser
   740 745 750
   Ile Ala Met Asn Asn Ile Asp Arg Phe Leu Thr Glu Ser Ser Ile Ser
   755 760 765
   Tyr Leu Met Lys Ile Ile Asn Glu Val Lys Ile Asn Lys Leu Arg Glu
   770 775 780
   Tyr Asp Glu Asn Val Lys Thr Tyr Leu Leu Asn Tyr Ile Ile Gln His
   785 790 795 800
   Gly Ser Ile Leu Gly Glu Ser Gln Gln Glu Leu Asn Ser Met Val Thr
   805 810 815
   Asp Thr Leu Asn Asn Ser Ile Pro Phe Lys Leu Ser Ser Tyr Thr Asp
   820 825 830
   Asp Lys Ile Leu Ile Ser Tyr Phe Asn Lys Phe Phe Lys Arg Ile Lys
   835 840 845
   Ser Ser Ser Val Leu Asn Met Arg Tyr Lys Asn Asp Lys Tyr Val Asp
   850 855 860
   Thr Ser Gly Tyr Asp Ser Asn Ile Asn Ile Asn Gly Asp Val Tyr Lys
   865 870 875 880
   Tyr Pro Thr Asn Lys Asn Gln Phe Gly Ile Tyr Asn Asp Lys Leu Ser
   885 890 895
   Glu Val Asn Ile Ser Gln Asn Asp Tyr Ile Ile Tyr Asp Asn Lys Tyr
   900 905 910
   Lys Asn Phe Ser Ile Ser Phe Trp Val Arg Ile Pro Asn Tyr Asp Asn
   915 920 925
   Lys Ile Val Asn Val Asn Asn Glu Tyr Thr Ile Ile Asn Cys Met Arg
   930 935 940
   Asp Asn Asn Ser Gly Trp Lys Val Ser Leu Asn His Asn Glu Ile Ile
   945 950 955 960
   Trp Thr Phe Glu Asp Asn Arg Gly Ile Asn Gln Lys Leu Ala Phe Asn
   965 970 975
   Tyr Gly Asn Ala Asn Gly Ile Ser Asp Tyr Ile Asn Lys Trp Ile Phe
   980 985 990
   Val Thr Ile Thr Asn Asp Arg Leu Gly Asp Ser Lys Leu Tyr Ile Asn
   995 1000 1005
   Gly Asn Leu Ile Asp Gln Lys Ser Ile Leu Asn Leu Gly Asn Ile
   1010 1015 1020
   His Val Ser Asp Asn Ile Leu Phe Lys Ile Val Asn Cys Ser Tyr
   1025 1030 1035
   Thr Arg Tyr Ile Gly Ile Arg Tyr Phe Asn Ile Phe Asp Lys Glu
   1040 1045 1050
   Leu Asp Glu Thr Glu Ile Gln Thr Leu Tyr Ser Asn Glu Pro Asn
   1055 1060 1065
   Thr Asn Ile Leu Lys Asp Phe Trp Gly Asn Tyr Leu Leu Tyr Asp
   1070 1075 1080
   Lys Glu Tyr Tyr Leu Leu Asn Val Leu Lys Pro Asn Asn Phe Ile
   1085 1090 1095
   Asp Arg Arg Lys Asp Ser Thr Leu Ser Ile Asn Asn Ile Arg Ser
   1100 1105 1110
   Thr Ile Leu Leu Ala Asn Arg Leu Tyr Ser Gly Ile Lys Val Lys
   1115 1120 1125
   Ile Gln Arg Val Asn Asn Ser Ser Thr Asn Asp Asn Leu Val Arg
   1130 1135 1140
   Lys Asn Asp Gln Val Tyr Ile Asn Phe Val Ala Ser Lys Thr His
   1145 1150 1155
   Leu Phe Pro Leu Tyr Ala Asp Thr Ala Thr Thr Asn Lys Glu Lys
   1160 1165 1170
   Thr Ile Lys Ile Ser Ser Ser Gly Asn Arg Phe Asn Gln Val Val
   1175 1180 1185
   Val Met Asn Ser Val Gly Asn Cys Thr Met Asn Phe Lys Asn Asn
   1190 1195 1200
   Asn Gly Asn Asn Ile Gly Leu Leu Gly Phe Lys Ala Asp Thr Val
   1205 1210 1215
   Val Ala Ser Thr Trp Tyr Tyr Thr His Met Arg Asp His Thr Asn
   1220 1225 1230
   Ser Asn Gly Cys Phe Trp Asn Phe Ile Ser Glu Glu His Gly Trp
   1235 1240 1245
   Gln Glu Lys
   1250
<210> 11
   <211> 3843
   <212> DNA
   <213> Clostridium botulinum
<400> 11
<210> 12
   <211> 1280
   <212> PRT
   <213> Clostridium botulinum
<400> 12
   Met Pro Val Val Ile Asn Ser Phe Asn Tyr Asn Asp Pro Val Asn Asp
   1 5 10 15
   Glu Thr Ile Leu Tyr Met Gln Lys Pro Tyr Glu Glu Arg Ser Arg Lys
   20 25 30
   Tyr Tyr Lys Ala Phe Glu Ile Met Pro Asn Val Trp Ile Met Pro Glu
   35 40 45
   Arg Asp Thr Ile Gly Thr Lys Pro Asp Glu Phe Gln Val Pro Asp Ser
   50 55 60
   Leu Lys Asn Gly Ser Ser Ala Tyr Tyr Asp Pro Asn Tyr Leu Thr Thr
   65 70 75 80
   Asp Ala Glu Lys Asp Arg Tyr Leu Lys Thr Met Ile Lys Leu Phe Asn
   85 90 95
   Arg Ile Asn Ser Asn Pro Thr Gly Lys Val Leu Leu Glu Glu Val Ser
   100 105 110
   Asn Ala Arg Pro Tyr Leu Gly Asp Asp Asp Thr Leu Ile Asn Glu Phe
   115 120 125
   Leu Pro Val Asn Val Thr Thr Ser Val Asn Ile Lys Phe Ser Thr Asp
   130 135 140
   Val Glu Ser Ser Ile Ile Ser Asn Leu Leu Val Leu Gly Ala Gly Pro
   145 150 155 160
   Asp Ile Phe Lys Ala Tyr Cys Thr Pro Leu Val Arg Phe Asn Lys Ser
   165 170 175
   Asp Lys Leu Ile Glu Pro Ser Asn His Gly Phe Gly Ser Ile Asn Ile
   180 185 190
   Leu Thr Phe Ser Pro Glu Tyr Glu His Ile Phe Asn Asp Ile Ser Gly
   195 200 205
   Gly Asn His Asn Ser Thr Glu Ser Phe Ile Ala Asp Pro Ala Ile Ser
   210 215 220
   Leu Ala His Glu Leu Ile His Ala Leu His Gly Leu Tyr Gly Ala Lys
   225 230 235 240
   Ala Val Thr His Lys Glu Ser Leu Val Ala Glu Arg Gly Pro Leu Met
   245 250 255
   Ile Ala Glu Lys Pro Ile Arg Leu Glu Glu Phe Leu Thr Phe Gly Gly
   260 265 270
   Glu Asp Leu Asn Ile Ile Pro Ser Ala Met Lys Glu Lys Ile Tyr Asn
   275 280 285
   Asp Leu Leu Ala Asn Tyr Glu Lys Ile Ala Thr Arg Leu Arg Glu Val
   290 295 300
   Asn Thr Ala Pro Pro Gly Tyr Asp Ile Asn Glu Tyr Lys Asp Tyr Phe
   305 310 315 320
   Gln Trp Lys Tyr Gly Leu Asp Arg Asn Ala Asp Gly Ser Tyr Thr Val
   325 330 335
   Asn Arg Asn Lys Phe Asn Glu Ile Tyr Lys Lys Leu Tyr Ser Phe Thr
   340 345 350
   Glu Ile Asp Leu Ala Asn Lys Phe Lys Val Lys Cys Arg Asn Thr Tyr
   355 360 365
   Phe Ile Lys Tyr Gly Phe Val Lys Val Pro Asn Leu Leu Asp Asp Asp
   370 375 380
   Ile Tyr Thr Val Ser Glu Gly Phe Asn Ile Gly Asn Leu Ala Val Asn
   385 390 395 400
   Asn Arg Gly Gln Asn Ile Asn Leu Asn Pro Lys Ile Ile Asp Ser Ile
   405 410 415
   Pro Asp Lys Gly Leu Val Glu Lys Ile Ile Lys Phe Cys Lys Ser Ile
   420 425 430
   Ile Pro Arg Lys Gly Thr Lys Gln Ser Pro Ser Leu Cys Ile Arg Val
   435 440 445
   Asn Asn Arg Glu Leu Phe Phe Val Ala Ser Glu Ser Ser Tyr Asn Glu
   450 455 460
   Ser Asp Ile Asn Thr Pro Lys Glu Ile Asp Asp Thr Thr Asn Leu Asn
   465 470 475 480
   Asn Asn Tyr Arg Asn Asn Leu Asp Glu Val Ile Leu Asp Tyr Asn Ser
   485 490 495
   Glu Thr Ile Pro Gln Ile Ser Asn Arg Thr Leu Asn Thr Leu Val Gln
   500 505 510
   Asp Asn Ser Tyr Val Pro Arg Tyr Asp Ser Asn Gly Thr Ser Glu Ile
   515 520 525
   Glu Glu Tyr Asp Val Val Asp Phe Asn Val Phe Phe Tyr Leu His Ala
   530 535 540
   Gln Lys Val Pro Glu Gly Glu Thr Asn Ile Ser Leu Thr Ser Ser Ile
   545 550 555 560
   Asp Thr Ala Leu Leu Glu Glu Ser Lys Val Tyr Thr Phe Phe Ser Ser
   565 570 575
   Glu Phe Ile Asp Thr Ile Asn Lys Pro Val Asn Ala Ala Leu Phe Ile
   580 585 590
   Asp Trp Ile Ser Lys Val Ile Arg Asp Phe Thr Thr Glu Ala Thr Gln
   595 600 605
   Lys Ser Thr Val Asp Lys Ile Ala Asp Ile Ser Leu Ile Val Pro Tyr
   610 615 620
   Val Gly Leu Ala Leu Asn Ile Val Ile Glu Ala Glu Lys Gly Asn Phe
   625 630 635 640
   Glu Glu Ala Phe Glu Leu Leu Gly Ala Gly Ile Leu Leu Glu Phe Val
   645 650 655
   Pro Glu Leu Thr Ile Pro Val Ile Leu Val Phe Thr Ile Lys Ser Tyr
   660 665 670
   Ile Asp Ser Tyr Glu Asn Lys Asn Lys Ala Ile Lys Ala Ile Asn Asn
   675 680 685
   Ser Leu Ile Glu Arg Glu Ala Lys Trp Lys Glu Ile Tyr Ser Trp Ile
   690 695 700
   Val Ser Asn Trp Leu Thr Arg Ile Asn Thr Gln Phe Asn Lys Arg Lys
   705 710 715 720
   Glu Gln Met Tyr Gln Ala Leu Gln Asn Gln Val Asp Ala Ile Lys Thr
   725 730 735
   Ala Ile Glu Tyr Lys Tyr Asn Asn Tyr Thr Ser Asp Glu Lys Asn Arg
   740 745 750
   Leu Glu Ser Lys Tyr Asn Ile Asn Asn Ile Glu Glu Glu Leu Asn Lys
   755 760 765
   Lys Val Ser Leu Ala Met Lys Asn Ile Glu Arg Phe Met Thr Glu Ser
   770 775 780
   Ser Ile Ser Tyr Leu Met Lys Leu Ile Asn Glu Ala Glu Val Gly Lys
   785 790 795 800
   Leu Lys Glu Tyr Asp Lys His Val Lys Ser Asp Leu Leu Asp Tyr Ile
   805 810 815
   Leu Tyr His Lys Leu Ile Leu Gly Glu Gln Thr Lys Glu Leu Ile Asp
   820 825 830
   Leu Val Thr Ser Thr Leu Asn Ser Ser Ile Pro Phe Glu Leu Ser Ser
   835 840 845
   Tyr Thr Asn Asp Lys Ile Leu Ile Ile Tyr Phe Asn Arg Leu Tyr Lys
   850 855 860
   Lys Ile Lys Asp Ser Ser Ile Leu Asp Met Arg Tyr Glu Asn Asn Lys
   865 870 875 880
   Phe Ile Asp Ile Ser Gly Tyr Gly Ser Asn Ile Ser Ile Asn Gly Asn
   885 890 895
   Val Tyr Ile Tyr Ser Thr Asn Arg Asn Gln Phe Gly Ile Tyr Ser Gly
   900 905 910
   Arg Leu Ser Glu Val Asn Ile Ala Gln Asn Asn Asp Ile Ile Tyr Asn
   915 920 925
   Ser Arg Tyr Gln Asn Phe Ser Ile Ser Phe Trp Val Thr Ile Pro Lys
   930 935 940
   His Tyr Arg Pro Met Asn Arg Asn Arg Glu Tyr Thr Ile Ile Asn Cys
   945 950 955 960
   Met Gly Asn Asn Asn Ser Gly Trp Lys Ile Ser Leu Arg Thr Ile Arg
   965 970 975
   Asp Cys Glu Ile Ile Trp Thr Leu Gln Asp Thr Ser Gly Asn Lys Glu
   980 985 990
   Lys Leu Ile Phe Arg Tyr Glu Glu Leu Ala Ser Ile Ser Asp Tyr Ile
   995 1000 1005
   Asn Lys Trp Ile Phe Val Thr Ile Thr Asn Asn Arg Leu Gly Asn
   1010 1015 1020
   Ser Arg Ile Tyr Ile Asn Gly Asn Leu Ile Val Glu Lys Ser Ile
   1025 1030 1035
   Ser Asn Leu Gly Asp Ile His Val Ser Asp Asn Ile Leu Phe Lys
   1040 1045 1050
   Ile Val Gly Cys Asp Asp Glu Thr Tyr Val Gly Ile Arg Tyr Phe
   1055 1060 1065
   Lys Val Phe Asn Thr Glu Leu Asp Lys Thr Glu Ile Glu Thr Leu
   1070 1075 1080
   Tyr Ser Asn Glu Pro Asp Pro Ser Ile Leu Lys Asp Tyr Trp Gly
   1085 1090 1095
   Asn Tyr Leu Leu Tyr Asn Lys Lys Tyr Tyr Leu Phe Asn Leu Leu
   1100 1105 1110
   Arg Lys Asp Lys Tyr Ile Thr Arg Asn Ser Gly Ile Leu Asn Ile
   1115 1120 1125
   Asn Gln Gln Arg Gly Val Thr Gly Gly Ile Ser Val Phe Leu Asn
   1130 1135 1140
   Tyr Lys Leu Tyr Glu Gly Val Glu Val Ile Ile Arg Lys Asn Ala
   1145 1150 1155
   Pro Ile Asp Ile Ser Asn Thr Asp Asn Phe Val Arg Lys Asn Asp
   1160 1165 1170
   Leu Ala Tyr Ile Asn Val Val Asp His Gly Val Glu Tyr Arg Leu
   1175 1180 1185
   Tyr Ala Asp Ile Ser Ile Thr Lys Ser Glu Lys Ile Ile Lys Leu
   1190 1195 1200
   Ile Arg Thr Ser Asn Pro Asn Asp Ser Leu Gly Gln Ile Ile Val
   1205 1210 1215
   Met Asp Ser Ile Gly Asn Asn Cys Thr Met Asn Phe Gln Asn Asn
   1220 1225 1230
   Asp Gly Ser Asn Ile Gly Leu Leu Gly Phe His Ser Asp Asp Leu
   1235 1240 1245
   Val Ala Ser Ser Trp Tyr Tyr Asn His Ile Arg Arg Asn Thr Ser
   1250 1255 1260
   Ser Asn Gly Cys Phe Trp Ser Phe Ile Ser Lys Glu His Gly Trp
   1265 1270 1275
   Lys Glu
   1280
<210> 13
   <211> 3894
   <212> DNA
   <213> Clostridium botulinum
<220>
   <221> misc_feature
   <222> (20)..(20)
   <223> n is a, c, g, or t
<400> 13
   atgccagtta atataaaaan ctttaattat aatgacccta ttaataatga tgacattatt      60
   atgatggaac cattcaatga cccagggcca ggaacatatt ataaagcttt taggattata     120
   gatcgtattt ggatagtacc agaaaggttt acttatggat ttcaacctga ccaatttaat     180
   gccagtacag gagtttttag taaagatgtc tacgaatatt acgatccaac ttatttaaaa     240
   accgatgctg aaaaagataa atttttaaaa acaatgatta aattatttaa tagaattaat     300
   tcaaaaccat caggacagag attactggat atgatagtag atgctatacc ttatcttgga     360
   aatgcatcta caccgcccga caaatttgca gcaaatgttg caaatgtatc tattaataaa     420
   aaaattatcc aacctggagc tgaagatcaa ataaaaggtt taatgacaaa tttaataata     480
   tttggaccag gaccagttct aagtgataat tttactgata gtatgattat gaatggccat     540
   tccccaatat cagaaggatt tggtgcaaga atgatgataa gattttgtcc tagttgttta     600
   aatgtattta ataatgttca ggaaaataaa gatacatcta tatttagtag acgcgcgtat     660
   tttgcagatc cagctctaac gttaatgcat gaacttatac atgtgttaca tggattatat     720
   ggaattaaga taagtaattt accaattact ccaaatacaa aagaattttt catgcaacat     780
   agcgatcctg tacaagcaga agaactatat acattcggag gacatgatcc tagtgttata     840
   agtccttcta cggatatgaa tatttataat aaagcgttac aaaattttca agatatagct     900
   aataggctta atattgtttc aagtgcccaa gggagtggaa ttgatatttc cttatataaa     960
   caaatatata aaaataaata tgattttgtt gaagatccta atggaaaata tagtgtagat    1020
   aaggataagt ttgataaatt atataaggcc ttaatgtttg gctttactga aactaatcta    1080
   gctggtgaat atggaataaa aactaggtat tcttatttta gtgaatattt gccaccgata    1140
   aaaactgaaa aattgttaga caatacaatt tatactcaaa atgaaggctt taacatagct    1200
   agtaaaaatc tcaaaacgga atttaatggt cagaataagg cggtaaataa agaggcttat    1260
   gaagaaatca gcctagaaca tctcgttata tatagaatag caatgtgcaa gcctgtaatg    1320
   tacaaaaata ccggtaaatc tgaacagtgt attattgtta ataatgagga tttatttttc    1380
   atagctaata aagatagttt ttcaaaagat ttagctaaag cagaaactat agcatataat    1440
   acacaaaata atactataga aaataatttt tctatagatc agttgatttt agataatgat    1500
   ttaagcagtg gcatagactt accaaatgaa aacacagaac catttacaaa ttttgacgac    1560
   atagatatcc ctgtgtatat taaacaatct gctttaaaaa aaatttttgt ggatggagat 1620
   agcctttttg aatatttaca tgctcaaaca tttccttcta atatagaaaa tctacaacta 1680
   acgaattcat taaatgatgc tttaagaaat aataataaag tctatacttt tttttctaca 1740
   aaccttgttg aaaaagctaa tacagttgta ggtgcttcac tttttgtaaa ctgggtaaaa 1800
   ggagtaatag atgattttac atctgaatcc acacaaaaaa gtactataga taaagtttca 1860
   gatgtatcca taattattcc ctatatagga cctgctttga atgtaggaaa tgaaacagct 1920
   aaagaaaatt ttaaaaatgc ttttgaaata ggtggagccg ctatcttaat ggagtttatt 1980
   ccagaactta ttgtacctat agttggattt tttacattag aatcatatgt aggaaataaa 2040
   gggcatatta ttatgacgat atccaatgct ttaaagaaaa gggatcaaaa atggacagat 2100
   atgtatggtt tgatagtatc gcagtggctc tcaacggtta atactcaatt ttatacaata 2160
   aaagaaagaa tgtacaatgc tttaaataat caatcacaag caatagaaaa aataatagaa 2220
   gatcaatata atagatatag tgaagaagat aaaatgaata ttaacattga ttttaatgat 2280
   atagatttta aacttaatca aagtataaat ttagcaataa acaatataga tgattttata 2340
   aaccaatgtt ctatatcata tctaatgaat agaatgattc cattagctgt aaaaaagtta 2400
   aaagactttg atgataatct taagagagat ttattggagt atatagatac aaatgaacta 2460
   tatttacttg atgaagtaaa tattctaaaa tcaaaagtaa atagacacct aaaagacagt 2520
   ataccatttg atctttcact atataccaag gacacaattt taatacaagt ttttaataat 2580
   tatattagta atattagtag taatgctatt ttaagtttaa gttatagagg tgggcgttta 2640
   atagattcat ctggatatgg tgcaactatg aatgtaggtt cagatgttat ctttaatgat 2700
   ataggaaatg gtcaatttaa attaaataat tctgaaaata gtaatattac ggcacatcaa 2760
   agtaaattcg ttgtatatga tagtatgttt gataatttta gcattaactt ttgggtaagg 2820
   actcctaaat ataataataa tgatatacaa acttatcttc aaaatgagta tacaataatt 2880
   agttgtataa aaaatgactc aggatggaaa gtatctatta agggaaatag aataatatgg 2940
   acattaatag atgttaatgc aaaatctaaa tcaatatttt tcgaatatag tataaaagat 3000
   aatatatcag attatataaa taaatggttt tccataacta ttactaatga tagattaggt 3060
   aacgcaaata tttatataaa tggaagtttg aaaaaaagtg aaaaaatttt aaacttagat 3120
   agaattaatt ctagtaatga tatagacttc aaattaatta attgtacaga tactactaaa 3180
   tttgtttgga ttaaggattt taatattttt ggtagagaat taaatgctac agaagtatct 3240
   tcactatatt ggattcaatc atctacaaat actttaaaag atttttgggg gaatccttta 3300
   agatacgata cacaatacta tctgtttaat caaggtatgc aaaatatcta tataaagtat 3360
   tttagtaaag cttctatggg ggaaactgca ccacgtacaa actttaataa tgcagcaata 3420
   aattatcaaa atttatatct tggtttacga tttattataa aaaaagcatc aaattctcgg 3480
   aatataaata atgataatat agtcagagaa ggagattata tatatcttaa tattgataat 3540
   atttctgatg aatcttacag agtatatgtt ttggtgaatt ctaaagaaat tcaaactcaa 3600
   ttatttttag cacccataaa tgatgatcct acgttctatg atgtactaca aataaaaaaa 3660
   tattatgaaa aaacaacata taattgtcag atactttgcg aaaaagatac taaaacattt 3720
   gggctgtttg gaattggtaa atttgttaaa gattatggat atgtttggga tacctatgat 3780
   aattattttt gcataagtca gtggtatctc agaagaatat ctgaaaatat aaataaatta 3840
   aggttgggat gtaattggca attcattccc gtggatgaag gatggacaga ataa 3894
<210> 14
   <211> 1297
   <212> PRT
   <213> Clostridium botulinum
<220>
   <221> misc_feature
   <222> (7)..(7)
   <223> Xaa can be any naturally occurring amino acid
<400> 14
   Met Pro Val Asn Ile Lys Xaa Phe Asn Tyr Asn Asp Pro Ile Asn Asn
   1               5                   10                  15
   Asp Asp Ile Ile Met Met Glu Pro Phe Asn Asp Pro Gly Pro Gly Thr
               20                  25                  30
   Tyr Tyr Lys Ala Phe Arg Ile Ile Asp Arg Ile Trp Ile Val Pro Glu
           35                  40                  45
   Arg Phe Thr Tyr Gly Phe Gln Pro Asp Gln Phe Asn Ala Ser Thr Gly
       50                  55                  60
   Val Phe Ser Lys Asp Val Tyr Glu Tyr Tyr Asp Pro Thr Tyr Leu Lys
   65                  70                  75                  80
   Thr Asp Ala Glu Lys Asp Lys Phe Leu Lys Thr Met Ile Lys Leu Phe
                   85                  90                  95
   Asn Arg Ile Asn Ser Lys Pro Ser Gly Gln Arg Leu Leu Asp Met Ile
               100                 105                 110
   Val Asp Ala Ile Pro Tyr Leu Gly Asn Ala Ser Thr Pro Pro Asp Lys
           115                 120                 125
   Phe Ala Ala Asn Val Ala Asn Val Ser Ile Asn Lys Lys Ile Ile Gln
       130                 135                 140
   Pro Gly Ala Glu Asp Gln Ile Lys Gly Leu Met Thr Asn Leu Ile Ile
   145                 150                 155                 160
   Phe Gly Pro Gly Pro Val Leu Ser Asp Asn Phe Thr Asp Ser Met Ile
                   165                 170                 175
   Met Asn Gly His Ser Pro Ile Ser Glu Gly Phe Gly Ala Arg Met Met
   180 185 190
   Ile Arg Phe Cys Pro Ser Cys Leu Asn Val Phe Asn Asn Val Gln Glu
   195 200 205
   Asn Lys Asp Thr Ser Ile Phe Ser Arg Arg Ala Tyr Phe Ala Asp Pro
   210 215 220
   Ala Leu Thr Leu Met His Glu Leu Ile His Val Leu His Gly Leu Tyr
   225 230 235 240
   Gly Ile Lys Ile Ser Asn Leu Pro Ile Thr Pro Asn Thr Lys Glu Phe
   245 250 255
   Phe Met Gln His Ser Asp Pro Val Gln Ala Glu Glu Leu Tyr Thr Phe
   260 265 270
   Gly Gly His Asp Pro Ser Val Ile Ser Pro Ser Thr Asp Met Asn Ile
   275 280 285
   Tyr Asn Lys Ala Leu Gln Asn Phe Gln Asp Ile Ala Asn Arg Leu Asn
   290 295 300
   Ile Val Ser Ser Ala Gln Gly Ser Gly Ile Asp Ile Ser Leu Tyr Lys
   305 310 315 320
   Gln Ile Tyr Lys Asn Lys Tyr Asp Phe Val Glu Asp Pro Asn Gly Lys
   325 330 335
   Tyr Ser Val Asp Lys Asp Lys Phe Asp Lys Leu Tyr Lys Ala Leu Met
   340 345 350
   Phe Gly Phe Thr Glu Thr Asn Leu Ala Gly Glu Tyr Gly Ile Lys Thr
   355 360 365
   Arg Tyr Ser Tyr Phe Ser Glu Tyr Leu Pro Pro Ile Lys Thr Glu Lys
   370 375 380
   Leu Leu Asp Asn Thr Ile Tyr Thr Gln Asn Glu Gly Phe Asn Ile Ala
   385 390 395 400
   Ser Lys Asn Leu Lys Thr Glu Phe Asn Gly Gln Asn Lys Ala Val Asn
   405 410 415
   Lys Glu Ala Tyr Glu Glu Ile Ser Leu Glu His Leu Val Ile Tyr Arg
   420 425 430
   Ile Ala Met Cys Lys Pro Val Met Tyr Lys Asn Thr Gly Lys Ser Glu
   435 440 445
   Gln Cys Ile Ile Val Asn Asn Glu Asp Leu Phe Phe Ile Ala Asn Lys
   450 455 460
   Asp Ser Phe Ser Lys Asp Leu Ala Lys Ala Glu Thr Ile Ala Tyr Asn
   465 470 475 480
   Thr Gln Asn Asn Thr Ile Glu Asn Asn Phe Ser Ile Asp Gln Leu Ile
   485 490 495
   Leu Asp Asn Asp Leu Ser Ser Gly Ile Asp Leu Pro Asn Glu Asn Thr
   500 505 510
   Glu Pro Phe Thr Asn Phe Asp Asp Ile Asp Ile Pro Val Tyr Ile Lys
   515 520 525
   Gln Ser Ala Leu Lys Lys Ile Phe Val Asp Gly Asp Ser Leu Phe Glu
   530 535 540
   Tyr Leu His Ala Gln Thr Phe Pro Ser Asn Ile Glu Asn Leu Gln Leu
   545 550 555 560
   Thr Asn Ser Leu Asn Asp Ala Leu Arg Asn Asn Asn Lys Val Tyr Thr
   565 570 575
   Phe Phe Ser Thr Asn Leu Val Glu Lys Ala Asn Thr Val Val Gly Ala
   580 585 590
   Ser Leu Phe Val Asn Trp Val Lys Gly Val Ile Asp Asp Phe Thr Ser
   595 600 605
   Glu Ser Thr Gln Lys Ser Thr Ile Asp Lys Val Ser Asp Val Ser Ile
   610 615 620
   Ile Ile Pro Tyr Ile Gly Pro Ala Leu Asn Val Gly Asn Glu Thr Ala
   625 630 635 640
   Lys Glu Asn Phe Lys Asn Ala Phe Glu Ile Gly Gly Ala Ala Ile Leu
   645 650 655
   Met Glu Phe Ile Pro Glu Leu Ile Val Pro Ile Val Gly Phe Phe Thr
   660 665 670
   Leu Glu Ser Tyr Val Gly Asn Lys Gly His Ile Ile Met Thr Ile Ser
   675 680 685
   Asn Ala Leu Lys Lys Arg Asp Gln Lys Trp Thr Asp Met Tyr Gly Leu
   690 695 700
   Ile Val Ser Gln Trp Leu Ser Thr Val Asn Thr Gln Phe Tyr Thr Ile
   705 710 715 720
   Lys Glu Arg Met Tyr Asn Ala Leu Asn Asn Gln Ser Gln Ala Ile Glu
   725 730 735
   Lys Ile Ile Glu Asp Gln Tyr Asn Arg Tyr Ser Glu Glu Asp Lys Met
   740 745 750
   Asn Ile Asn Ile Asp Phe Asn Asp Ile Asp Phe Lys Leu Asn Gln Ser
   755 760 765
   Ile Asn Leu Ala Ile Asn Asn Ile Asp Asp Phe Ile Asn Gln Cys Ser
   770 775 780
   Ile Ser Tyr Leu Met Asn Arg Met Ile Pro Leu Ala Val Lys Lys Leu
   785 790 795 800
   Lys Asp Phe Asp Asp Asn Leu Lys Arg Asp Leu Leu Glu Tyr Ile Asp
   805 810 815
   Thr Asn Glu Leu Tyr Leu Leu Asp Glu Val Asn Ile Leu Lys Ser Lys
   820 825 830
   Val Asn Arg His Leu Lys Asp Ser Ile Pro Phe Asp Leu Ser Leu Tyr
   835 840 845
   Thr Lys Asp Thr Ile Leu Ile Gln Val Phe Asn Asn Tyr Ile Ser Asn
   850 855 860
   Ile Ser Ser Asn Ala Ile Leu Ser Leu Ser Tyr Arg Gly Gly Arg Leu
   865 870 875 880
   Ile Asp Ser Ser Gly Tyr Gly Ala Thr Met Asn Val Gly Ser Asp Val
   885 890 895
   Ile Phe Asn Asp Ile Gly Asn Gly Gln Phe Lys Leu Asn Asn Ser Glu
   900 905 910
   Asn Ser Asn Ile Thr Ala His Gln Ser Lys Phe Val Val Tyr Asp Ser
   915 920 925
   Met Phe Asp Asn Phe Ser Ile Asn Phe Trp Val Arg Thr Pro Lys Tyr
   930 935 940
   Asn Asn Asn Asp Ile Gln Thr Tyr Leu Gln Asn Glu Tyr Thr Ile Ile
   945 950 955 960
   Ser Cys Ile Lys Asn Asp Ser Gly Trp Lys Val Ser Ile Lys Gly Asn
   965 970 975
   Arg Ile Ile Trp Thr Leu Ile Asp Val Asn Ala Lys Ser Lys Ser Ile
   980 985 990
   Phe Phe Glu Tyr Ser Ile Lys Asp Asn Ile Ser Asp Tyr Ile Asn Lys
   995 1000 1005
   Trp Phe Ser Ile Thr Ile Thr Asn Asp Arg Leu Gly Asn Ala Asn
   1010 1015 1020
   Ile Tyr Ile Asn Gly Ser Leu Lys Lys Ser Glu Lys Ile Leu Asn
   1025 1030 1035
   Leu Asp Arg Ile Asn Ser Ser Asn Asp Ile Asp Phe Lys Leu Ile
   1040 1045 1050
   Asn Cys Thr Asp Thr Thr Lys Phe Val Trp Ile Lys Asp Phe Asn
   1055 1060 1065
   Ile Phe Gly Arg Glu Leu Asn Ala Thr Glu Val Ser Ser Leu Tyr
   1070 1075 1080
   Trp Ile Gln Ser Ser Thr Asn Thr Leu Lys Asp Phe Trp Gly Asn
   1085 1090 1095
   Pro Leu Arg Tyr Asp Thr Gln Tyr Tyr Leu Phe Asn Gln Gly Met
   1100 1105 1110
   Gln Asn Ile Tyr Ile Lys Tyr Phe Ser Lys Ala Ser Met Gly Glu
   1115 1120 1125
   Thr Ala Pro Arg Thr Asn Phe Asn Asn Ala Ala Ile Asn Tyr Gln
   1130 1135 1140
   Asn Leu Tyr Leu Gly Leu Arg Phe Ile Ile Lys Lys Ala Ser Asn
   1145 1150 1155
   Ser Arg Asn Ile Asn Asn Asp Asn Ile Val Arg Glu Gly Asp Tyr
   1160 1165 1170
   Ile Tyr Leu Asn Ile Asp Asn Ile Ser Asp Glu Ser Tyr Arg Val
   1175 1180 1185
   Tyr Val Leu Val Asn Ser Lys Glu Ile Gln Thr Gln Leu Phe Leu
   1190 1195 1200
   Ala Pro Ile Asn Asp Asp Pro Thr Phe Tyr Asp Val Leu Gln Ile
   1205 1210 1215
   Lys Lys Tyr Tyr Glu Lys Thr Thr Tyr Asn Cys Gln Ile Leu Cys
   1220 1225 1230
   Glu Lys Asp Thr Lys Thr Phe Gly Leu Phe Gly Ile Gly Lys Phe
   1235 1240 1245
   Val Lys Asp Tyr Gly Tyr Val Trp Asp Thr Tyr Asp Asn Tyr Phe
   1250 1255 1260
   Cys Ile Ser Gln Trp Tyr Leu Arg Arg Ile Ser Glu Asn Ile Asn
   1265 1270 1275
   Lys Leu Arg Leu Gly Cys Asn Trp Gln Phe Ile Pro Val Asp Glu
   1280 1285 1290
   Gly Trp Thr Glu
   1295
<210> 15
   <211> 4400
   <212> DNA
   <213> Clostridium tetani
<400> 15
   tagcattaaa aaaattagaa cctatagtaa ataaattaat taatatatag tttttataat 60
   ttaattatga ataatattct taagataaaa agtaaatttt taaaaattta aattttcagt 120
   ttacaaaaaa taacctgatt atgttatatg taattgtaaa aaacatataa aaaatcagaa 180
   aaatttagga ggtatattat taatggatta aataataatt ttttaattta cttttgatta 240
   ataaatatta aatgtttatt ttaattagga gatgatacgt atgccaataa ccataaataa 300
   ttttagatat agtgatcctg ttaataatga tacaattatt atgatggagc caccatactg 360
   taagggtcta gatatctatt ataaggcttt caaaataaca gatcgtattt ggatagtgcc 420
   ggaaaggtat gaatttggga caaaacctga agattttaac ccaccatctt cattaataga 480
   aggtgcatct gagtattacg atccaaatta tttaaggact gattctgata aagatagatt 540
   tttacaaacc atggtaaaac tgtttaacag aattaaaaac aatgtagcag gtgaagcctt 600
   attagataag ataataaatg ccatacctta ccttggaaat tcatattcct tactagacaa 660
   gtttgataca aactctaatt cagtatcttt taatttatta gaacaagacc ccagtggagc 720
   aactacaaaa tcagcaatgc tgacaaattt aataatattt ggacctgggc ctgttttaaa 780
   taaaaatgag gttagaggta ttgtattgag ggtagataat aaaaattact tcccatgtag 840
   agatggtttt ggctcaataa tgcaaatggc attttgccca gaatatgtac ctacctttga 900
   taatgtaata gaaaatatta cgtcactcac tattggcaaa agcaaatatt ttcaagatcc 960
   agcattacta ttaatgcacg aacttataca tgtactacat ggtttatacg gaatgcaggt 1020
   atcaagccat gaaattattc catccaaaca agaaatttat atgcagcata catatccaat 1080
   aagtgctgaa gaactattca cttttggcgg acaggatgct aatcttataa gtattgatat 1140
   aaaaaacgat ttatatgaaa aaactttaaa tgattataaa gctatagcta acaaacttag 1200
   tcaagtcact agctgcaatg atcccaacat tgatattgat agctacaaac aaatatatca 1260
   acaaaaatat caattcgata aagatagcaa tggacaatat attgtaaatg aggataaatt 1320
   tcagatacta tataatagca taatgtatgg ttttacagag attgaattgg gaaaaaaatt 1380
   taatataaaa actagacttt cttattttag tatgaatcat gaccctgtaa aaattccaaa 1440
   tttattagat gatacaattt acaatgatac agaaggattt aatatagaaa gcaaagatct 1500
   gaaatctgaa tataaaggac aaaatatgag ggtaaataca aatgctttta gaaatgttga 1560
   tggatcaggc ctagtttcaa aacttattgg cttatgtaaa aaaattatac caccaacaaa 1620
   tataagagaa aatttatata atagaactgc atcattaaca gatttaggag gagaattatg 1680
   tataaaaatt aaaaatgaag atttaacttt tatagctgaa aaaaatagct tttcagaaga 1740
   accatttcaa gatgaaatag ttagttataa tacaaaaaat aaaccattaa attttaatta 1800
   ttcgctagat aaaattattg tagattataa tctacaaagt aaaattacat tacctaatga 1860
   taggacaacc ccagttacaa aaggaattcc atatgctcca gaatataaaa gtaatgctgc 1920
   aagtacaata gaaatacata atattgatga caatacaata tatcaatatt tgtatgctca 1980
   aaaatctcct acaactctac aaagaataac tatgactaat tctgttgatg acgcattaat 2040
   aaattccacc aaaatatatt catattttcc atctgtaatc agtaaagtta accaaggtgc 2100
   acaaggaatt ttattcttac agtgggtgag agatataatt gatgatttta ccaatgaatc 2160
   ttcacaaaaa actactattg ataaaatttc agatgtatcc actattgttc cttatatagg 2220
   acccgcatta aacattgtaa aacaaggcta tgagggaaac tttataggcg ctttagaaac 2280
   taccggagtg gttttattat tagaatatat tccagaaatt actttaccag taattgcagc 2340
   tttatctata gcagaaagta gcacacaaaa agaaaagata ataaaaacaa tagataactt 2400
   tttagaaaaa agatatgaaa aatggattga agtatataaa ctagtaaaag caaaatggtt 2460
   aggcacagtt aatacgcaat tccaaaaaag aagttatcaa atgtatagat ctttagaata 2520
   tcaagtagat gcaataaaaa aaataataga ctatgaatat aaaatatatt caggacctga 2580
   taaggaacaa attgccgacg aaattaataa tctgaaaaac aaacttgaag aaaaggctaa 2640
   taaagcaatg ataaacataa atatatttat gagggaaagt tctagatcat ttttagttaa 2700
   tcaaatgatt aacgaagcta aaaagcagtt attagagttt gatactcaaa gcaaaaatat 2760
   tttaatgcag tatataaaag caaattctaa atttataggt ataactgaac taaaaaaatt 2820
   agaatcaaaa ataaacaaag ttttttcaac accaattcca ttttcttatt ctaaaaatct 2880
   ggattgttgg gttgataatg aagaagatat agatgttata ttaaaaaaga gtacaatttt 2940
   aaatttagat attaataatg atattatatc agatatatct gggtttaatt catctgtaat 3000
   aacatatcca gatgctcaat tggtgcccgg aataaatggc aaagcaatac atttagtaaa 3060
   caatgaatct tctgaagtta tagtgcataa agctatggat attgaatata atgatatgtt 3120
   taataatttt accgttagct tttggttgag ggttcctaaa gtatctgcta gtcatttaga 3180
   acaatatggc acaaatgagt attcaataat tagctctatg aaaaaacata gtctatcaat 3240
   aggatctggt tggagtgtat cacttaaagg taataactta atatggactt taaaagattc 3300
   cgcgggagaa gttagacaaa taacttttag ggatttacct gataaattta atgcttattt 3360
   agcaaataaa tgggttttta taactattac taatgataga ttatcttctg ctaatttgta 3420
   tataaatgga gtacttatgg gaagtgcaga aattactggt ttaggagcta ttagagagga 3480
   taataatata acattaaaac tagatagatg taataataat aatcaatacg tttctattga 3540
   taaatttagg atattttgca aagcattaaa tccaaaagag attgaaaaat tatacacaag 3600
   ttatttatct ataacctttt taagagactt ctggggaaac cctttacgat atgatacaga 3660
   atattattta ataccagtag cttctagttc taaagatgtt caattgaaaa atataacaga 3720
   ttatatgtat ttgacaaatg cgccatcgta tactaacgga aaattgaata tatattatag 3780
   aaggttatat aatggactaa aatttattat aaaaagatat acacctaata atgaaataga 3840
   ttcttttgtt aaatcaggtg attttattaa attatatgta tcatataaca ataatgagca 3900
   cattgtaggt tatccgaaag atggaaatgc ctttaataat cttgatagaa ttctaagagt 3960
   aggttataat gccccaggta tccctcttta taaaaaaatg gaagcagtaa aattgcgtga 4020
   tttaaaaacc tattctgtac aacttaaatt atatgatgat aaaaatgcat ctttaggact 4080
   agtaggtacc cataatggtc aaataggcaa cgatccaaat agggatatat taattgcaag 4140
   caactggtac tttaatcatt taaaagataa aattttagga tgtgattggt actttgtacc 4200
   tacagatgaa ggatggacaa atgattaaac agattgatat gttcatgatt actctatata 4260
   aaaaattaaa taatataaca atctagctat attatttttg attattttct taatatatac 4320
   taataaaata atcaaaatag agcctatctt aaattactga agggctgtgt caaaataaga 4380
   ttttgacaca gcctctactt 4400
<210> 16
   <211> 1315
   <212> PRT
   <213> Clostridium tetani
<400> 16
   Met Pro Ile Thr Ile Asn Asn Phe Arg Tyr Ser Asp Pro Val Asn Asn
   1               5                   10                  15
   Asp Thr Ile Ile Met Met Glu Pro Pro Tyr Cys Lys Gly Leu Asp Ile
               20                  25                  30
   Tyr Tyr Lys Ala Phe Lys Ile Thr Asp Arg Ile Trp Ile Val Pro Glu
           35                  40                  45
   Arg Tyr Glu Phe Gly Thr Lys Pro Glu Asp Phe Asn Pro Pro Ser Ser
       50                  55                  60
   Leu Ile Glu Gly Ala Ser Glu Tyr Tyr Asp Pro Asn Tyr Leu Arg Thr
   65                  70                  75                  80
   Asp Ser Asp Lys Asp Arg Phe Leu Gln Thr Met Val Lys Leu Phe Asn
                   85                  90                  95
   Arg Ile Lys Asn Asn Val Ala Gly Glu Ala Leu Leu Asp Lys Ile Ile
               100                 105                 110
   Asn Ala Ile Pro Tyr Leu Gly Asn Ser Tyr Ser Leu Leu Asp Lys Phe
           115                 120                 125
   Asp Thr Asn Ser Asn Ser Val Ser Phe Asn Leu Leu Glu Gln Asp Pro
       130                 135                 140
   Ser Gly Ala Thr Thr Lys Ser Ala Met Leu Thr Asn Leu Ile Ile Phe
   145 150 155 160
   Gly Pro Gly Pro Val Leu Asn Lys Asn Glu Val Arg Gly Ile Val Leu
   165 170 175
   Arg Val Asp Asn Lys Asn Tyr Phe Pro Cys Arg Asp Gly Phe Gly Ser
   180 185 190
   Ile Met Gln Met Ala Phe Cys Pro Glu Tyr Val Pro Thr Phe Asp Asn
   195 200 205
   Val Ile Glu Asn Ile Thr Ser Leu Thr Ile Gly Lys Ser Lys Tyr Phe
   210 215 220
   Gln Asp Pro Ala Leu Leu Leu Met His Glu Leu Ile His Val Leu His
   225 230 235 240
   Gly Leu Tyr Gly Met Gln Val Ser Ser His Glu Ile Ile Pro Ser Lys
   245 250 255
   Gln Glu Ile Tyr Met Gln His Thr Tyr Pro Ile Ser Ala Glu Glu Leu
   260 265 270
   Phe Thr Phe Gly Gly Gln Asp Ala Asn Leu Ile Ser Ile Asp Ile Lys
   275 280 285
   Asn Asp Leu Tyr Glu Lys Thr Leu Asn Asp Tyr Lys Ala Ile Ala Asn
   290 295 300
   Lys Leu Ser Gln Val Thr Ser Cys Asn Asp Pro Asn Ile Asp Ile Asp
   305 310 315 320
   Ser Tyr Lys Gln Ile Tyr Gln Gln Lys Tyr Gln Phe Asp Lys Asp Ser
   325 330 335
   Asn Gly Gln Tyr Ile Val Asn Glu Asp Lys Phe Gln Ile Leu Tyr Asn
   340 345 350
   Ser Ile Met Tyr Gly Phe Thr Glu Ile Glu Leu Gly Lys Lys Phe Asn
   355 360 365
   Ile Lys Thr Arg Leu Ser Tyr Phe Ser Met Asn His Asp Pro Val Lys
   370 375 380
   Ile Pro Asn Leu Leu Asp Asp Thr Ile Tyr Asn Asp Thr Glu Gly Phe
   385 390 395 400
   Asn Ile Glu Ser Lys Asp Leu Lys Ser Glu Tyr Lys Gly Gln Asn Met
   405 410 415
   Arg Val Asn Thr Asn Ala Phe Arg Asn Val Asp Gly Ser Gly Leu Val
   420 425 430
   Ser Lys Leu Ile Gly Leu Cys Lys Lys Ile Ile Pro Pro Thr Asn Ile
   435 440 445
   Arg Glu Asn Leu Tyr Asn Arg Thr Ala Ser Leu Thr Asp Leu Gly Gly
   450 455 460
   Glu Leu Cys Ile Lys Ile Lys Asn Glu Asp Leu Thr Phe Ile Ala Glu
   465 470 475 480
   Lys Asn Ser Phe Ser Glu Glu Pro Phe Gln Asp Glu Ile Val Ser Tyr
   485 490 495
   Asn Thr Lys Asn Lys Pro Leu Asn Phe Asn Tyr Ser Leu Asp Lys Ile
   500 505 510
   Ile Val Asp Tyr Asn Leu Gln Ser Lys Ile Thr Leu Pro Asn Asp Arg
   515 520 525
   Thr Thr Pro Val Thr Lys Gly Ile Pro Tyr Ala Pro Glu Tyr Lys Ser
   530 535 540
   Asn Ala Ala Ser Thr Ile Glu Ile His Asn Ile Asp Asp Asn Thr Ile
   545 550 555 560
   Tyr Gln Tyr Leu Tyr Ala Gln Lys Ser Pro Thr Thr Leu Gln Arg Ile
   565 570 575
   Thr Met Thr Asn Ser Val Asp Asp Ala Leu Ile Asn Ser Thr Lys Ile
   580 585 590
   Tyr Ser Tyr Phe Pro Ser Val Ile Ser Lys Val Asn Gln Gly Ala Gln
   595 600 605
   Gly Ile Leu Phe Leu Gln Trp Val Arg Asp Ile Ile Asp Asp Phe Thr
   610 615 620
   Asn Glu Ser Ser Gln Lys Thr Thr Ile Asp Lys Ile Ser Asp Val Ser
   625 630 635 640
   Thr Ile Val Pro Tyr Ile Gly Pro Ala Leu Asn Ile Val Lys Gln Gly
   645 650 655
   Tyr Glu Gly Asn Phe Ile Gly Ala Leu Glu Thr Thr Gly Val Val Leu
   660 665 670
   Leu Leu Glu Tyr Ile Pro Glu Ile Thr Leu Pro Val Ile Ala Ala Leu
   675 680 685
   Ser Ile Ala Glu Ser Ser Thr Gln Lys Glu Lys Ile Ile Lys Thr Ile
   690 695 700
   Asp Asn Phe Leu Glu Lys Arg Tyr Glu Lys Trp Ile Glu Val Tyr Lys
   705 710 715 720
   Leu Val Lys Ala Lys Trp Leu Gly Thr Val Asn Thr Gln Phe Gln Lys
   725 730 735
   Arg Ser Tyr Gln Met Tyr Arg Ser Leu Glu Tyr Gln Val Asp Ala Ile
   740 745 750
   Lys Lys Ile Ile Asp Tyr Glu Tyr Lys Ile Tyr Ser Gly Pro Asp Lys
   755 760 765
   Glu Gln Ile Ala Asp Glu Ile Asn Asn Leu Lys Asn Lys Leu Glu Glu
   770 775 780
   Lys Ala Asn Lys Ala Met Ile Asn Ile Asn Ile Phe Met Arg Glu Ser
   785 790 795 800
   Ser Arg Ser Phe Leu Val Asn Gln Met Ile Asn Glu Ala Lys Lys Gln
   805 810 815
   Leu Leu Glu Phe Asp Thr Gln Ser Lys Asn Ile Leu Met Gln Tyr Ile
   820 825 830
   Lys Ala Asn Ser Lys Phe Ile Gly Ile Thr Glu Leu Lys Lys Leu Glu
   835 840 845
   Ser Lys Ile Asn Lys Val Phe Ser Thr Pro Ile Pro Phe Ser Tyr Ser
   850 855 860
   Lys Asn Leu Asp Cys Trp Val Asp Asn Glu Glu Asp Ile Asp Val Ile
   865 870 875 880
   Leu Lys Lys Ser Thr Ile Leu Asn Leu Asp Ile Asn Asn Asp Ile Ile
   885 890 895
   Ser Asp Ile Ser Gly Phe Asn Ser Ser Val Ile Thr Tyr Pro Asp Ala
   900 905 910
   Gln Leu Val Pro Gly Ile Asn Gly Lys Ala Ile His Leu Val Asn Asn
   915 920 925
   Glu Ser Ser Glu Val Ile Val His Lys Ala Met Asp Ile Glu Tyr Asn
   930 935 940
   Asp Met Phe Asn Asn Phe Thr Val Ser Phe Trp Leu Arg Val Pro Lys
   945 950 955 960
   Val Ser Ala Ser His Leu Glu Gln Tyr Gly Thr Asn Glu Tyr Ser Ile
   965 970 975
   Ile Ser Ser Met Lys Lys His Ser Leu Ser Ile Gly Ser Gly Trp Ser
   980 985 990
   Val Ser Leu Lys Gly Asn Asn Leu Ile Trp Thr Leu Lys Asp Ser Ala
   995 1000 1005
   Gly Glu Val Arg Gln Ile Thr Phe Arg Asp Leu Pro Asp Lys Phe
   1010 1015 1020
   Asn Ala Tyr Leu Ala Asn Lys Trp Val Phe Ile Thr Ile Thr Asn
   1025 1030 1035
   Asp Arg Leu Ser Ser Ala Asn Leu Tyr Ile Asn Gly Val Leu Met
   1040 1045 1050
   Gly Ser Ala Glu Ile Thr Gly Leu Gly Ala Ile Arg Glu Asp Asn
   1055 1060 1065
   Asn Ile Thr Leu Lys Leu Asp Arg Cys Asn Asn Asn Asn Gln Tyr
   1070 1075 1080
   Val Ser Ile Asp Lys Phe Arg Ile Phe Cys Lys Ala Leu Asn Pro
   1085 1090 1095
   Lys Glu Ile Glu Lys Leu Tyr Thr Ser Tyr Leu Ser Ile Thr Phe
   1100 1105 1110
   Leu Arg Asp Phe Trp Gly Asn Pro Leu Arg Tyr Asp Thr Glu Tyr
   1115 1120 1125
   Tyr Leu Ile Pro Val Ala Ser Ser Ser Lys Asp Val Gln Leu Lys
   1130 1135 1140
   Asn Ile Thr Asp Tyr Met Tyr Leu Thr Asn Ala Pro Ser Tyr Thr
   1145 1150 1155
   Asn Gly Lys Leu Asn Ile Tyr Tyr Arg Arg Leu Tyr Asn Gly Leu
   1160 1165 1170
   Lys Phe Ile Ile Lys Arg Tyr Thr Pro Asn Asn Glu Ile Asp Ser
   1175 1180 1185
   Phe Val Lys Ser Gly Asp Phe Ile Lys Leu Tyr Val Ser Tyr Asn
   1190 1195 1200
   Asn Asn Glu His Ile Val Gly Tyr Pro Lys Asp Gly Asn Ala Phe
   1205 1210 1215
   Asn Asn Leu Asp Arg Ile Leu Arg Val Gly Tyr Asn Ala Pro Gly
   1220 1225 1230
   Ile Pro Leu Tyr Lys Lys Met Glu Ala Val Lys Leu Arg Asp Leu
       1235                1240                1245
   Lys Thr Tyr Ser Val Gln Leu Lys Leu Tyr Asp Asp Lys Asn Ala
       1250                1255                1260
   Ser Leu Gly Leu Val Gly Thr His Asn Gly Gln Ile Gly Asn Asp
       1265                1270                1275
   Pro Asn Arg Asp Ile Leu Ile Ala Ser Asn Trp Tyr Phe Asn His
       1280                1285                1290
   Leu Lys Asp Lys Ile Leu Gly Cys Asp Trp Tyr Phe Val Pro Thr
       1295                1300                1305
   Asp Glu Gly Trp Thr Asn Asp
       1310                1315

## Description

The present invention pertains to recombinant neurotoxin polypeptides and the manufacture thereof. Specifically, it relates to a polynucleotide encoding a neurotoxin polypeptide comprising a light chain, a linker and a heavy chain, wherein the linker is a modified linker comprising an heterologous amino acid sequence which confers at least one physicochemical property to the polypeptide which allows for separation of partially processed and/or unprocessed neurotoxin polypeptides from processed neurotoxin polypeptides, said heterologous amino acid sequence being flanked N- and C-terminally by a protease recognition and cleavage site. Further encompassed by the present invention are vectors and host cells comprising the polynucleotide of the invention as well as polypeptides encoded by the said polynucleotide and methods for the manufacture of processed neurotoxin polypeptides. The neurotoxin of the invention is Botulinum neurotoxin.

Clostridium botulinum and Clostridium tetani produce highly potent neurotoxins, i.e. botulinum toxins (BoNTs) and tetanus toxin (TeNT), respectively. These Clostridial neurotoxins specifically bind to neuronal cells and disrupt neurotransmitter release. Each toxin is synthesized as an inactive single-chain protein of approximately 150 kDa. The posttranslational processing involves formation of disulfide bridges, and limited proteolysis (nicking) by bacterial protease(s). Active dichain neurotoxin consists of two chains, an N-terminal light chain of approx. 50 kDa and a heavy chain of approx. 100 kDa linked by a disulfide bond. Neurotoxins structurally consist of three domains, i.e. the catalytic light chain, the heavy chain encompassing the translocation domain (N-terminal half) and the receptor binding domain (C-terminal half), see Krieglstein 1990, Eur J Biochem 188, 39; Krieglstein 1991, Eur J Biochem 202, 41; Krieglstein 1994, J Protein Chem 13, 49.

Clostridium botulinum secretes seven antigenically distinct serotypes designated A to G of the botulinum neurotoxin (BoNT). All serotypes together with the related tetanus neurotoxin (TeNT) secreted by Clostridium tetani, are Zn²⁺-endoproteases that block synaptic exocytosis by cleaving SNARE proteins. CNTs cause the flaccid muscular paralysis seen in botulism and tetanus, see Fischer 2007, PNAS 104, 10447.

Despite its toxic effects, botulinum toxin complex has been used as a therapeutic agent in a large number of diseases. Botulinum toxin serotype A was approved for human use in the United States in 1989 for the treatment of strabism, blepharospasm, and other disorders. It is commercially available as a Botulinum toxin A protein preparation, for example, under the tradename BOTOX (Allergan Inc) and under the tradename DYSPORT (Ipsen Ltd). For therapeutic application the complex is injected directly into the muscle to be treated. At physiological pH, the toxin is released from the protein complex and the desired pharmacological effect takes place. An improved BoNT/A preparation being free of complexing proteins is available under the tradename XEOMIN (Merz Pharmaceuticals GmbH). The effect of Botulinum toxin is only temporary, which is the reason why repeated administration of Botulinum toxin may be required to maintain a therapeutic effect.

The Clostridial neurotoxins weaken voluntary muscle strength and are effective in the treatment of strabism, focal dystonia, including cervical dystonia, and benign essential blepharospasm. They have been further shown to relief hemifacial spasm, and focal spasticity, and moreover, to be effective in a wide range of other indications, such as gastrointestinal disorders, hyperhidrosis, and cosmetic wrinkle correction, see Jost 2007, Drugs 67, 669.

For the manufacture of Clostridial neurotoxins, whether isolated from the originating wild-type clostridial bacteria, or from other host cell species such as E. coli, the purification of the neurotoxin from the host cell lysates after fermentation is of particular importance. In the classical production process *from Clostridium Botulinum,* different precipitation- and extraction steps followed by a concentration step and further distinct chromatographic steps are usually applied in order to obtain purified neurotoxin, see DasGupta 1984, Toxicon 22, 415; Sathyamoorthy 1985, J Biol Chemistry 260, 10461. Similarly, in a recombinant production process from other host cells such as E. coli, various processing steps such as cell rupture, lysate clarification, and multiple chromatographic purification steps are used for isolation of the neurotoxin.

Currently, all available neurotoxin preparations comprise, in addition to the desired active (processed) dichain neurotoxin, a proteolytically unprocessed precursor and/or partially processed neurotoxin polypeptide. The proteolytically unprocessed precursor or partially processed polypeptide differs from the active (processed) dichain neurotoxin polypeptide in only a few amino acids. Therefore, they can hardly be distinguished based on their chemical and physical properties. On the other hand, the ratio of proteolytically unprocessed precursor and/or partially processed neurotoxin polypeptide of the total protein ratio is still significant in such preparations. Said amount of unprocessed precursor and/or partially processed neurotoxin is inherent to the biological production systems, which are not completely controllable. Thus, the amount of undesired proteolytically unprocessed precursor and/or partially processed Neurotoxin polypeptide in Neurotoxin preparations is predefined and, currently, rather difficult to reduce.

Processing of neurotoxins was also achieved by incubation of the precursor polypeptide with an E. coli lysate containing a protease activity elicited by an unknown E. coli protease which is capable of cleaving the neurotoxin polypeptides such that mature dichain polypeptides are obtained (see WO2006/076902).

WO2004/024909 discloses a Botulinum neurotoxin which is capable of simultaneous activation and removal of a purification tag.

WO2010/124998, which is prior art under Art. 54(3), discloses a Botulinum neurotoxin containing a linker sequence flanked by protease sites between the light and heavy chains.

Means and methods for a more efficient manufacture of neurotoxins by reducing the amount of the unprocessed and/or partially processed neurotoxin polypeptides and thereby improving the quality of neurotoxin preparations are highly desirable but not yet available.

Thus, the technical problem underlying the present invention may be seen as the provision of means and methods for improving the manufacture of neurotoxin polypeptides by complying with the aforementioned needs. The technical problem is solved by the embodiments characterized in the claims and herein below.

The present invention relates, thus, to a polynucleotide encoding a neurotoxin polypeptide comprising a light chain, a linker and a heavy chain, wherein the linker is a modified linker comprising an heterologous amino acid sequence which confers at least one physicochemical property to the polypeptide which allows for separation of partially processed and/or unprocessed neurotoxin polypeptides from processed neurotoxin polypeptides, said heterologous amino acid sequence being flanked N- and C-terminally by a protease recognition and cleavage site.

The term "polynucleotide" as used herein refers to single- or double-stranded DNA molecules as well as to RNA molecules. Encompassed by the said term is genomic DNA, cDNA, hnRNA, mRNA as well as all naturally occurring or artificially modified derivatives of such molecular species. The polynucleotide may be in an aspect a linear or circular molecule. Moreover, in addition to the nucleic acid sequences encoding the aforementioned neurotoxin polypeptide, a polynucleotide of the present invention may comprise additional sequences required for proper transcription and/or translation such as 5'- or 3'-UTR sequences. The polynucleotide of the present invention encodes a neurotoxin polypeptide comprising a modified, linker. The neurotoxin polypeptide and, in particular, its light chain and heavy chain are derivable from one of the antigenically different serotypes of Botulinum Neurotoxins, i.e. BoNT/A, BoNT/B, BoNT/Cl, BoNT/D, BoNT/E, BoNT/F, BoNT/G, or Tetanus Neurotoxin (TeNT). In an aspect, said light and heavy chain of the neurotoxin polypeptide are the light and heavy chain of a neurotoxin selected from the group consisting of: BoNT/A, BoNT/B, BoNT/C1, BoNT/D, BoNT/E, BoNT/F, BoNT/G or TeNT. In another aspect, the said polynucleotide comprises a nucleic acid sequence as shown in SEQ ID NO: 1 (BoNT/A), SEQ ID NO: 3 (BoNT/B), SEQ ID NO: 5 (BoNT/C1), SEQ ID NO: 7 (BoNT/D), SEQ ID NO: 9 (BoNT/E), SEQ ID NO: 11 (BoNT/F), SEQ ID NO: 13 (BoNT/G) or SEQ ID NO: 15 (TeNT) which has been modified in the linker as specified herein. Moreover, encompassed is in an aspect a polynucleotide comprising a nucleic acid sequence encoding an amino acid sequence as shown in any one of SEQ ID NO: 2 (BoNT/A), SEQ ID NO: 4 (BoNT/B), SEQ ID NO: 6 (BoNT/C1), SEQ ID NO: 8 (BoNT/D), SEQ ID NO: 10 (BoNT/E), SEQ ID NO: 12 (BoNT/F), SEQ ID NO: 14 (BoNT/G) or SEQ ID NO: 16 (TeNT) which has been modified in the linker as specified herein.

The invention is limited to Botulinum neurotoxin as the toxin. The embodiments relating to Tenanus neurotoxin are disclosed for illustration only.

In another aspect, the said polynucleotide is a variant of the aforementioned polynucleotides comprising one or more nucleotide substitutions, deletions and/or additions which in still another aspect may result in a polypeptide having one or more amino acid substitutions, deletions and/or additions. Moreover, a variant polynucleotide of the invention shall in another aspect comprise a nucleic acid sequence variant being at least 40%, at least 50%, at least 60%, at least 70%, at least 75%, at least 80%, at least 85%, at least 90%, at least 95%, at least 98% or at least 99% identical to the nucleic acid sequence as shown in any one of SEQ ID NOs: 1, 3, 5, 7, 9, 11, 13 or 15 or a nucleic acid sequence variant which encodes an amino acid sequence being at least 40%, at least 50%, at least 60%, at least 70%, at least 75%, at least 80%, at least 85%, at least 90%, at least 95%, at least 98% or at least 99% identical to the amino acid sequence as shown in any one of SEQ ID NOs: 2, 4, 6, 8, 10, 12, 14, or 16. The term "identical" as used herein refers to sequence identity characterized by determining the number of identical amino acids between two nucleic acid sequences or amino acid sequences wherein the sequences are aligned so that the highest order match is obtained. It can be calculated using published techniques or methods codified in computer programs such as, for example, BLASTP, BLASTN or FASTA (Altschul 1990, J Mol Biol 215, 403). The percent identity values are, in one aspect, calculated over the entire amino acid sequence. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Higgins 1989, CABIOS 5, 151) or the programs Gap and BestFit (Needleman 1970, J Mol Biol 48; 443; Smith 1981, Adv Appl Math 2, 482), which are part of the GCG software packet (Genetics Computer Group 1991, 575 Science Drive, Madison, Wisconsin, USA 53711), may be used. The sequence identity values recited above in percent (%) are to be determined, in another aspect of the invention, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments. In an aspect, each of the aforementioned variant polynucleotides encodes a polypeptide retaining one or more and, in another aspect, all of the biological properties of the respective Neurotoxin polypeptide, i.e. the BoNT/A, BoNT/B, BoNT/C1, BoNT/D, BoNT/E, BoNT/F, BoNT/G or Tetanus Neurotoxin (TeNT). Those of skill in the art will appreciate that full biological activity is maintained only after proteolytic activation, even though it is conceivable that the unprocessed precursor can exert some biological functions or be partially active. "Biological properties" as used herein refers to (a) receptor binding, (b) internalization, (c) translocation across the endosomal membrane into the cytosol, and/or (d) endoproteolytic cleavage of proteins involved in synaptic vesicle membrane fusion. In vivo assays for assessing biological activity include the mouse LD50 assay and the ex vivo mouse hemidiaphragm assay as described by Pearce et al. (Pearce 1994, Toxicol Appl Pharmacol 128: 69-77) and Dressier et al. (Dressier 2005, Mov Disord 20:1617-1619, Keller 2006, Neuroscience 139: 629-637). The biological activity is commonly expressed in Mouse Units (MU). As used herein, 1 MU is the amount of neurotoxic component, which kills 50% of a specified mouse population after intraperitoneal injection, i.e. the mouse i.p. LD50. In a further aspect, the variant polynucleotides can encode Neurotoxins having improved or altered biological properties, e.g., they may comprise cleavage sites which are improved for enzyme recognition or may be improved for receptor binding or any other property specified above.

Neurotoxin polypeptides as referred to herein comprise an N-terminal light chain, an intermediate linker and a C-terminal heavy chain. The neurotoxins are translated as single chain precursor molecules and become proteolytically cleaved into a mature dichain form during processing. Proteolytical cleavage occurs at the linker in a manner such that the linker is either cleaved once or is removed after cleavage at the N- and C-termini of the linker. Dependent on the efficacy of the processing, in addition to the uncleaved single chain construct, partially processed neurotoxin polypeptides may also be generated, i.e. neurotoxin polypeptides which retain the linker either at the light or heavy chain due to improper cleavage. The endogenous linker of the neurotoxin polypeptides is modified in accordance with the present invention such that it comprises a heterologous (i.e. not endogenous) amino acid sequence which binds with high affinity to a purification matrix, said heterologous amino acid sequence being flanked N- and C-terminally by a protease recognition and cleavage site. Said modification, in an aspect, may be a modification of individual amino acids in the endogenous linker yielding the modified linker. Alternatively and in another aspect, said modification may be the replacement of the endogenous linker by a heterologous linker. Suitable techniques for carrying out such modifications to the linker are well known in the art and include mutagenesis techniques as well as standard cloning and PCR based techniques.

The term "heterologous amino acid sequence which confers at least one physicochemical property to the polypeptide which allows for separation of partially processed or unprocessed neurotoxin polypeptides from processed neurotoxin polypeptides" refers to an amino acid sequence which does not naturally occur in the linker (i.e. heterologous) of the neurotoxin to be modified and which confers at least one physicochemical property to the neurotoxin molecular species comprising the said linker, i.e. the unprocessed and/or partially processed neurotoxin. The said physicochemical property, in an aspect, shall be an altered affinity for a purification matrix as defined elsewhere herein in detail or may be an altered physical property such as the molecular weight which allows for efficient physical separation.

In an aspect of the polynucleotide of the present invention, said heterologous amino acid sequence is a heterologous amino acid sequence which binds with high affinity to a purification matrix.

The term "heterologous amino acid sequence which binds with high affinity to a purification matrix" relates to an amino acid sequence which does not endogenously (i.e. naturally occur) in the linker. Said amino acid sequence shall confer high affinity binding to a purification matrix. Dependent on the purification matrix to be used, this can be achieved either by individual amino acids which are present in the sequence and which are capable of physically or chemically interacting with matrix components or peptide sequence stretches or three-dimensional structures formed thereby being capable of interacting with the matrix. The latter case of high affinity binding includes also binding based on protein-protein interactions. Suitable high affinity protein-protein interactions which can be applied in this context are antibody-epitope interactions. For example, the purification matrix may contain an antibody which specifically recognizes and binds with high affinity to a epitope comprised by the heterologous amino acid sequence. Further, receptor-ligand or other hight affinity protein-protein interactions can be applied. For example, the purification matrix may be coupled to biotin and the heterologous amino acid sequence may comprise streptavidin or avidin or tags derived therefrom. Further heterologous amino acid sequences to be used in particular aspects of the present invention are specified elsewhere herein. A "purification matrix" as meant herein refers to a three dimensional structure or spatial arrangement capable of binding to the aforementioned amino acid sequence of the modified linker. Well-known matrices comprise polypeptides, glass, polystyrene, polypropylene, polyethylene, polyethylene glycol (PEG), dextran, nylon, amylases, natural and modified celluloses, polyacrylamides, gabbros, and magnetite. A solid matrix is, in an aspect of the invention, a polysaccharide matrix selected from the group consisting of: sepharose, sephadex, superdex; agarose, sephacell, micro-cellulose, and alginate-beads. In another aspect, said solid matrix can consist of glass-beads, and/or polypeptide matrices.

In a further aspect of the polynucleotide of the invention, said heterologous amino acid sequence increases the molecular weight of the neurotoxin polypeptide such that the partially processed and/or unprocessed neurotoxin polypeptides can be physically separated from processed neurotoxin polypeptide.

The said heterologous amino acid sequence which is comprised in this aspect by the linker will increase the molecular weight of the linker containing neurotoxin molecular species such that the said linker containing molecular species can be separated from the processed neurotoxin, i.e. the molecular species which does not contain the modified linker, by physical separation processes. The said processes, in an aspect, include size exclusion chromatography, ultrafiltration, preparative gel electrophoresis technologies, or centrifugation based technologies. Amino acids which can be introduced into the modified linker to increase te molecular weight include essentially all available naturally occurring or artificial amino acids which are capable of being introduced via peptide bonds into the peptide backbone. Moreover, in an aspect the amino acids may be chemically modified in order to improve or facilitate the physical separation. In such a case, moieties which contribute a high molecular weight can be coupled to the amino acids. In another aspect, a larger stretch of unmodified amino acids may be sufficient to increase the molecular weight of the modified linker to an extent which allows for physical separation.

It will be understood that in an aspect, the modified linker comprises (i) a heterologous amino acid sequence which binds with high affinity to a purification matrix and (ii) a heterologous amino acid sequence which increases the molecular weight of the neurotoxin polypeptide such that the partially processed and/or unprocessed neurotoxin polypeptides can be physically separated from processed neurotoxin polypeptide.

The said heterologous amino acid sequence shall be N- and C-terminally flanked by a protease recognition and cleavage site. It is envisaged by the present invention that the linker will be entirely removed after the processing is completed. This can be achieved by introducing two protease recognition and cleavage sites flanking the N- and C-terminal end of the linker sequence. Upon cleavage of the cleavage sites, a peptide with the linker sequence will be released and the dimerized light and heavy chain of the neurotoxin polypeptide remain as the mature dichain neurotoxin polypeptide. In an aspect, it is envisaged to introduce at the N- and C-terminal end of the linker sequence recognition and cleavage sites for the same protease. In another aspect, it is envisaged to introduce at the N- and C-terminal end of the linker sequence recognition and cleavage sites for different proteases. Dependent on the protease to be used in accordance with the present invention, the protease recognition site may differ from the cleavage site or may be identical thereto. Moreover, an exogenous protease can be used in an aspect according to the present invention. Alternatively, an endogenous protease activity which is present in the linker or other parts of the neurotoxin polypeptide, such as the neurotoxin protease activity, encoded by the polynucleotide of the invention can be used in another aspect of the invention. The latter autocatalytic protease cleavage system for a *single* cleavage site has been described in more detail in US 7,556,817 which is herewith incorporated by reference. In an aspect, said protease recognition and cleavage site is selected from the group consisting of: neurotoxin light chain protease recognition and cleavage site from SNAP 25, recognition and cleavage site from an E. coli protease, Thrombin recognition and cleavage site, Factor X recognition and cleavage site, Trypsin recognition and cleavage site, Enterokinase recognition and cleavage site, TEV protease recognition and cleavage site, HRV 3c recognition and cleavage site, and PreScission Protease recognition and cleavage site.

Advantageously, it has been found in accordance with the present invention that a neurotoxin polypeptide with a modified linker as specified above can be more efficiently purified due to the presence of heterologous amino acid sequences conferring altered physicochemical properties to linker containing polypeptides which allow for an efficient and reliable purification, such as affinity binding to a matrix or increased molecular weight which allows for improved physical separation. Moreover, solubility can be increased dependent on the size and/or the hydrophilicity of the linker. In addition, further properties which are suitable for the manufacture of neurotoxin polypeptides can be introduced into the linker, such as detectable markers, e.g., fluorescent proteins. These properties persist only during processing since the respective amino acid sequence will be removed as a result of the processing steps from the mature neurotoxin polypeptides.

In an aspect of the polynucleotide of the present invention, said heterologous amino acid sequence which binds with high affinity to a purification matrix comprises a purification tag.

The term "purification tag" refers to an amino acid sequence which allows for purification of the polynucleotide comprising it. This can be achieved in an aspect due to the presence of individual amino acids, peptide sequences or three dimensional peptide structures which are capable of physically or chemically interacting with the purification matrix. Suitable tags include affinity tags and epitope tags. In this context, it is well known that certain amino acids are capable of forming complexes with defined matrices to be used for purification, such as histidines which are capable of physico-chemically interacting with metal ions such as nickel or cobalt embedded in a suitable matrix such as sepharose or agarose. Further peptide sequences or three dimensional structures can be found in chitin binding proteins (CBP), maltose binding proteins (MBP) or glutathione-S-transferases (GST). In a further aspect, protein-protein interaction based purification matrix/purification tag systems can be used such as streptavidin/biotin systems which are also well known in the art. In yet another aspect, the tag may confer a physic-chemical property to the polypeptide containing it which allows for purification, such as fluorescence. Suitable tags in this context include the well known fluorescent proteins, such as green fluorescent protein (GFP), blue fluorescent protein (BFP) or others. In addition to these tags which have an inherent affinity for some matrices, epitope tags can be applied in another aspect. Such epitope tags consists of short amino acid stretches which constitute a peptide epitope that can be specifically recognized by an antibody. Epitope tags are also well known in the art and include FLAG tags, MYC-tags, or HA-tags. Thus, in an aspect, the purification tag is selected from the group consisting of: His-tag, Myc-tag, FLAG-tag, strep-tag, MBP-tag, NusA tag, GST-tag, streptavidin and avidin.

In another aspect of the polynucleotide of the invention, said heterologous amino acid sequence which binds with high affinity to a purification matrix comprises (i) at least one amino acid domain conferring high affinity binding to the purification matrix and (ii) at least one amino acid domain which prevents interference of the heterologous amino acid sequence with the formation of proper disulfide bonds between the light and heavy chain.

In an aspect, said at least one domain conferring high affinity binding to the purification matrix comprises a purification tag as described elsewhere herein. In a further aspect, said at least one amino acid domain enables and/or facilitates proper disulfide bond formation between the heavy and the light chain and/or prevents interference of the heterologous amino acid sequence with the formation of proper disulfide bonds between the light and heavy chain. In an aspect, the said domain forms a three dimensional structure wherein the C- and N-terminal amino acids of the heterologous amino acid sequence are in physical proximity. As a result of the physical proximity of the N- and C-terminal amino acid which, in an aspect, is in the range of the proximity of the sulphur atoms of a disulphide bound, i.e. about 2.0 Angström, the remaining part of the linker will protrude from the light and heavy chain of the neurotoxin molecule. Accordingly, the properties which are conferred by the linker such as high affinity binding or an increased solubility of the molecule, are not in physical proximity and, thus, do not interfere with proper intramolecular disulphide bond formation between the light and the heavy chain. Moreover, in an aspect the protruding linker also prevents sterically the formation of intermolecular disulphide bond formation between separate neurotoxin molecules. In an aspect, the said three dimensional structure, which brings the cysteine residues forming the intramolecular disulphide bridge between light and heavy chain in close proximity, is an antiparallel coiled coil or an antiparallel beta-sheet structure. In an aspect, said antiparallel coiled coil is derived from 1FHA ferritin, 1VSG variant surface glycoprotein, 1LIGb Asp receptor, 1PRCm photosynthetic reaction centre, 2CCY cytochrome C', 256B cyctochrome b562, 3LZM T4 lysozyme, 1COL1a colicin, 1PRC1 photosynthetic reaction centre, 2CTS citrate synthase, 1ROP Rop protein, 3HHR human growth hormone or 1 LPE apolipoprotein A3. How to generate such structures is well known in the art and described, e.g., in Gernert 1995, Protein Science 4(11): 2252-22560 or Hadley 2008, Proc Natl. Acad. Sci. USA 15:105(2):530-5, both are herewith incorporated by reference.

In another aspect of the polynucleotide of the invention, said heterologous amino acid sequence further comprises at least one detectable marker amino acid sequence.

In an aspect, said detectable marker amino acid sequence is selected from the group consisting of: an amino acid sequence of a fluorescent protein, an amino acid sequence of an enzyme capable of generating a detectable signal, and an amino acid sequence of a detectable tag. In an aspect, the fluorescent protein is GFP or BFP. In another aspect, the enzyme capable of generating a detectable signal is selected from beta galactosidase (GAL4), firefly luciferase, or chloramphenicol transferase (CAT). In yet another aspect, the detectable tag is an epitope tag such as those specified elsewhere in this specification (NusA-Tag).

In another aspect, the modified linker comprises further nucleic acid sequences which encode polypeptide domains conferring properties to the polypeptide which improve the purification or enable or improve solubility. Such polypeptide domains, in an aspect, may be additional purification tags as recited elsewhere herein and which allow for an improved purification via affinity binding. Further, polypeptide domains may enable or facilitate solubility due to the presence of hydrophilic amino acid residues. Suitable domains are well known in the art.

It is to be understood that the definitions and explanations of the terms made above apply mutatis mutandis for all aspects described in this specification in the following except as otherwise indicated.

The present invention also relates to a vector comprising the polynucleotide of the present invention.

The term "vector", preferably, encompasses phage, plasmid, viral or retroviral vectors as well as artificial chromosomes, such as bacterial or yeast artificial chromosomes. Moreover, the term also relates to targeting constructs which allow for random or site- directed integration of the targeting construct into genomic DNA. Such target constructs, preferably, comprise DNA of sufficient length for either homologous or heterologous recombination as described in detail below. The vector encompassing the polynucleotides of the present invention, in an aspect, further comprises selectable markers for propagation and/or selection in a host. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerens. Alternatively, a plasmid vector may be introduced by heat shock or electroporation techniques. Should the vector be a virus, it may be packaged in vitro using an appropriate packaging cell line prior to application to host cells. Retroviral vectors may be replication competent or replication defective. In the latter case, viral propagation generally will occur only in complementing host/cells. Moreover, in an aspect of the invention, the polynucleotide is operatively linked to expression control sequences allowing expression in prokaryotic or eukaryotic host cells or isolated fractions thereof in the said vector. Expression of the polynucleotide comprises transcription of the polynucleotide into a translatable mRNA. Regulatory elements ensuring expression in host cells are well known in the art. In an aspect, they comprise regulatory sequences ensuring initiation of transcription and/or poly-A signals ensuring termination of transcription and stabilization of the transcript. Additional regulatory elements may include transcriptional as well as translational enhancers. Possible regulatory elements permitting expression in prokaryotic host cells comprise, e.g., the lac-, trp- or tac- promoter in E. coli, and examples for regulatory elements permitting expression in eukaryotic host cells are the AOX1- or the GAL1- promoter in yeast or the CMV-, SV40-, RSV-promoter (Rous sarcoma virus), CMV-enhancer, SV40-enhancer or a globin intron in mammalian and other animal cells. Other expression systems envisaged by the invention shall permit expression in insect cells, such as polyhedrin promoter based systems.

Moreover, inducible expression control sequences may be used in an expression vector encompassed by the present invention. Such inducible vectors may comprise tet or lac operator sequences or sequences inducible by heat shock or other environmental factors. Suitable expression control sequences are well known in the art. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pBluescript (Stratagene), pCDM8, pRc/CMV, pcDNA1, pcDNA3 (Invitrogen) or pSPORT41 (Invitrogen) or baculovirus-derived vectors. Preferably, said vector is an expression vector and a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994).

The present invention further pertains to a host cell comprising the polynucleotide or the vector of the present invention.

The term "host cell" as used herein encompasses prokaryotic and eukaryotic host cells. In an aspect the host cell is a bacterial cell and, in another aspect, a Firmicutes bacterial cell. In one aspect, the said bacterial host cell is an E.coli host cell. In another aspect, it is a Clostridium host cell. In a further aspect, the said Clostridium host cell is a Clostridium botulinum host cell, in even a further aspect, a cell of one of the aforementioned seven different serotypes of Clostridium botulinum. In yet another aspect, the bacterial host cell is a Clostridium tetani host cell. In a further aspect, the host cell is a Bacillus host cell and in a particular aspect a Bacillus megaterium host cell. A eukaryotic host cell, in an aspect, is a cell of an animal cell line suitable for production of toxic proteins or a fungal host cell such as a yeast host cell. A host cell as referred to herein, thus, encompasses in an aspect yeast, mammalian, plant or insect cells either as primary cells or as cell lines.

The present invention also relates to a polypeptide encoded by the polynucleotide of the present invention.

The term "polypeptide" as used herein encompasses isolated or essentially purified polypeptides being essentially free of other polypeptides including the complexing proteins (HA70, HA17, HA33, or NTNH (NBP)) of the host cell or polypeptide preparations comprising other proteins in addition. Moreover, the term includes chemically modified polypeptides. Such modifications may be artificial modifications or naturally occurring modifications. As referred to above, the polypeptide of the present invention shall have the biological properties of the Neurotoxin polypeptides referred to above. The polypeptide of the invention, in an aspect, can be manufactured by a method of manufacturing a polypeptide as described elsewhere herein in more detail. In an aspect of the invention, a polypeptide preparation is also envisaged which comprises a complex of the Neurotoxin polypeptide and its complexing proteins referred to above.

Moreover, the present invention encompasses a method for the manufacture of a processed neurotoxin polypeptide comprising the steps of:
a) contacting the unprocessed neurotoxin polypeptide of the present invention with a purification matrix under conditions and for a time sufficient for allowing binding of the said unprocessed neurotoxin polypeptide to the purification matrix;
b) contacting the unprocessed neurotoxin polypeptide bound to the purification matrix with a protease which is capable of cleaving the neurotoxin polypeptide at the protease recognition and cleavage sites resulting in processed and partially processed and/or unprocessed neurotoxin polypeptides; and
c) removing the released, processed neurotoxin polypeptide from the purification matrix.

It will be understood that the methods for manufacturing neurotoxin polypeptides specified herein may comprise additional steps such as steps pertaining to the synthesis of the neurotoxins, washing steps or steps concerning the formulation of the purified neurotoxin polypeptides into neurotoxin preparations which can be applied as pharmaceutical or cosmetic compositions, respectively.

The term "contacting" as used herein refers to bringing at least two different compounds in physical proximity as to allow physical and/or chemical interaction of said compounds. In the aforementioned method, unprocessed neurotoxin according to the present invention, i.e. neurotoxin polypeptides comprising a modified linker as specified above, is contacted with a purification matrix for a time and under conditions sufficient to allow binding of said unprocessed neurotoxin to the matrix. The said time and conditions will dependent on the type of binding which shall be achieved between the linker and the matrix. The person skilled in the art is well aware of which conditions need to be applied for a given combination of purification matrix and linker. Contacting may be carried out by means of chromatography or batch-wise. Upon binding of the unprocessed neurotoxin, washing steps may be performed in order to remove (recover) unbound unprocessed neurotoxin and other host cell proteins.

Subsequently, the unprocessed neurotoxin polypeptide bound to the purification matrix shall be contacted with a protease which is capable of cleaving the neurotoxin polypeptide at the protease recognition and cleavage sites under time and conditions which allow for protease activity. The protease in this context may be provided as a purified enzyme preparation or as a cell lysate from a cell expressing said protease endogenously or exogenously. As a result of the protease activity, the neurotoxin polypeptides with the modified linker will be cleaved into processed and partially processed neurotoxin polypeptides. Some neurotoxin polypeptides may even remain in uncleaved form as unprocessed neurotoxin polypeptides. Suitable conditions for the aforementioned protease treatment depend on the protease to be used and can be adapted by the skilled artisan without further ado.

The processed neurotoxin polypeptides will upon cleavage loose their modified linkers which are responsible for binding to the purification matrix and are, thus, released from the matrix. Contrary, the partially processed or unprocessed neurotoxin polypeptides remain bound to the purification matrix due to the presence of the modified linkers in said molecules. The mature, processed neurotoxin polypeptides can be removed from the purification matrix by washing with an appropriate buffer or water.

In a particular aspect of the aforementioned method, the linker of the neurotoxin polypeptide comprises an epitope tag as specified elsewhere herein and the purification matrix contains an antibody which specifically binds to the said epitope tag. In an aspect, said purification matrix is applied for column affinity chromatography as follows. A solution comprising the unprocessed neurotoxin polypeptide according to the invention is applied to the column. The solution is maintained in the column for a time and under conditions which allow specific binding of the epitope tags in the modified linkers of the unprocessed neurotoxin polypeptides to the purification matrix. Subsequently, excessive unprocessed neurotoxin polypeptide is removed by washing steps. After said washing steps, a solution comprising a protease capable of recognizing and cleaving the recognition and cleavage sites flanking the modified linker is applied. The unprocessed neurotoxin polypeptide bound to the purification matrix is incubated with the protease as to allow proteolytic cleavage at both cleavage sites. As a consequence of the said cleavage reaction, mature, processed neurotoxin polypeptide is released from the purification matrix while unprocessed or partially processed neurotoxin polypeptide remains bound to the purification matrix due to its association with the linker. The released processed neurotoxin can then be eluted from the column.

In another particular aspect of the aforementioned method, the linker of the neurotoxin polypeptide comprises a poly-histidine tag, such as a hexa-His tag, as specified elsewhere herein and the purification matrix represents a nickel-, cobalt-, copper- and zinc- containing sepharose or other chromatography media. In an aspect, said purification matrix is applied for column affinity chromatography as follows. A solution comprising the unprocessed neurotoxin polypeptide according to the invention is applied to the column. The solution is maintained in the column for a time and under conditions which allow specific binding of the hexa-His tag in the modified linkers of the unprocessed neurotoxin polypeptides to the nickel or cobalt ions in the purification matrix. Subsequently, excessive unprocessed neurotoxin polypeptide and especially contaminating host cell proteins are removed by washing steps. After said washing steps, a solution comprising a protease capable of recognizing and cleaving the recognition and cleavage sites flanking the modified linker is applied. The unprocessed neurotoxin polypeptide bound to the purification matrix is incubated with the protease as to allow proteolytic cleavage at both cleavage sites. As a consequence of the said cleavage reaction, mature, processed neurotoxin polypeptide is released from the purification matrix while unprocessed or partially processed neurotoxin polypeptide remains bound to the purification matrix due to its association with the linker. The released processed neurotoxin can than be eluted from the column.

Further, the present invention pertains to a method for the manufacture of a processed neurotoxin polypeptide comprising the steps of:
a) contacting a neurotoxin polypeptide of the present invention with a protease which is capable of cleaving the neurotoxin polypeptide at the protease recognition and cleavage sites;
b) contacting the processed, unprocessed and/or partially processed neurotoxin polypeptide obtained in step a) with a purification matrix under conditions and for a time sufficient for allowing binding of the said unprocessed and/or partially processed neurotoxin polypeptide to the purification matrix; and
c) removing the released, processed neurotoxin polypeptide from the purification matrix.

In an aspect of the aforementioned method, step a) is carried out in a host cell expressing the polynucleotide of the invention and a protease which is capable of cleaving the neurotoxin polypeptide at the protease recognition and cleavage sites. In an aspect, said protease can be expressed endogenously in the said host cell. In another aspect, the said protease is expressed from an expression construct which has been introduced into the said host cell. In an aspect, the host cell is a host cell lysate or a host cell as referred to herein elsewhere.

Moreover, the invention contemplates a method for the manufacture of a processed neurotoxin polypeptide comprising the steps of:
a) contacting a neurotoxin polypeptide of the present invention with a protease which is capable of cleaving the neurotoxin polypeptide at the protease recognition and cleavage sites;
b) subjecting the processed, unprocessed and/or partially processed neurotoxin polypeptide obtained in step a) to physical conditions for a time sufficient for allowing physical separation of partially processed or unprocessed neurotoxin polypeptides from processed neurotoxin polypeptides; and
c) removing processed neurotoxin polypeptide after said physical separation.

The term "subjecting" as used herein refers to applying the physical conditions to the processed, unprocessed and/or partially processed neurotoxin polypeptide. Dependent on the physical conditions which are applied for separation, the person skilled in the art knows how said subjecting may be carried out. In an aspect, subjecting comprises performing size exclusion chromatography, the principles of which are further defined elsewhere herein. In another aspect, subjecting comprises filtration, e.g., ultrafiltration. In another aspect, subjecting comprises centrifugation, e.g., ultracentrifugation. In yet another aspect, subjecting comprises gel electrophoresis, in an aspect, in a preparative scale. After the molecular species of the neurotoxin present after cleavage have been physically separated into the fully processed neurotoxin polypeptide molecules and the unprocessed and/or partially processed neurotoxin molecules, the said processed neurotoxin polypeptides can be removed by suitable measures well known in the art. If in an aspect size exclusion chromatography is applied for physical separation, the processed neurotoxin polypeptides can be obtained from a size exclusion chromatography column by collecting the eluate into different fractions. The processed and the unprocessed and/or partially processed neurotoxin polypeptides will be present in different fractions in such a case.

In another aspect of the methods of the invention, the manufacture of the neurotoxin polypeptide comprises further purification steps. In one aspect, said further purification steps comprise size exclusion chromatography.

By size exclusion chromatography as used in the present invention, particles are separated based on their size, i.e. on their hydrodynamic volume. A mobile phase is either an aqueous solution used to transport the sample (gel filtration chromatography), or an organic solvent (gel permeation chromatography). A stationary phase is either a gel medium (polyacrylamide, dextran or agarose) and filter under low pressure, or a silica, or crosslinked polystyrene medium under a higher pressure. In even another aspect, said size exclusion chromatography is performed as column chromatography. In a further aspect of the method of the present invention, said size exclusion chromatography is performed using molecular sieves with distinct pore sizes such as activated carbon, silica gel, zeolite.

The method of the present invention, in another aspect, further comprises ion exchange chromatography.

Ion exchange chromatography as used in the present invention separates molecules based on differences between the overall charge of the proteins and related compounds. It is used for protein purification, for purification of oligonucleotides, peptides, or other charged molecules. Such molecules may be present in the solution to be applied to the method of the purification as contaminations. The protein or the related compound of interest, in the present case the Neurotoxin, must have a charge opposite to that of the functional group attached to the resin in order to bind. Because this interaction is ionic, binding must take place under low ionic conditions. Elution is achieved by increasing the ionic strength to break up the ionic interaction, or by changing the pH of the protein. In an aspect of the method of the invention, said exchange chromatography is performed as column chromatography. The ion exchange chromatography as used in the present invention is in a further aspect performed by cation and/or anion chromatography. In anion exchange chromatography as used herein the surface charge of the solutes (proteins, peptides, nucleic acids, endotoxins) which bind will be net negative, thus to get binding of a specific protein one should be near or above the pI of that protein. Commonly used anion exchange resins are Q-resin (Q Sepharose), a Quaternary amine; and DEAE (DiEthylAminoEthane) resin. Generally, an ion exchange resin is an insoluble matrix of small beads having a charged surface, used as an artificial zeolite. Different types of resins can be distinguished based on their functional groups including strongly acidic resins (sulfonic acid groups, eg. sodium polystyrene sulfonate or polyAMPS), strongly basic resins, (quaternary amino groups, e.g. trimethylammonium groups, eg. polyAPTAC), weakly acidic resins (mostly, carboxylic acid groups), weakly basic resins (primary, secondary, and/or ternary amino groups, e.g., polyethylene amine). There are also specialised types of resins can be further distinguishes including chelating resins (iminodiacetic acid, thiourea). In cation exchange chromatography as used herein, the surface charge of the solutes (proteins, peptides, nucleic acids, endotoxins) which bind will be net positive, thus to get binding of a specific protein one should be near or below the pI of that protein. Commonly used cation exchange resins are S-resin, sulfate derivatives; and CM resins, carboxylate derived ions.

Further purification techniques which can be used in additional purification steps according to the method of the present invention include hydrophobic interaction chromatography, multimodal chromatography (e.g. Capto MMC or Capto Adhere), affinity chromatography (e.g. Blue Sepharose) and/or hydroxyapatite matrices.

In an aspect of the method of the present invention said ion exchange chromatography is carried out prior to and/or after contacting the processed, unprocessed and/or partially processed neurotoxin polypeptide with the purification matrix. In another aspect of the method of the invention, said ion exchange chromatography as used herein is carried out prior to contacting the processed, unprocessed and/or partially processed neurotoxin polypeptide with the purification matrix. Due to this measure, the risk of potential cross-reactivity or unspecific binding during affinity chromatography can be further avoided and reduced.

The method of the present invention allows for the manufacture of active processed neurotoxin polypeptide essentially free of unprocessed or partially processed precursor polypeptide and, thus, obtaining higher amounts of the active processed neurotoxin polypeptide. Such neurotoxin polypeptides can be used for formulating medicaments or cosmetic compositions. Essentially free in this context means that the unprocessed and/or partially processed neurotoxin is below a certain threshold, in an aspect, less than 2.5 %, less than 1 %, less than 0.5 % or less than 0.1 % of the total neurotoxin comprised by a given sample.

Thus, the present invention also relates to a method for the manufacture of a medicament comprising the steps of the aforementioned method and the further step of formulating the proteolytically processed neurotoxin polypeptide as medicament.

The term "medicament" as used herein refers, in one aspect, to a pharmaceutical composition containing the biologically active (proteolytically processed) neurotoxin polypeptide as pharmaceutical active compound, wherein the pharmaceutical composition may be used for human or non-human therapy of various diseases or disorders in a therapeutically effective dose.

A pharmaceutical composition as used herein comprises the biologically active (proteolytically processed) Neurotoxin polypeptide, and in one aspect, one or more pharmaceutically acceptable carrier. The active Neurotoxin can be present in liquid or lyophilized form. In an aspect, said compound can be present together with glycerol, protein stabilizers (e.g., human serum albumin (HAS)) or non-protein. stabilizers.

The pharmaceutical composition is, in one aspect, administered topically. Conventionally used drug administration is administered intra-muscular, subcutaneous (near glands). However, depending on the nature and the mode of action of a compound the pharmaceutical composition may be administered by other routes as well.

The compound, i.e. the biologically active (proteolytically processed) neurotoxin polypeptide is the active ingredient of the composition, and is in one aspect administered in conventional dosage forms prepared by combining the drug with standard pharmaceutical carriers according to conventional procedures. These procedures may involve mixing, granulating, and compression, or dissolving the ingredients as appropriate to the desired preparation. It will be appreciated that the form and character of the pharmaceutical acceptable carrier or diluent is dictated by the amount of active ingredient with which it is to be combined, the route of administration, and other well-known variables.

The carrier(s) must be acceptable in the sense of being compatible with the other ingredients of the formulation and being not deleterious to the recipient thereof. The pharmaceutical carrier employed may include a solid, a gel, or a liquid. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil, water, emulsions, various types of wetting agents, and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania.

The diluent(s) is/are selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or non-toxic, non-therapeutic, non-immunogenic stabilizers and the like.

A therapeutically effective dose refers to an amount of the compound to be used in a pharmaceutical composition which prevents, ameliorates or treats the symptoms accompanying a disease or condition referred to in this specification. Therapeutic efficacy and toxicity of the compound can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The dosage regimen will be determined by the attending physician and other clinical factors. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment.

The pharmaceutical compositions and formulations referred to herein are administered at least once in order to treat or ameliorate or prevent a disease or condition recited in this specification. However, the said pharmaceutical compositions may be administered more than one time.

Specific pharmaceutical compositions are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent. The resulting formulations are to be adapted to the mode of administration. Dosage recommendations shall be indicated in the prescribers or users instructions in order to anticipate dose adjustments depending on the considered recipient.

The medicament may in a further aspect of the invention comprise drugs in addition to the biologically active (proteolytically processed) neurotoxin polypeptide which are added to the pharmaceutical composition during its formulation. Finally, it is to be understood that the formulation of a pharmaceutical composition takes place under GMP standardized conditions or the like in order to ensure quality, pharmaceutical security, and effectiveness of the medicament.

The present disclosure, in general, contemplates a composition comprising proteolytically processed neurotoxin polypeptide obtainable by the method of the present invention.

The term "composition" refers to any composition formulated in solid, liquid, aerosol (or gaseous) form. Said composition comprises the compound optionally together with suitable auxiliary compounds such as diluents or carriers or further ingredients. In this context, it is distinguished between auxiliary compounds, i.e. compounds which do not contribute to the effects elicited by the compound upon application of the composition for its desired purpose, and further ingredients, i.e. compounds which contribute a further effect or modulate the effect of the compound. Suitable diluents and/or carriers depend on the purpose for which the composition is to be used and the other ingredients. The person skilled in the art can determine such suitable diluents and/or carriers without further ado. Examples of suitable carriers and/or diluents are disclosed elsewhere herein.

In a further aspect of the disclosure, the aforementioned composition is a medicament as specified elsewhere in the description in more detail. In one aspect the said medicament can be used for prevention and/or treatment of at least one of the following diseases and disorders: voluntary muscle strength, focal dystonia, including cervical, cranial dystonia, and benign essential blepharospasm, hemifacial spasm, and focal spasticity, gastrointestinal disorders, hyperhidrosis, and cosmetic wrinkle correction, in a further aspect also Blepharospasm, oromandibular dystonia, jaw opening type, jaw closing type, bruxism, Meige syndrome, lingual dystonia, apraxia of eyelid, opening cervical dystonia, antecollis, retrocollis, laterocollis, torticollis, pharyngeal dystonia, laryngeal dystonia, spasmodic dysphonia/adductor type, spasmodic dysphonia/abductor type, spasmodic dyspnea, limb dystonia, arm dystonia, task specific dystonia, writer's cramp, musician's cramps, golfer's cramp, leg dystonia, thigh adduction, thigh abduction knee flexion, knee extension, ankle flexion, ankle extension, equinovarus, deformity foot dystonia, striatal toe, toe flexion, toe extension, axial dystonia, pisa syndrome, belly dancer dystonia, segmental dystonia, hemidystonia, generalised dystonia, dystonia in lubag, dystonia in corticobasal degeneration, dystonia in lubag, tardive dystonia, dystonia in spinocerebellar ataxia, dystonia in Parkinson's disease, dystonia in Huntington's disease, dystonia in Hallervorden Spatz disease, dopa-induced dyskinesias/dopa-induced dystonia, tardive dyskinesias/tardive dystonia, paroxysmal dyskinesias/dystonias, kinesiogenic non-kinesiogenic action-induced palatal myoclonus, myoclonus myokymia, rigidity, benign muscle cramps, hereditary chin trembling, paradoxic jaw muscle activity, hemimasticatory spasms, hypertrophic branchial myopathy, maseteric hypertrophy, tibialis anterior hypertrophy, nystagmus, oscillopsia supranuclear gaze palsy, epilepsia, partialis continua, planning of spasmodic torticollis operation, abductor vocal cord paralysis, recalcitant mutational dysphonia, upper oesophageal sphincter dysfunction, vocal fold granuloma, stuttering Gilles de Ia Tourette syndrome, middle ear myoclonus, protective larynx closure, postlaryngectomy, speech failure, protective ptosis, entropion sphincter Odii dysfunction, pseudoachalasia, nonachalsia, oesophageal motor disorders, vaginismus, postoperative immobilisation tremor, bladder dysfunction, detrusor sphincter dyssynergia ,bladder sphincter spasm, hemifacial spasm, reinnervation dyskinesias, cosmetic use craw's feet, frowning facial asymmetries, mentalis dimples, stiff person syndrome, tetanus prostate hyperplasia, adipositas, treatment infantile cerebral palsy strabismus, mixed paralytic concomitant, after retinal detachment surgery, after cataract surgery, in aphakia myositic strabismus, myopathic strabismus, dissociated vertical deviation, as an adjunct to strabismus surgery, esotropia, exotropia, achalasia, anal fissures, exocrine gland hyperactivity, Frey syndrome, Crocodile Tears syndrome, hyperhidrosis, axillar palmar plantar rhinorrhea ,relative hypersalivation in stroke, in Parkinsosn's, in amyotrophic lateral sclerosis spastic conditions, in encephalitis and myelitis autoimmune processes, multiple sclerosis, transverse myelitis, Devic syndrome, viral infections, bacterial infections, parasitic infections, fungal infections, in hereditary spastic paraparesis postapoplectic syndrome hemispheric infarction, brainstem infarction, myelon infarction, in central nervous system trauma, hemispheric lesions, brainstem lesions, myelon lesion, in central nervous system hemorrhage, intracerebral hemorrhage, subarachnoidal hemorrhage, subdural hemorrhage, intraspinal hemorrhage, in neoplasias, hemispheric tumors, brainstem tumors, myelon tumors. For details and symptoms see, e.g., Jost 2007, Drugs 67(5), 669 or Dressier 2000 in Botulinum Toxin Therapy, Thieme Verlag, Stuttgart, New York.

In another aspect of the disclosure, the composition is a cosmetic composition which can be formulated as described for a pharmaceutical composition above. For a cosmetic composition, likewise, it is envisaged that the compound of the present disclosure is in an aspect used in substantially pure form. Cosmetic compositions are, in a further aspect, to be applied intramuscular. In an even further aspect of the disclosure, cosmetic compositions comprising the neurotoxin can be formulated as an anti-wrinkle solution.

### FIGURES

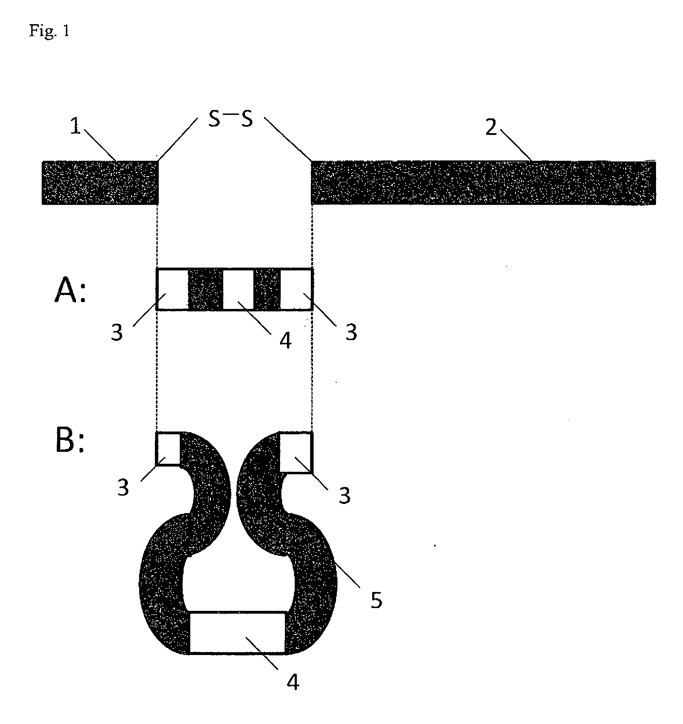

**Figure 1** is a schematic drawing of two embodiments according to the present invention (A and B). (A) shows a neurotoxin polypeptide with light chain (1) and heavy chain (2) with a linker comprising an epitope tag (4) flanked by two protease recognition and cleavage sites (3). (B) shows a neurotoxin polypeptide with light chain (1) and heavy chain (2) with a linker comprising an epitope tag (4) within a coiled coil structure (5) flanked by two protease recognition and cleavage sites (3).

## Claims

1. A polynucleotide encoding a neurotoxin polypeptide comprising a light chain, a linker and a heavy chain, wherein the linker is a modified linker comprising a heterologous amino acid sequence which confers at least one physicochemical property to the polypeptide which allows for separation of partially processed or unprocessed neurotoxin polypeptides from processed neurotoxin polypeptides, said heterologous amino acid sequence being flanked N- and C-terminally by a protease recognition and cleavage site, wherein the neurotoxin is a Botulinum neurotoxin.

2. The polynucleotide of claim 1, wherein said heterologous amino acid sequence binds with high affinity to a purification matrix.

3. The polynucleotide of claim 1 or 2, wherein said heterologous amino acid sequence which binds with high affinity to a purification matrix comprises a purification tag.

4. The polynucleotide of claim 1, wherein said heterologous amino acid sequence increases the molecular weight of the neurotoxin polypeptide such that the partially processed and/or unprocessed neurotoxin polypeptides can be physically separated from processed neurotoxin polypeptide.

5. The polynucleotide of any one of claims 1, 2 or 4, wherein said heterologous amino acid sequence which binds with high affinity to a purification matrix comprises (i) at least one amino acid domain conferring high affinity binding to the purification matrix and (ii) at least one amino acid domain which brings the cysteine residues forming the intramolecular disulphide bridge between light and heavy chain in close proximity and/or prevents interference of the heterologous amino acid sequence with the formation of proper disulfide bonds between the light and heavy chain.

6. The polynucleotide of claim 5, wherein said at least one domain conferring high affinity binding to the purification matrix comprises a purification tag.

7. The polynucleotide of claim 5 or 6, wherein said at least one amino acid domain which prevents interference of the heterologous amino acid sequence with the formation of proper disulfide bonds between the light and heavy chain forms a three dimensional structure which brings the cysteine residues forming the intramolecular disulphide bridge between light and heavy chain in physical proximity.

8. The polynucleotide of claim 7, wherein said three dimensional structure formed by the C- and N-terminal amino acids of the heterologous amino acid sequence is a antiparallel coiled coil or an antiparallel beta-sheet structure.

9. The polynucleotide of any one of claims 5 to 8, wherein said heterologous amino acid sequence which binds with high affinity to a purification matrix further comprises at least one detectable marker amino acid sequence.

10. The polynucleotide of claim 9, wherein said detectable marker amino acid sequence is selected from the group consisting of: an amino acid sequence of a fluorescent protein, an amino acid sequence of an enzyme capable of generating a detectable signal, and an amino acid sequence of a detectable tag.

11. The polynucleotide of claim 3 or any one of claims 6 to 9, wherein said purification tag is selected from the group consisting of: His-tag, Myc-tag, FLAG-tag, strep-tag, MBP-tag, NusA tag, GST-tag, streptavidin and avidin.

12. The polynucleotide of any one of claims 1 to 11, wherein said protease recognition and cleavage site is selected from the group consisting of: neurotoxin light chain protease recognition and cleavage site from SNAP 25, recognition and cleavage site from an E. coli protease, Thrombin recognition and cleavage site, Factor X recognition and cleavage site, Trypsin recognition and cleavage site, Enterokinase recognition and cleavage site, TEV protease recognition and cleavage site, HRV 3c recognition and cleavage site, and PreScission Protease recognition and cleavage site.

13. The polynucleotide of any one of claims 1 to 12, wherein said light and heavy chain of the neurotoxin polypeptide are the light and heavy chain of a neurotoxin selected from the group consisting of: BoNT/A, BoNT/B, BoNT/Cl, BoNT/D, BoNT/E, BoNT/F, BoNT/G or TeNT.

14. A vector comprising the polynucleotide of any one of claims 1 to 13.

15. A host cell comprising the polynucleotide of any one of claims 1 to 13 or the vector of claim 14.

16. The host cell of claim 15, wherein said host cell is a bacterial cell.

17. A polypeptide encoded by the polynucleotide of any one of claims 1 to 13.

18. A method for the manufacture of a processed neurotoxin polypeptide comprising the steps of:
a) contacting the unprocessed neurotoxin polypeptide of claim 17 with a purification matrix under conditions and for a time sufficient for allowing binding of the said unprocessed neurotoxin polypeptide to the purification matrix;
b) contacting the unprocessed neurotoxin polypeptide bound to the purification matrix with a protease which is capable of cleaving the neurotoxin polypeptide at the protease recognition and cleavage sites resulting in processed and partially processed and/or unprocessed neurotoxin polypeptides; and
c) removing the released, processed neurotoxin polypeptide from the purification matrix.

19. A method for the manufacture of a processed neurotoxin polypeptide comprising the steps of:
a) contacting a neurotoxin polypeptide of claim 17 with a protease which is capable of cleaving the neurotoxin polypeptide at the protease recognition and cleavage sites;
b) contacting the processed, unprocessed and/or partially processed neurotoxin polypeptide obtained in step a) with a purification matrix under conditions and for a time sufficient for allowing binding of the said unprocessed and/or partially processed neurotoxin polypeptide to the purification matrix; and
c) removing the released, processed neurotoxin polypeptide from the purification matrix.

20. The method of claim 19, wherein step a) is carried out in a host cell or a lysate thereof expressing the polynucleotide of any one of claims 1 to 13 and a protease which is capable of cleaving the neurotoxin polypeptide at the protease recognition and cleavage sites.

21. A method for the manufacture of a processed neurotoxin polypeptide comprising the steps of:
a) contacting a neurotoxin polypeptide of claim 17 with a protease which is capable of cleaving the neurotoxin polypeptide at the protease recognition and cleavage sites;
b) subjecting the processed, unprocessed and/or partially processed neurotoxin polypeptide obtained in step a) to physical conditions for a time sufficient for allowing physical separation of partially processed or unprocessed neurotoxin polypeptides from processed neurotoxin polypeptides; and
c) removing processed neurotoxin polypeptide after said physical separation.

## Patentansprüche

1. Polynukleotid, das für ein Neurotoxin codiert, das eine leichte Kette, einen Linker und eine schwere Kette umfasst, wobei es sich bei dem Linker um einen modifizierten Linker handelt, der eine heterologe Aminosäuresequenz umfasst, die dem Polypeptid mindestens eine chemisch-physikalische Eigenschaft verleiht, die die Trennung von teilweise prozessierten oder unprozessierten Neurotoxinpolypeptiden von prozessierten Neurotoxinpolypeptiden gestattet, wobei die heterologe Aminosäuresequenz N- und C-terminal von einer Proteaseerkennungs- und -spaltungsstelle flankiert ist, wobei es sich bei dem Neurotoxin um ein Botulinum-Neurotoxin handelt.

2. Polynukleotid nach Anspruch 1, wobei die heterologe Aminosäuresequenz mit der Affinität an eine Aufreinigungsmatrix bindet.

3. Polynukleotid nach Anspruch 1 oder 2, wobei die heterologe Aminosäuresequenz, die mit der Affinität an eine Aufreinigungsmatrix bindet, ein Aufreinigungs-Tag umfasst.

4. Polynukleotid nach Anspruch 1, wobei die heterologe Aminosäuresequenz das Molekulargewicht des Neurotoxinpolypeptids so erhöht, dass die teilweise prozessierten und/oder unprozessierten Neurotoxinpolypeptide physikalisch von prozessiertem Neurotoxinpolypeptid abgetrennt werden können.

5. Polynukleotid nach einem der Ansprüche 1, 2 oder 4, wobei die heterologe Aminosäuresequenz, die hochaffin an eine Aufreinigungsmatrix bindet, Folgendes umfasst: (i) mindestens eine Aminosäuredomäne, die der Aufreinigungsmatrix hochaffine Bindung verleiht, und (ii) mindestens eine Aminosäuredomäne, die die Cysteinreste, die die intramolekulare Disulfidbrücke zwischen der leichten und der schweren Kette bilden, in starke Nähe zueinander bringt und/oder verhindert, dass die heterologe Aminosäuresequenz die Bildung von ordentlichen Disulfidbindungen zwischen der leichten und der schweren Kette beeinträchtigt.

6. Polynukleotid nach Anspruch 5, wobei die mindestens eine Domäne, die der Aufreinigungsmatrix hochaffine Bindung verleiht, ein Aufreinigungs-Tag umfasst.

7. Polynukleotid nach Anspruch 5 oder 6, wobei die mindestens eine Aminosäuredomäne, die verhindert, dass die heterologe Aminosäuresequenz die Bildung von ordnungsgemäßen Disulfidbindungen zwischen der leichten und der schweren Kette beeinträchtigt, eine dreidimensionale Struktur ausbildet, die die Cysteinreste, die die intramolekulare Disulfidbrücke zwischen der leichten und der schweren Kette bildet, in physikalische Nähe zueinander bringt.

8. Polynukleotid nach Anspruch 7, wobei es sich bei der dreidimensionalen Struktur, die von den C- und N-terminalen Aminosäuren der heterologen Aminosäuresequenz gebildet wird, um eine antiparallele Superhelix oder eine antiparallele Beta-Faltblattstruktur handelt.

9. Polynukleotid nach einem der Ansprüche 5 bis 8, wobei die heterologe Aminosäuresequenz, die mit der Affinität an eine Aufreinigungsmatrix bindet, weiterhin mindestens eine nachweisbare Aminosäuremarkersequenz umfasst.

10. Polynukleotid nach Anspruch 9, wobei die nachweisbare Aminosäuremarkersequenz aus der Gruppe bestehend aus den Folgenden ausgewählt ist: einer Aminosäuresequenz eines fluoreszierenden Proteins, einer Aminosäuresequenz eines Enzyms, das fähig ist, ein nachweisbares Signal zu erzeugen, und einer Aminosäuresequenz eines nachweisbaren Tags.

11. Polynukleotid nach Anspruch 3 oder einem der Ansprüche 6 bis 9, wobei das Aufreinigungs-Tag aus der Gruppe bestehend aus den Folgenden ausgewählt ist: His-Tag, Myc-Tag, FLAG-Tag, strep-Tag, MBP-Tag, NusA-Tag, GST-Tag, Streptavidin und Avidin.

12. Polynukleotid nach einem der Ansprüche 1 bis 11, wobei die Proteaseerkennungs- und -spaltstelle aus der Gruppe bestehend aus den Folgenden ausgewählt ist: aus SNAP 25 stammende Proteaseerkennungs- und -spaltstelle der leichten Kette des Neurotoxins, Erkennungs- und Spaltstelle von einer E. coli-Protease, Thrombinerkennungs- und -spaltstelle, Faktor-X-Erkennungs- und -spaltstelle, Trypsinerkennungs- und -spaltstelle, Enterokinaseerkennungs- und -spaltstelle, TEV-Protease-Erkennungs- und -spaltstelle, HRV-3c-Erkennungs- und -spaltstelle sowie PreScission-Protease-Erkennungs- und -spaltstelle.

13. Polynukleotid nach einem der Ansprüche 1 bis 12, wobei es sich bei der leichten und der schweren Kette des Neurotoxinpolypeptids um die leichte und schwere Kette eines Neurotoxins, ausgewählt aus der Gruppe bestehend aus BoNT/A, BoNT/B, BoNT/C1, BoNT/D, BoNT/E, BoNT/F, BoNT/G oder TeNT handelt.

14. Vektor, umfassend das Polynukleotid nach einem der Ansprüche 1 bis 13.

15. Wirtszelle, umfassend das Polynukleotid nach einem der Ansprüche 1 bis 13 oder den Vektor nach Anspruch 14.

16. Wirtszelle nach Anspruch 15, wobei es sich bei der Wirtszelle um eine Bakterienzelle handelt.

17. Polypeptid, das von dem Polynukleotid nach einem der Ansprüche 1 bis 13 codiert wird.

18. Verfahren zur Herstellung eines prozessierten Neurotoxinpolypeptids, umfassend die folgenden Schritte:
a) Inkontaktbringen des unprozessierten Neurotoxinpolypeptids nach Anspruch 17 mit einer Aufreinigungsmatrix unter Bedingungen und über einen Zeitraum, die ausreichen bzw. der ausreicht, um ein Binden des unprozessierten Neurotoxinpolypeptids an die Aufreinigungsmatrix zu gestatten;
b) Inkontaktbringen des an die Aufreinigungsmatrix gebundenen unprozessierten Neurotoxinpolypeptids mit einer Protease, die fähig ist, das Neurotoxinpolypeptid an den Proteaseerkennungs- und -spaltstellen zu spalten, was zu prozessierten und teilweise prozessierten und/oder unprozessierten Neurotoxinpolypeptiden führt; und
c) Entfernen des freigesetzten prozessierten Neurotoxinpolypeptids von der Aufreinigungsmatrix.

19. Verfahren zur Herstellung eines prozessierten Neurotoxinpolypeptids, umfassend die folgenden Schritte:
a) Inkontaktbringen eines Neurotoxinpolypeptids nach Anspruch 17 mit einer Protease, die fähig ist, das Neurotoxinpolypeptid an den Proteaseerkennungs- und -spaltstellen zu spalten;
b) Inkontaktbringen des in Schritt a) erhaltenen prozessierten, unprozessierten und/oder teilweise prozessierten Neurotoxinpolypeptids mit einer Aufreinigungsmatrix unter Bedingungen und über einen Zeitraum, die ausreichen bzw. der ausreicht, um ein Binden des unprozessierten und/oder teilweise prozessierten Neurotoxinpolypeptids an die Aufreinigungsmatrix zu gestatten; und
c) Entfernen des freigesetzten prozessierten Neurotoxinpolypeptids von der Aufreinigungsmatrix.

20. Verfahren nach Anspruch 19, wobei Schritt a) in einer Wirtszelle oder einem Lysat davon, die/das das Polynukleotid nach einem der Ansprüche 1 bis 13 exprimiert, und einer Protease, die fähig ist, das Neurotoxinpolypeptid an den Proteaseerkennungs- und -spaltstellen zu spalten, erfolgt.

21. Verfahren zur Herstellung eines prozessierten Neurotoxinpolypeptids, umfassend die folgenden Schritte:
a) Inkontaktbringen eines Neurotoxinpolypeptids nach Anspruch 17 mit einer Protease, die fähig ist, das Neurotoxinpolypeptid an den Proteaseerkennungs- und -spaltstellen zu spalten;
b) Exponieren des in Schritt a) erhaltenen prozessierten, unprozessierten und/oder teilweise prozessierten Neurotoxinpolypeptids gegenüber physikalischen Bedingungen über einen Zeitraum, der ausreicht, die physikalische Trennung von teilweise prozessierten oder unprozessierten Neurotoxinpolypeptiden von prozessierten Neurotoxinpolypeptiden zu gestatten; und
c) Entfernen von prozessiertem Neurotoxinpolypeptid nach der physikalischen Trennung.

## Revendications

1. Polynucléotide codant pour un polypeptide de neurotoxine comprenant une chaîne légère, un lieur et une chaîne lourde, **caractérisé en ce que** le lieur est un lieur modifié comprenant une séquence d'acides aminés hétérologue qui confère au moins une propriété physico-chimique au polypeptide qui permet la séparation de polypeptides de neurotoxine partiellement traités ou non traités de polypeptides de neurotoxine traités, ladite séquence d'acides aminés hétérologue étant flanquée de façon N- et C-terminale par un site de reconnaissance et de clivage de protéase, la neurotoxine étant une neurotoxine de Botulinum.

2. Polynucléotide de la revendication 1, **caractérisé en ce que** ladite séquence d'acides aminés hétérologue se lie avec une affinité élevée à une matrice de purification.

3. Polynucléotide de la revendication 1 ou 2, **caractérisé en ce que** ladite séquence d'acides aminés hétérologue qui se lie avec une affinité élevée à une matrice de purification comprend une étiquette de purification.

4. Polynucléotide de la revendication 1, **caractérisé en ce que** ladite séquence d'acides aminés hétérologue augmente le poids moléculaire du polypeptide de neurotoxine de sorte que les polypeptides de neurotoxine partiellement traités et/ou non traités puissent être physiquement séparés du polypeptide de neurotoxine traité.

5. Polynucléotide de l'une quelconque des revendications 1, 2 ou 4, **caractérisé en ce que** ladite séquence d'acides aminés hétérologue qui se lie avec une affinité élevée à une matrice de purification comprend (i) au moins un domaine d'acide aminé conférant une liaison à affinité élevée à la matrice de purification et (ii) au moins un domaine d'acide aminé qui amène les résidus de cystéine formant le pont disulfure intramoléculaire entre la chaîne légère et la chaîne lourde à proximité étroite et/ou prévient l'interférence de la séquence d'acides aminés hétérologue avec la formation de liaisons disulfure appropriées entre les chaînes légère et lourde.

6. Polynucléotide de la revendication 5, **caractérisé en ce que** ledit au moins un domaine conférant une liaison à affinité élevée à la matrice de purification comprend une étiquette de purification.

7. Polynucléotide de la revendication 5 ou 6, **caractérisé en ce que** ledit au moins un domaine d'acide aminé qui prévient l'interférence de la séquence d'acides aminés hétérologue avec la formation de liaisons disulfure correctes entre les chaînes légère et lourde forme une structure tridimensionnelle qui amène les résidus de cystéine formant le pont disulfure intramoléculaire entre les chaînes légère et lourde physiquement à proximité.

8. Polynucléotide de la revendication 7, **caractérisé en ce que** ladite structure tridimensionnelle formée par les acides aminés C- et N-terminaux de la séquence d'acides aminés hétérologue est une structure de superhélice antiparallèle ou de feuillet bêta antiparallèle.

9. Polynucléotide de l'une quelconque des revendications 5 à 8, **caractérisé en ce que** ladite séquence d'acides aminés hétérologue, qui se lie avec une affinité élevée à une matrice de purification comprend en outre au moins une séquence d'acides aminés marqueuse détectable.

10. Polynucléotide de la revendication 9, **caractérisé en ce que** ladite séquence d'acides aminés marqueuse détectable est choisie dans le groupe constitué de : une séquence d'acides aminés d'une protéine fluorescente, une séquence d'acides aminés d'une enzyme capable de générer un signal détectable, et une séquence d'acides aminés d'une étiquette détectable.

11. Polynucléotide de la revendication 3 ou l'une quelconque des revendications 6 à 9, **caractérisé en ce que** ladite étiquette de purification est choisie dans le groupe constitué de : une étiquette His, une étiquette Myc, une étiquette FLAG, une étiquette strep, une étiquette MBP, une étiquette NusA, une étiquette GST, la streptavidine et l'avidine.

12. Polynucléotide de l'une quelconque des revendications 1 à 11, **caractérisé en ce que** ledit site de reconnaissance et de clivage de protéase est choisi dans le groupe constitué des : site de reconnaissance et de clivage de protéase de chaîne légère de neurotoxine de SNAP 25, site de reconnaissance et de clivage d'une protéase de E. coli, site de reconnaissance et de clivage de thrombine, site de reconnaissance et de clivage de facteur X, site de reconnaissance et de clivage de trypsine, site de reconnaissance et de clivage d'entérokinase, site de reconnaissance et de clivage de protéase TEV, site de reconnaissance et de clivage de HRV 3c, et site de reconnaissance et de clivage de PreScission Protease.

13. Polynucléotide de l'une quelconque des revendications 1 à 12, **caractérisé en ce que** lesdites chaînes légère et lourde du polypeptide de neurotoxine sont les chaînes légère et lourde d'une neurotoxine, choisie dans le groupe constitué de : BoNT/A, BoNT/B, BoNT/C1, BoNT/D, BoNT/E, BoNT/F, BoNT/G ou TeNT.

14. Vecteur comprenant le polynucléotide de l'une quelconque des revendications 1 à 13.

15. Cellule hôte comprenant le polynucléotide de l'une quelconque des revendications 1 à 13 ou le vecteur de la revendication 14.

16. Cellule hôte de la revendication 15, ladite cellule hôte étant une cellule bactérienne.

17. Polypeptide codé par le polynucléotide de l'une quelconque des revendications 1 à 13.

18. Procédé de fabrication d'un polypeptide de neurotoxine traité comprenant les étapes de :
a) mise en contact du polypeptide de neurotoxine non traité de la revendication 17 avec une matrice de purification dans des conditions et pendant un temps suffisant pour permettre la liaison dudit polypeptide de neurotoxine non traité à la matrice de purification ;
b) mise en contact du polypeptide de neurotoxine non traité lié à la matrice de purification avec une protéase qui est capable de cliver le polypeptide de neurotoxine aux sites de reconnaissance et de clivage de protéase pour obtenir des polypeptides de neurotoxine traités et partiellement traités et/ou non traités ; et
c) le retrait du polypeptide de neurotoxine traité libéré de la matrice de purification.

19. Procédé de fabrication d'un polypeptide de neurotoxine traité comprenant les étapes de :
a) mise en contact d'un polypeptide de neurotoxine de la revendication 17 avec une protéase qui est capable de cliver le polypeptide de neurotoxine aux sites de reconnaissance et de clivage de protéase ;
b) mise en contact du traité, polypeptide de neurotoxine non traité et/ou partiellement traité obtenu dans l'étape a) avec une matrice de purification dans des conditions et pendant un temps suffisant pour permettre la liaison dudit polypeptide de neurotoxine non traité et/ou partiellement traité à la matrice de purification ; et
c) le retrait du polypeptide de neurotoxine traité libéré de la matrice de purification.

20. Procédé de la revendication 19, dans lequel l'étape a) est conduite dans une cellule hôte ou un lysat de celui-ci exprimant le polynucléotide de l'une quelconque des revendications 1 à 13 et une protéase qui est capable de cliver le polypeptide de neurotoxine aux sites de reconnaissance et de clivage de protéase.

21. Procédé de fabrication d'un polypeptide de neurotoxine traité comprenant les étapes de :
a) mise en contact d'un polypeptide de neurotoxine de la revendication 17 avec une protéase qui est capable de cliver le polypeptide de neurotoxine aux sites de reconnaissance et de clivage de protéase ;
b) soumission du polypeptide de neurotoxine traité, non traité et/ou partiellement traité obtenu dans l'étape a) à des conditions physiques pendant un temps suffisant pour permettre la séparation physique de polypeptides de neurotoxine partiellement traités ou non traités des polypeptides de neurotoxine traités ;et
c) le retrait du polypeptide de neurotoxine traité après ladite séparation physique.
